# EUROPEAN PATENT APPLICATION

(11) **EP 1 997 431 A1**
(43) Date of publication of application: **03.12.2008**
(21) Application number: 07739352.8
(22) Date of filing: 22.03.2007
(51) Int. Cl.: A61B 5/151, A61B 5/1486, A61B 5/15, A61B 5/157, G01N 27/28, G01N 27/327, G01N 27/416

(54) **BIOSENSOR AND APPARATUS FOR MEASURING CONCENTRATION OF COMPONENTS**

(30) Priority: 22.03.2006 JP 2006078430; 24.03.2006 JP 2006082303; 13.04.2006 JP 2006110673; 14.04.2006 JP 2006111805
(71) Applicant: Panasonic Corporation, Kadoma-shi Osaka 571-8501 (JP)
(72) Inventor: YAJIMA, Hiroyoshi c/o Matsushita Electric Industrial Co., Ltd., 7F Twin 21 OBP Panasonic Tower 2-1-61, Shiromi, Chuo-ku Osaka 540-6207 (JP); EMOTO, Fumiaki c/o Matsushita Electric Industrial Co., Ltd., 7F Twin 21 OBP Panasonic Tower 2-1-61, Shiromi, Chuo-ku Osaka 540-6207 (JP); MIYAJI, Toshiaki c/o Matsushita Electric Industrial Co., Ltd., 7F Twin 21 OBP Panasonic Tower 2-1-61, Shiromi, Chuo-ku Osaka 540-6207 (JP); YOSHIOKA, Toshihiko c/o Matsushita Electric Industrial Co., Ltd., 7F Twin 21 OBP Panasonic Tower 2-1-61, Shiromi, Chuo-ku Osaka 540-6207 (JP); KAWASE, Yuki c/o Matsushita Electric Industrial Co., Ltd., 7F Twin 21 OBP Panasonic Tower 2-1-61, Shiromi, Chuo-ku Osaka 540-6207 (JP); MATSUBARA, Naoki c/o Matsushita Electric Industrial Co., Ltd., 7F Twin 21 OBP Panasonic Tower 2-1-61, Shiromi, Chuo-ku Osaka 540-6207 (JP)
(74) Representative: Grünecker, Kinkeldey, Stockmair & Schwanhäusser Anwaltssozietät
(86) International application number: PCT/JP2007/055909
(87) International publication number: WO 2007/108513

(57) **Abstract**

An object of the invention is to provide a component concentration measuring apparatus by blood sampling by laser perforating, capable of preventing leak of an abnormal odor generated during laser perforating, and improving user's convenience. In a component concentration measuring apparatus of the invention, a slide sheet 6 in which a film is provided on the optical axis of a laser beam, and an insertion holder 7 the inside of which is hollow are inserted into a main body 2 equipped with a laser device, a focusing lens, an analyzing device that analyzes components of a body fluid by an enzyme reaction of a specimen reagent, a laser operation button 5, a display 3, a display switching button 4, a chargeable battery, and an electric circuit on which a memory that stores the operation of the laser device, analysis of component concentration by the output of the analyzing device, and analysis results is mounted. In a biosensor 8 in which a film is provided on the optical axis of the laser beam, a protrusion of the insertion holder 7 and an opening of the biosensor 8 fit to each other.

## Description

### <Technical Field>

The present invention relates to a component concentration measuring apparatus that can perforate the skin of a human body with a laser beam, and rapidly and easily quantify a slight amount of various specific components in a sampled biological sample, and a biosensor used for the component concentration measuring apparatus.

### <Background Art>

A conventional biosensor will be described along with a conceptual diagram.

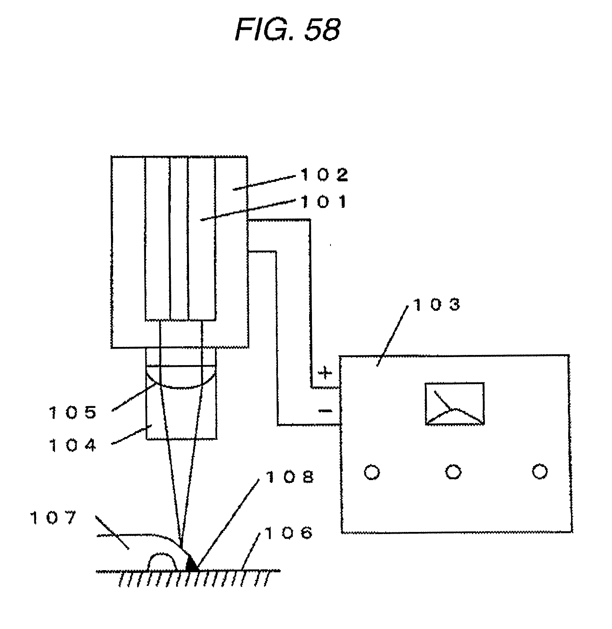

Fig. 58 shows a conventional laser device, and an aspect in which a palmar side of a fingertip is irradiated with a laser beam using the conventional laser device. Reference numeral 101 represents an internal solid-state laser oscillator, reference numeral 102 represents a main body in which the solid-state laser oscillator 101 is built, and a power source and a control device are provided in a controller 103 as a separate body. A lens hood 104 is provided on the output side of a laser beam in the main body 102, a focusing lens 105 is provided inside the lens hood 104, and a laser beam output from the solid-state laser oscillator 101 is focused by the focusing lens 105.

In order to sample blood from a fingertip, a portion of a palmar of a person's finger 107 whose blood is to be sampled is put on a platform 106 toward the focusing lens 105. The finger rest 108 is bent at the inside thereof such that the person's fingertip may enter slightly, and is fixed to an upper face of the platform 106 such that a laser beam may be focused in the vicinity of the outside of an upper edge of the rest.

When the finger 107 of the person whose blood is to be sampled is irradiated with a laser beam, a minute wound is made in an irradiation spot, and blood oozes out, Then, the blood of the person whose blood is to be sampled is sampled by a separately provided biosensor, and a slight amount of specific components in the blood is quantified (for example, refer to Patent Document 1).

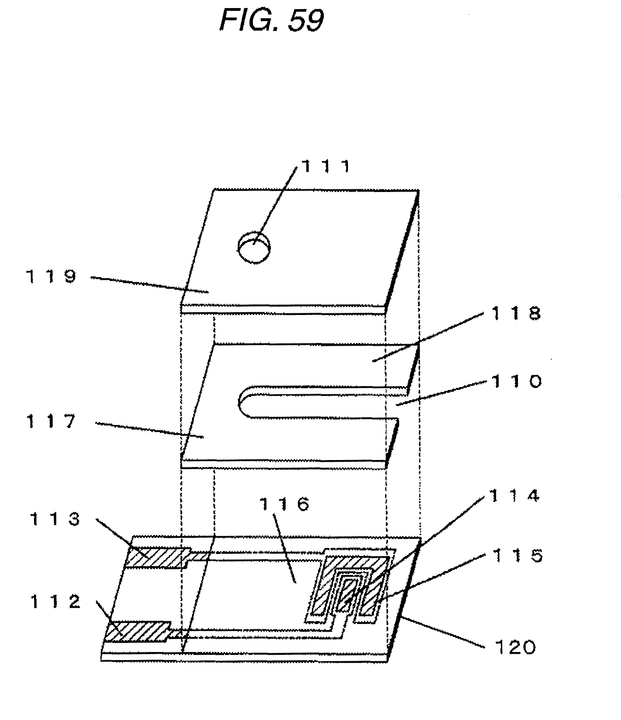

Further, Fig. 59 is an exploded perspective view of a glucose sensor manufactured as one embodiment of a conventional biosensor. On an insulating substrate 120, silver paste and conductive carbon paste including a resin binder are printed, and an electrode system composed of leads 112 and 113, and a measuring electrode 114, and a counter electrode 115 is formed, Moreover, an insulating layer 116 is formed by printing of insulating paste.

As for the system of the electrodes 114 and 115, after polishing of an exposed portion, an aqueous solution of carboxymethyl cellulose (hereinafter referred to as CMC that is a hydrophilic polymer is spread on the electrodes, thereby forming a CMC layer, and a substance in which glucose oxidase (GOD) as an enzyme is dissolved in a posphate buffer solution is spread so as to cover the CMC layer, thereby forming a reaction layer composed of a CMC-GOD layer.

Three members of the substrate 120, a spacer 117 made of a resin plate, and a cover 119, as shown in Fig. 59, are bonded and integrated so that the respective members may have the positional relationship shown by broken lines. An end of a cut portion becomes an inlet 110 of a sample solution when integrated, and a central portion forms a spatial portion 118. Further, the cover 118 has a hole, which becomes an outlet 111 when integrated.

When blood that is a sample solution is brought into contact with the inlet 110, the blood is guided to the inside from the inlet 110. At this time, the air within the spatial portion 118 is rapidly discharged from the outlet 111. As such, the blood propagates rapidly on electrode surfaces, and fills the spatial portion. The blood dissolves CMC and becomes viscose liquid, and the glucose in the blood reacts with oxygen by the action of the glucose oxidase carried on the electrodes, thereby generating hydrogen peroxide. By applying a voltage to between the electrodes, hydrogen peroxide generated by an enzyme reaction oxidizes with the measuring electrode, and a oxidation current value accompanying this corresponds to the concentration of the glucose that is a substance (for example, refer to Patent Document 2).

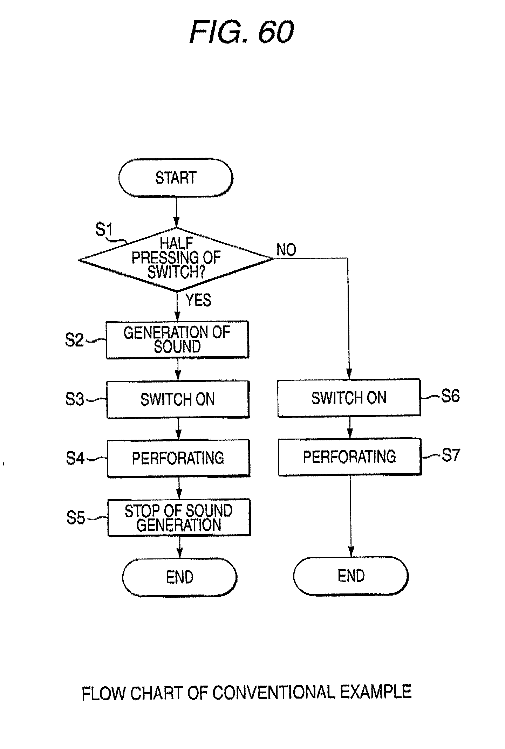

On the other hand, when a laser beam perforates a skin (laser perforating), the technique of alleviating user's pain is conventionally studied. Fig. 60 is a flow chart showing the operation of the conventional laser perforating device. In the conventional laser perforating device, if a start button of the device is pushed, whether a switch is pressed halfway is determined in Step S1. If a sound is desired to be generated (Yes), the switch is pressed halfway, and the process proceeds to Step S2 where a sound, such as music, is generated from a speaker. Next, by pushing a perforating switch in Step S3, the process proceeds to Step S4 where perforating is performed. Next, after lapse of a fixed period of time, the process proceeds to Step S5 where the sound flowing from the speaker stops.

Further, if the switch is pressed halfway in Step S1, the process proceeds to Step S6 where a perforating switch is pressed. Accordingly, in Step S7, the sound from the speaker does not flow but perforating is performed (for example, refer to Patent Document 3). As such, in the conventional laser perforating device, a speaker was provided in the laser perforating device so as to generate music, or a sound that cancels an operation sound during perforating. This alleviated the pain during perforating.
Patent Document 1: JP-A-04-314428
Patent Document 2: JP-A-01-291153
Patent Document 3: JP-A-2005-131088

### <Disclosure of the Invention>

### <Problems to be solved by the Invention>

However, there were the following problems in a component concentration measuring apparatus by the combination of the conventional laser device and biosensor.

The first is making a user smell an abnormal odor of a plume generated during perforating of a skin by a laser beam, and feel remarkable displeasure, and contamination caused by adhesion of the plume to optical components. The perforating of a skin by a laser beam is thermal processing of skin tissue by heating or evaporation of water, by absorption of laser beam energy into water that is a principal component of the skin tissue. At this time, a plume that is an evaporant is generated in space.

The skin is composed of epidermis, dermis, and subcutaneous tissue in this order from the outside. In order to sample blood, it is necessary to perforate the skin to capillary vessels in a dermal papilla on the side of a first-class epidermis of the dermis, and most of the perforating is performed on the epidermis. In a palmar skin, such as a finger pulp, particularly, the epidermis of the skin is thick, has a thickness of 1 to 1.5 mm, and most of the skin is a stratum corneum that has protein keratin as a principal component. Volatile substances that are generated as amino acids that are components of the protein are decomposed drift within the plume. The stratum corneum is composed of the fibrous tissue of the protein keratin, and fibers are bonded by cystine bonds that are bonds of a sulfur portion of amino acid cystine that is contained relatively much.

As these amino acids are decomposed during evaporation, volatile sulfur compounds or nitrogen compounds are generated, and particularly, a person feels them as an abnormal odor. Further, when these evaporants adhere to an optical component of a laser beam, the transmittance of the optical component declines due to absorption of the laser beam of the adhering matters, and seizing of the adhering matters occurs.

The second is a problem of infection while blood is sampled by the laser device. In order to focus a laser beam and to perforate a skin in a region of 1 mm square or less, the distance between a focusing lens and a skin should be kept constant with the precision of at least 1 cm or less. Therefore, in reality, the skin is pressed against a certain fixed structure, and is irradiated with a laser beam. Since this structure is used multiple times by the same user, or is used by two or more persons, there is a fear of infection via this structure. Further, as also described earlier, a plume is generated when perforating is performed with a laser beam. As this plume adheres, there is also a fear of infection,

The third is complicatedness during use. After blood sampling by a laser device, a user should fetch blood into a separately provided sensor, connect the sensor with a display, and quantify a slight amount of various specific components in the blood, Moreover, user's convenience may be lowered when there are a large number of replacement parts for solving the problems up to now.

Further, in the laser irradiation conditions of the conventional laser perforating device, the focusing diameter of a laser beam is as large as 0.5 mm or more, and a scar may remain one week or more. Although there are differences among individuals in the thickness and water amount of skins, in order to reliably perform perforating with a laser beam pulse of one shot to stably perform blood, excessive laser beam energy was radiated consequently. For this reason, there was a case that pain becomes strong because the perforating depth of a skin is too deep.

As a result of inventor's study, it was turned out that the focusing diameter of a laser beam within which a scar does not remain is 0.15 mm or less, and preferably 0.1 mm or less. Further, the inventors have found out that the individual difference in laser beam energy required for blood sampling is about twice if the focusing diameter is 0.5 mm (conventional), and becomes as small as about dozen of percents if the focusing diameter is 0.15 mm or less. Moreover, the inventor have found out that the laser beam energy required for blood sampling can also be significantly small energy of about several tens of mJ if the focusing diameter is 0.15 mm or less.

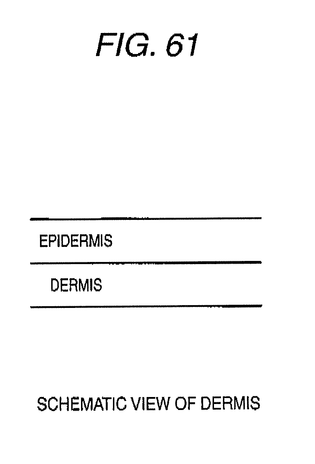

The inventors have found out the following facts, as a result of studying blood sampling from a skin by laser perforating in detail. Fig. 61 is a schematic view of a skin. The skin is composed of epidermis and dermis in this order from the outside, and in order to sample blood, it is necessary to damage capillary vessels within a dermal papilla in the dermis. However, a free nerve terminal that detects a pain also exists in the vicinity the dermal papilla. The epidermis consists of five kinds of layers including a stratum corneum on the uppermost surface.

Generally, blood sampling is performed from the skin of a fingertip or a palm. This is because the blood glucose level in the capillary vessels of the fingertip or palm can be measured without a time lag, for example when a diabetic's blood glucose level is measured. It is said that the fingertip that is the most general blood sampling spot has a thickness of 1 to 1.5 mm in the epidermis, particularly, stratum corneum. Further, the stratum corneum is dead tissue, and has a different appearance from living tissues, such as a dermal papilla of a dermis with a capillary vessels.

When the inventors observed the shape of a skin in its depth direction during laser perforating, they found out that, for example, when the surface of the skin is irradiated with a laser focusing pattern, the epidermis that is mainly composed of a stratum corneum has a substantially cylindrical sectional shape, and the dermis has a conical cross-sectional shape.

In order to realize a laser irradiation method with little pain, it is important to control the depth of the sectional shape of a conical laser-perforated hole in the dermis to necessary minimum. If the depth is excessive, the probability of stimulating the free nerve terminal, and making a user feel a pain becomes high, and bleeding may occur more than needed. Further, if the dermis is perforated deeply, as a reaction of heating and evaporation of the dermis tissue by the optical absorption of laser beam energy, an impulse wave (compression wave) in a direction opposite to the evaporation may be generated, and this also may become a kind of pain.

On the other hand, as mentioned above, in the conventional laser perforating device, a pain during perforating was alleviated by providing a speaker in the laser perforating device and by generating music or a sound that cancels the sound of operation during perforating. However, if a user always listens to the same music, etc., he/she may be accustomed to the music, etc. As a result, user's consciousness cannot be concentrated on the music, etc., and the pain during perforating cannot be alleviated.

Further, since the timing with which laser perforating is performed after a perforating switch is pushed is always the same, the user may be conscious of the timing of the laser perforating unconsciously, so that the pain during perforating cannot be alleviated,

Further, in the conventional laser perforating device, the focusing diameter or the time width of a laser pulse beam could be designed uniformly. However, since the optimal conditions for enabling reliable blood sampling differ from every user, the variable width of the laser pulse beam energy was set, and the user set the laser pulse beam energy individually. For this reason, in order for the user to search for laser pulse beam energy with which blood can be sampled, first, he/she should perform test laser perforating, so that blood cannot be sampled with single laser perforating. Further, since the searched conditions change with time from day to day, the conditions should be searched in each case.

The invention has been made to solve these conventional problems. Thus, one object of the invention is to provide a biosensor and a component concentration measuring apparatus, capable of keeping a user from feeling an abnormal odor during perforating with a laser beam, and performing simple and easy measurement without a fear of infection by use.

Further, an object of the invention is to provide a laser irradiation method of a laser perforating device capable of performing stable blood sampling without leaving a scar, and with little pain during perforating.

Further, an invention of the invention is to provide a laser perforating device and a laser perforating method in which a user is made not to be conscious of a moment of laser perforating, user's detection level of pain is made low, and the pain during perforating is little.

Further, an object of the invention is to provide a laser perforating device and a laser perforating method that makes it possible for a user to reliably sample blood without test perforating.

Moreover, an object of the invention is to provide the operating state of a laser perforating device, and specifically, to monitor the degree of deterioration of a flash lamp that is a main deterioration factor of this device. Further, another object is to monitor the number of times of use of a laser perforating device.

### <Means for Solving the Problems>

A component concentration measuring apparatus of invention includes:
a main body having at least a laser device that emits a laser beam, a focusing means of the laser beam, an analyzing device that analyzes components of a humour, and a display means that displays a result calculated by the analyzing device; a sheet; an insertion body having an opening that does not shield the laser beam; and a test paper. Here, the main body provides an opening in the direction of an optical axis of the laser beam. The opening is mounted with the insertion body, and one end of the insertion body is mounted with the test paper. The sheet provides a film that is located between the focusing means and the insertion body, and that is not perforated with the laser beam.

By this configuration, since a user does not smell an abnormal odor in a plume generated during laser perforating, and a test paper to be touched with user's sampling spot is replaced at every sampling, there is no fear of infection, and since the user can perform sampling and analysis with one instrument, simple and easy use is achieved.

Further, in the component concentration measuring apparatus of the invention, the test paper is provided with an electrode for component measurement, the insertion body is provided with an electrode, and the electrode for component measurement of the test paper and the electrode of the insertion body are electrically connected by fitting with the test paper.

By this configuration, a user can perform component analysis without removing a test paper and remounting it for component analysis again during the component analysis of a body fluid sampled by laser perforating, and thus simple and easy use becomes possible.

Further, in the component concentration measuring apparatus of the invention, the analyzing device analyzes the components of the body fluid by an enzyme reaction of a specimen reagent.

By this configuration, since a user does not smell an abnormal odor in a plume generated during laser perforating, and a test paper to be touched with user's sampling spot is replaced at every sampling, there is no fear of infection, and since the user can perform sampling and analysis with one instrument, simple and easy use is achieved.

Furthermore, in the component concentration measuring apparatus of the invention, the analyzing device is an optical device that analyzes the components of the body fluid by a color reaction of the specimen reagent.

By this configuration, since a user can perform sampling and analysis with one instrument, simple and easy use is achieved.

Further, in the component concentration measuring apparatus of the invention, the test paper provides a film that is perforated with the laser beam on the optical axis of the laser beam.

By this configuration, a plume generated during laser perforating is discharged through the perforating hole from a user's skin. As a result, the plume does not leak toward the user, so that the user can be prevented from smelling an abnormal odor in the plume generated during laser perforating.

Further, in the component concentration measuring apparatus of the invention, the test paper further provides a film that is not perforated with the laser beam on the optical axis of the laser beam.

By this configuration, a plume generated during laser perforating is discharged through the perforating hole from a user's skin, and is confined within the test paper. Thus, the plume does not leak toward the user, so that the user can be prevented from smelling an abnormal odor in the plume generated during laser perforating.

Further, in the component concentration measuring apparatus of the invention, the test paper has also a function as the sheet.

By this configuration, a user does not need to possess and manage two kinds of sheet and biosensor that are different in structure, and user's convenience can be improved.

Further, in the component concentration measuring apparatus of the invention, the test paper provides a film to be used as the sheet that is not perforated with the laser beam, on the optical axis of the laser beam when used as the sheet.

By this configuration, since a user does not need to possess and manage two kinds of sheet and biosensor that are different in structure, and it becomes unnecessary to replace the sheet at every use, user's convenience can be improved,

Furthermore, in the component concentration measuring apparatus of the invention, an asymmetrical structure is provided on the side where the insertion body fits into the main body.

By this configuration, a user does not mistake the insertion direction, and thus, simple and easy use becomes possible.

Furthermore, the component concentration measuring apparatus of the invention provides a structure in which the fitting between the test paper and the insertion body is performed by an asymmetrical structure.

By this configuration, a user does not mistake the fitting direction, and thus, simple and easy use becomes possible.

Furthermore, the component concentration measuring apparatus of the invention provides a structure in which the fitting between the test paper and the insertion body is performed by the fitting between the opening of the test paper, and a protrusion of the insertion body.

By this configuration, a user does not mistake the fitting direction, and slip-out hardly occurs. Thus, simple and easy use becomes possible,

Furthermore, in the component concentration measuring apparatus of the invention, the film of the sheet, the insertion body, and at least a portion or all of the film of the test paper are provided with a deodorizing function.

By this configuration, an abnormal odor in a plume generated during laser perforating can be eliminated.

Furthermore, in the component concentration measuring apparatus of the invention, the test paper, the insertion body, and at least a portion or all of the sheet are provided with an antibacterial function.

By this configuration, any infection when being used by a user can be prevented.

A biosensor of the invention is a biosensor for analyzing components in a specimen sample using perforating by a laser beam for collection of the specimen sample. Here, the sensor forms a sample supply passage through which the specimen sample supplied is sucked to between first and second substrates, at least by pasting between the first and second substrates, a filter, and a reagent that reacts with the components in the specimen sample are arranged within the sample supply passage, and an air hole that leads to the outside from the sample supply passage is provided in the second substrate. The reagent has an enzyme and a chromogen that reacts specifically with the components, openings that share a portion or all are provided outside the sample supply passage in the first and second substrates, and at least any one of the openings of the first and second substrates is provided with a film.

By this configuration, a plume including an abnormal odor generated during perforating by a laser beam is confined in a sensor board, and leak of the plume to the outside can be suppressed.

Further, in the biosensor of the invention, the film has a deodorizing function.

By this configuration, malodorous components in a plume generated during perforating by a laser beam can be eliminated.

Furthermore, in the biosensor of the invention, the first and second substrates are further provided with openings that share a portion or all other than the openings.

By this configuration, it is possible to reliably establish connection with the laser perforating device including a laser oscillator. Thereby, the relative distance between the laser oscillator and the sensor can be determined, and the shape of a laser-perforated hole can be made stable.

A biosensor for laser perforating of the invention is a biosensor for laser perforating for analyzing components in a specimen sample using perforating by a laser beam for collection of the specimen sample. The sensor forms a sample supply passage through which the specimen sample supplied is sucked to between first and second substrates, at least by pasting between the first and second substrates, a reagent that reacts with the components in the specimen sample is provided within the sample supply passage, and an air hole that leads to the outside from the sample supply passage is provided in the second substrate. The sensor has an electrode system, the electrode system includes at least a measuring electrode and an electrode couple, and the reaction is detected by the electrode system. Openings that share a portion or all are provided outside the sample supply passage in the first and second substrates, and a film is provided so as to cover at least any one of the openings of the first and second substrates.

By this configuration, a plume including an abnormal odor generated during perforating by a laser beam is confined in a sensor board, and leak of the plume to the outside can be suppressed.

Further, in the biosensor for laser perforating of the invention, the film has a deodorizing function.

By this configuration, malodorous components in a plume generated during perforating by a laser beam can be eliminated.

Furthermore, in the biosensor for laser perforating of the invention, the first and second substrates are further provided with openings that share a portion or all other than the openings.

By this configuration, it is possible to reliably establish connection with the laser perforating device including a laser oscillator. Thereby, the relative distance between the laser oscillator and the sensor can be determined, and the shape of a laser-perforated hole can be made stable.

Furthermore, in the biosensor for laser perforating of the invention, the first and second substrates are further provided with at least two openings that share a portion or all other than the openings so that the electrode system may be exposed.

By this configuration, it is possible to reliably establish connection with a device including a laser oscillator and an analyzing device. Thereby, the relative distance between the laser oscillator and the sensor can be determined, and the shape of a laser-perforated hole can be made stable. Furthermore, it is possible to electrically connect the sensor and the device, thereby monitoring a connection state.

Further, a component concentration measuring apparatus of the invention includes: a main body having at least a laser device that emits a laser beam, a focusing means of the laser beam, an analyzing device of components of a humour, and a display means that displays a result calculated by the analyzing device; a sheet; an insertion body having an opening through which the laser beam passes; and a test paper. The main body provides an opening in the direction of an optical axis of the laser beam. The opening is mounted with the insertion body, and one end of the insertion body is mounted with the test paper. The sheet is located between the focusing means and the insertion body, and the sheet has a function of the test paper.

By this configuration, since a user does not smell an abnormal odor in a plume generated during laser perforating, and there is also no contamination of optical components, which are focusing means, by a plume, it is possible to achieve simple and easy use that the user can manage a sheet and a sensor with one kind of consumable articles.

Further, the sheet provides a film that is not perforated with the laser beam when used as the sheet and that is perforated with the laser beam when used as the test paper, on the optical axis of the laser beam.

By this configuration, since a user does not smell an abnormal odor in a plume generated during laser perforating, and there is also no contamination of optical components, which are focusing means, by a plume, it is possible to achieve simple and easy use that the user can manage a sheet and a sensor with one kind of consumable articles.

Furthermore, the sheet provides a film that is perforated with the laser beam on the optical axis of the laser beam when used as the test paper, and provides a film that is not perforated with the laser beam on the optical axis of the laser beam when used as the sheet.

By this configuration, since a user does not smell an abnormal odor in a plume generated during laser perforating, and there is also no contamination of optical components, which are focusing means, by a plume, it is possible to achieve simple and easy use that the user can manage a sheet and a sensor with one kind of consumable articles.

Furthermore, a function to detect insertion of the sheet into the main body is further provided.

Moreover, user's forgetting of the insertion of a sheet can be prevented, and accordingly, any contamination of optical components, which are focusing means, by a plume can be prevented reliably.

Furthermore, the function to detect the insertion of the sheet into the main body is performed by mechanical contact operation by the insertion of the sheet.

By this configuration, user's forgetting of the insertion of a sheet can be prevented, and accordingly, any contamination of optical components, which are focusing means, by a plume can be prevented reliably.

Furthermore, the function to detect the insertion of the sheet into the main body is performed by electrical connection with the electrode provided in the sheet.

By this configuration, user's forgetting of the insertion of a sheet can be prevented, and accordingly, any contamination of optical components, which are focusing means, by a plume can be prevented reliably.

Furthermore, at least one or both of the film of the sheet and the insertion body is provided with a deodorizing function.

By this configuration, an abnormal odor in a plume generated during laser perforating can be eliminated.

Furthermore, at least one or both of the insertion body and the sheet is provided with an antibacterial function.

By this configuration, any infection when being used by a user can be prevented.

A perforating adapter according to the invention is a perforating adapter to be mounted on a laser perforating device that irradiates a skin with a laser beam, thereby performing perforating. The adapter includes: a hollow body having openings at both ends thereof, and first and second films provided so as to close the openings of both the ends. The first film absorbs the laser beam, and is perforated, and the second film transmits the laser beam without absorbing the laser beam.

According to the above configuration, a closed space can be formed by the first film, the hollow body, and the second film. Thereby, when laser perforating is performed with the first film pressed against a skin, a generated plume can be confined in the closed space. Therefore, a user can perform perforating without smelling an abnormal odor and feeling displeasure. Further, by replacing a perforating adapter at every use, there is no fear of infection during use, and perforating can be performed simply and easily.

Further, in the perforating adapter according to the invention, the hollow body has a protrusion formed around the first film.

According to the above configuration, since the protrusion will press the periphery of a predetermined laser perforating spot, it is possible to mitigate a stimulus that a user feels during laser perforating. Further, when blood is sampled by perforating, it is possible to reliably sample blood.

Further, in the perforating adapter according to the invention, at least one of the first film, the hollow body, and the second film is provided with a deodorizing function.

According to above configuration, an abnormal odor in a plume generated during laser perforating can be eliminated.

Further, in the perforating adapter according to the invention, the first film is provided with an antibacterial function.

According to the above configuration, infection via the perforating adapter can be prevented.

Furthermore, a laser perforating device according to the invention comprises the perforating adapter according to the invention so as to be detachable with a central axis of the hollow body and a laser optical axis being made to coincide with each other.

According to the above configuration, a closed space can be formed by the first film, the hollow body, and the second film. Thereby, when laser perforating is performed with the first film pressed against a skin, a generated plume can be confined in the closed space. Therefore, a user can perform perforating without smelling an abnormal odor and feeling displeasure. Further, according to the above configuration, since a perforating adapter to touch user's skin can be replaced at every use, there is no fear of infection, and perforating can be performed simply and easily.

The invention realizes stable blood sampling and laser perforating with little pain by making laser irradiation conditions for laser-perforating the dermis and laser irradiation conditions for laser-perforating the epidermis different from each other, and performing a plurality of times of irradiation with different kinds of laser pulse beam energy.

Specifically, a first laser pulse beam that perforates the epidermis is made the same or longer in time width and is made larger in laser beam energy than a second laser pulse beam that perforates the dermis. Here, it is also effective for stable blood sampling to divide a laser pulse beam that perforates the epidermis into a plurality of laser pulse beams. In this case, when the irradiation conditions of the laser pulse beams that perforates the epidermis, and the laser pulse beam that perforates the dermis are compared as individual laser pulse beams, they may be the same, or the laser pulse beam that perforates the dermis may be longer in time width and larger in laser beam energy than each of the laser pulse beams that perforates the epidermis.

Further, a laser irradiation method according to the invention is a laser irradiation method that irradiates a skin including epidermis and dermis with a laser pulse beam, thereby performing laser perforating. The method includes the steps of: radiating a laser pulse beam for perforating the epidermis, and radiating a laser pulse beam for perforating the dermis in a perforating spot of the epidermis.

According to the above configuration, a laser pulse beam for perforating dermis is radiated to the same position as a laser pulse beam for perforating epidermis, and a mortar-shaped perforating hole is formed in the dermis. Therefore, the dermis is not perforated deeply, any scar does not remain, pain during perforating is little, and stable blood sampling can be performed.

Further, in the laser irradiation method according to the invention, the laser pulse beam for perforating the epidermis is the same or longer in time width and is larger in energy than the laser pulse beam for perforating the dermis.

According to the above configuration, the depth of a cross-sectional shape of a laser-perforated hole in the dermis can be controlled to necessary minimum, any scar does not remain, pain during perforating is little, and stable blood sampling can be performed.

Further, in the laser irradiation method according to the invention, the time width of the laser pulse beam for perforating the epidermis is 100 to 400 µs, and the time width of the laser pulse beam for perforating the dermis is 50 to 300 µs.

According to the above configuration, since the dermis is not perforated deeply, any scar does not remain, pain during perforating is little, and stable blood sampling can be performed.

Further, in the laser irradiation method according to the invention, the difference between the time width of the laser pulse beam for perforating the epidermis and the time width of the laser pulse beam for perforating the dermis is 50 to 200 µs.

According to the above configuration, since a person whose blood is sampled perceives one perforating, pain during perforating is little, and stable blood sampling can be performed.

Further, in the laser irradiation method according to the invention, the irradiation interval between the laser pulse beam for perforating the epidermis and the laser pulse beam for perforating the dermis is 500 ms or less,

According to the above configuration, since a person whose blood is sampled perceives one perforating, pain during perforating is little, and stable blood sampling can be performed.

Further, in the laser irradiation method according to the invention, the total of the energy when the laser pulse beam for perforating the epidermis and the laser pulse beam for perforating the dermis irradiate is 100 to 300 J per one square centimeters.

According to the above configuration, the energy of a laser pulse beam for blood sampling can be minimized, any scar does not remain, pain during perforating is little, and stable blood sampling can be performed.

Further, in the laser irradiation method according to the invention, the energy of the laser pulse beam for perforating the dermis is 10 to 40% of the total of the energy of the laser pulse beam for perforating the epidermis and the laser pulse beam for perforating the dermis.

According to the above configuration, the depth of a cross-sectional shape of a conical laser-perforated hole in the dermis can be controlled to necessary minimum, any scar does not remain, pain during perforating is little, and stable blood sampling can be performed.

Further, in the laser irradiation method according to the invention, the focusing diameter of the laser pulse beam is 0.15 mm or less.

According to the above configuration, the differences among individuals in the energy of a laser pulse beam required for blood sampling can be made small, any scar does not remain, pain during perforating is little, and stable blood sampling can be performed.

Further, in the laser irradiation method according to the invention, the laser pulse beam for perforating the epidermis includes a plurality of laser pulse beams.

According to the above configuration, since a laser pulse beam for perforating the epidermis can be finely controlled according to differences among individuals in thickness, water amount, etc. of a skin, any scar does not remain, pain during perforating is little, and stable blood sampling can be performed.

Further, in the laser irradiation method according to the invention, the time width of each of the plurality of laser pulse beams for perforating the epidermis is 100 to 400 µs.

According to the above configuration, since a person whose blood is sampled perceives one perforating, pain during perforating is little, and stable blood sampling can be performed.

Further, in the laser irradiation method according to the invention, the interval of the plurality of laser pulse beams for perforating the epidermis is 500 ms or less.

According to the above configuration, since a person whose blood is sampled perceives one perforating, pain during perforating is little, and stable blood sampling can be performed.

Further, in the laser irradiation method according to the invention, the total of the energy when the plurality of laser pulse beams for perforating the epidermis and the laser pulse beam for perforating the dermis irradiate is 5 to 100 J per one square centimeters.

According to the above configuration, the energy of a laser pulse beam for blood sampling can be minimized, any scar does not remain, pain during perforating is little, and stable blood sampling can be performed.

A laser perforating device according to the invention is a laser perforating device that irradiates a skin with a laser beam, thereby performing perforating, includes a periodic sound generating means that generates a periodic sound. Perforating is performed during generation of the periodic sound.

According to the above configuration, by generating a periodic sound, user's consciousness is diverted, and a user is made not to be conscious of the moment of perforating. Thereby, a threshold level that the user feels pain becomes low. Since laser perforating is performed in this state, the pain during perforating can be reduced.

Further, in the laser perforating device according to invention, the periodic sound is a sound whose center frequency is repeated in monotone of 20 to 100 Hz.

According to the above configuration, since perforating is performed in a state where user's consciousness is directed to a sound repeated in monotone and the detection level of a user about pain is made low, the pain during the perforating can be alleviated.

Further, in the laser perforating device according to the invention, the sound that is repeated in monotone includes an operation sound of a pump, and an engine sound.

According to the above configuration, since perforating is performed in a state where user's consciousness is directed to a sound repeated in monotone, such as an operation sound of a pump, and an engine sound, and the detection level of a user about pain is made low, the pain during the perforating can be alleviated.

Further, in the laser perforating device according to invention, the periodic sound is a sound that has a repeated tempo of 60 to 208 beats per 1 minute.

According to the above configuration, since perforating is performed in a state where user's consciousness is directed to a sound with repeated tempo, and the detection level of a user about pain is made low, the pain during the perforating can be alleviated.

Further, in the laser perforating device according to the invention, the sound having the repeated tempo includes a metronome sound, an ON/OFF sound of a mechanical switch, and a sound that strikes a keyboard.

According to the above configuration, since perforating is performed in a state where user's consciousness is directed to a sound with repeated tempo, a metronome sound, an ON/OFF sound of a mechanical switch, and a sound that strikes a keyboard, and the detection level of a user about pain is made low, the pain during the perforating can be alleviated.

Further, in the laser perforating device according to the invention, the periodic sound generating means generates a plurality of kinds of periodic sounds that are different in sound quality or period, and randomly changes the kind of a periodic sound to be generated at every perforating.

According to the above configuration, since a plurality of kinds of periodic sounds that are different in sound quality or period are changed randomly, a user is not accustomed to an ambient sound, and user's consciousness is diverted from perforating. Thus, even in a case where a laser perforating device continues being used, the effect of alleviating pain during perforating can be maintained.

Further, in the laser perforating device according to invention, the time from the generation of the periodic sound to perforating is changed randomly at every perforating.

Further, since the time from the generation of the periodic sound to perforating is changed randomly at every perforating, a user cannot predict the timing of the laser perforating, and the pain during perforating can be alleviated.

Further, a laser perforating method according to the invention is a laser perforating method that irradiates a skin with a laser beam, thereby performing perforating. The method includes steps of: randomly selecting and generating a predetermined periodic sound from a plurality of kinds of periodic sounds that are different in sound quality or period, and performing perforating after lapse of the random time from the generation of the predetermined periodic sound.

According to the above configuration, since a plurality of kinds of periodic sounds that are different in sound quality or period are changed randomly, a user is not accustomed to an ambient sound. Further, since the time from the generation of the periodic sound to perforating is changed randomly, a user cannot predict the timing of the laser perforating, and the pain during perforating can be alleviated.

A laser perforating device according to the invention is a laser perforating device that irradiates a skin with a laser beam, thereby performing perforating. The laser perforating device includes an adjusting means that adjusts the energy of the laser pulse beam according to the water amount of the skin measured by a water-amount measuring sensor that measures the water amount of the skin.

According to the above configuration, the energy of a laser pulse beam that is suitable according to the water amount of a skin can be set, Therefore, test perforating is not performed, or a user himself does not perform setting of a laser pulse beam, and reliable blood sampling with little pain can be performed according to differences among individuals, daily fluctuation, and time fluctuation.

Further, the laser perforating device according to the invention includes the water-amount measuring sensor. Further, in the laser perforating device according to the invention, the water-amount measuring sensor includes a sensor electrode that is set on the surface of a main body of the laser perforating device to measure the water amount of the skin.

According to the above configuration, since the sensor electrode for measuring the water amount of a skin is set on the surface of a main body of the laser perforating device, the water amount of the skin can be measured easily.

Further, the laser perforating device according to the invention includes a holding means that detachably holds a perforating cap having the water-amount measuring sensor. The perforating cap includes a hallow body having openings at both ends thereof, and first and second films provided so as to close the openings of both the ends. The first film transmits the laser beam and absorbs the laser beam, and is perforated, and the second film transmits the laser beam without absorbing the laser beam. The water-amount measuring sensor includes a sensor electrode that is set on the same plane of the first film to measure the water amount of the skin.

According to the above configuration, since the perforating cap includes a sensor electrode that measures the water amount of a skin, simple, easy, reliable blood sampling can be performed with no fear of infection during use by replacing the perforating cap at every use.

Further, the laser perforating device according to the invention includes a setting button that inputs user's attributes. The adjusting means adjusts the energy of the laser pulse beam according to an attribute input from the setting button.

According to the above configuration, the energy of a laser pulse beam is adjusted according to user's attributes, such as the age, sex, race, or skin type, which are input from the setting button. Thus, it is possible to set laser pulse beam energy that is optimal according to the condition of a skin, and it is possible to perform reliable blood sampling with little pain.

Further, the laser perforating device according to the invention includes a humidity sensor that measures the humidity of the ambient air. The adjusting means adjusts the energy of the laser pulse beam according to the humidity of the ambient air measured by the humidity sensor.

According to the above configuration, since laser pulse beam energy is adjusted according to the humidity of the ambient air closely related to the water amount of a skin, it is possible to exactly set minimum laser pulse beam energy that is suitable for blood sampling.

Further, a laser perforating method according to the invention is a laser perforating method that irradiates a skin with a laser beam, thereby performing perforating. The method includes measuring the water amount of the skin, and adjusting the energy of the laser pulse beam according to the measured water amount of the skin.

According to the above configuration, the energy of a laser pulse beam that is suitable according to the water amount of a skin can be set. Therefore, test perforating is not performed, or a user himself does not perform setting of a laser pulse beam, and reliable blood sampling with little pain can be performed according to differences among individuals, daily fluctuation, and time fluctuation.

Further, an insertion holder of the invention is an insertion holder to be mounted on a laser perforating device that irradiates a skin with a laser beam, thereby performing perforating. The insertion holder includes: a hollow body having openings at both ends thereof; and a film that is provided so as to close one end of the openings, and transmits the laser beam without absorbing the laser beam; a biosensor provided so as to close the other end of the openings; and an electrode that is provided at an outer or inner peripheral face of the hollow body to electrically connect the laser perforating device and the biosensor.

According to the above configuration, the insertion holder has been constituted by the biosensor, the insertion holder, and the slide sheet that are three components, but is now constituted by one insertion holder. Thus, there is an advantage that the number of parts to be replaced when a user uses this device can be only one.

Further, in the insertion holder of the invention, a fingerrest film is formed in a portion of the surface of the biosensor, and a specimen reagent supply passage opening is formed in a side face of the biosensor.

Further, in the insertion holder of the invention, the biosensor has a measuring electrode and an electrode couple that are formed in a reagent layer. Further, an insertion holder of the invention is an insertion holder to be mounted on a laser perforating device that irradiates a skin with a laser beam, thereby performing perforating. The insertion holder includes: a hollow body formed by bending a biosensor, and joining ends of the biosensor; a film that is provided so as to close one end of the hollow body, and transmits the laser beam without absorbing the laser beam; a film that is provided so as to close the other end of the hollow body, and absorbs the laser beam and is perforated; and an electrode that is provided at an outer or inner peripheral face of the hollow body to electrically connect the laser perforating device and the biosensor.

Further, a laser irradiation method of the invention is a laser irradiation method of irradiating a skin via a film with a laser pulse beam, thereby performing laser perforating. The method includes the steps of: radiating a first laser pulse beam for perforating the film, and irradiating a second laser pulse beam for perforating the skin, in a perforating spot of the film.

Further, in the laser irradiation method of the invention, the intensity of the first laser pulse beam is weaker than that of the second laser pulse beam.

According to the above configuration, a through hole is formed in the film by the first laser pulse beam, and when there are differences among individuals in the laser perforating conditions of skins, stable blood sampling is allowed by adjusting the second laser pulse beam.

Further, a biosensor of the invention is a biosensor to be mounted on a laser perforating device that irradiates a skin with a laser beam, thereby performing perforating. The biosensor includes: a first substrate that has a reagent layer patched thereon, and has a first opening through which the laser beam passes; a second substrate that is arranged so as to face the first substrate at a predetermined spacing therefrom, has a second opening corresponding to the first opening, and has an air hole in a position more distant from the second opening than the reagent layer; and a specimen reagent supply passage formed from the first opening to the reagent layer between the first substrate and the second substrate.

According to the above configuration, a user can supply the blood that is an object to be measured to the biosensor as it is even if he/she does not bring his/her finger to the opening of the specimen reagent supply passage after the laser perforating.

Further, in the biosensor of the invention, the optical axis of the laser beam is set parallel to a centerline of the first opening, and in a position closer to the specimen reagent supply passage than the centerline of the first opening.

According to the above configuration, since the blood that has oozed out is closer to the specimen reagent supply passage than the sensor opening center, it is possible to more efficiently reach the reagent layer by the capillary phenomenon.

Further, a laser perforating device of the invention is a laser perforating device that irradiate a skin with a laser beam excited by a flash lamp, thereby performing perforating. The laser perforating device includes an optical sensor that detects light emission of the flash lamp to monitor the degree of deterioration of the flash lamp.

According to the above configuration, it is possible to notify a user of the maintenance time of the laser perforating device, and it is possible to increase the reliability as the perforating device.

Further, the laser perforating device of the invention includes a notifying means that notifies a user of a message that requests the maintenance of the device on the basis of the number of times of use of the device detected by the photosensor.

According to the above configuration, it is possible to notify a user of the maintenance time according to the number of times of use, and it is possible to charge a medical cost, etc. according to the number of times of use within a certain period to a person who bears the medical cost, such as a diabetic patient.

### <Effects of the Invention>

The invention provides a component concentration measuring apparatus that perforates a skin with a laser beam to sample a slight amount of a body fluid and to measure components of the body fluid by color reaction with a specimen reagent. In this component concentration measuring apparatus, a main body is provided with at least a laser device, a focusing means of the laser beam, an optical device that analyzes the color reaction, an analyzing device of the components, and a display means of analysis results. The main body has an opening in the optical axis of the laser beam. A sheet provided with a film that is not perforated with the laser beam, and an insertion body that has an opening that does not shield the laser beam are detachably inserted into the opening. Furthermore, the test paper that analyzes the components of the body fluid is provided so as to fit into the insertion body. Thereby, it is possible to provide the component concentration measuring apparatus having the following effects. That is, since a user does not smell an abnormal odor in a plume generated during laser perforating, and a test paper to be touched with user's sampling spot is replaced at every sampling, there is no fear of infection, and since the user can perform sampling and analysis with one instrument, simple and easy use is achieved.

Further, the invention provides a component concentration measuring apparatus that perforates a skin with a laser beam to sample a slight amount of a body fluid and to measure components of the body fluid. In this component concentration measuring apparatus, a main body is provided with at least a laser device, a focusing means of the laser beam, an analyzing device of the components, and a display means of analysis results. The main body has an opening in the optical axis of the laser beam. A sheet provided with a film that is not perforated with the laser beam, and an insertion body that has an opening that does not shield the laser beam is detachably inserted into the opening. Furthermore, the test paper of the body fluid is provided so as to fit into the insertion body. Thereby, it is possible to provide the component concentration measuring apparatus having the following effects. That is, since a user does not smell an abnormal odor in a plume generated during laser perforating, and a test paper to be touched with user's sampling spot is replaced at every sampling, there is no fear of infection, and since the user can perform sampling and analysis with one instrument, simple and easy use is achieved.

Further, according to the invention, an opening that passes through a substrate of a sensor is provided, and a film is provided so as to cover the opening. Thereby, it is possible to a biosensor for laser perforating having the effect that a plume including an odor generated during laser perforating can be prevented from leaking to outside.

Further, the invention provides a component concentration measuring apparatus that perforates a skin with a laser beam to sample a slight amount of a body fluid and to measure components of the body fluid. The component concentration measuring apparatus includes a main body provided with at least a laser device that emits a laser beam, a focusing means of the laser beam, an analyzing device of the components of the body fluid, and a display means that displays results calculated by the analyzing device; a sheet; an insertion body having an opening that does not shield the laser beam; and a test paper. The main body provides an opening in the direction of an optical axis of the laser beam. The opening is mounted with the insertion body, and one end of the insertion body is mounted with the test paper. The sheet is located between the focusing means and the insertion body, and the sheet has a function of the test paper. Thereby, it is possible to provide the component concentration measuring apparatus having the following effects. That is, since a user does not smell an abnormal odor in a plume generated during laser perforating, contamination of optical components, which are focusing means, by a plume can also be prevented, and a test paper to be touched with user's sampling spot is replaced at every sampling, there is no fear of infection, and since the user can manage at least a sheet and a test paper with one kind of consumable articles, and the user can perform sampling and analysis with one instrument, simple and easy use is achieved.

Further, according to the invention, a closed space can be formed by a first film, a hollow body, and a second film. Thereby, when laser perforating is performed with the first film pressed against a skin, a generated plume can be confined in the doused space. Therefore, a user can perform perforating without smelling an abnormal odor and feeling displeasure.

According to the invention, a laser pulse beam for perforating dermis is radiated to the same position as a laser pulse beam for perforating epidermis, and a mortar-shaped perforating hole is formed in the dermis. Therefore, the dermis is not perforated deeply, any scar does not remain, pain during perforating is little, and stable blood sampling can be performed.

According to the invention, by generating a periodic sound, user's consciousness is diverted, and a user is made not to be conscious of the moment of perforating. Thereby, a threshold level that the user feels pain becomes low. Since laser perforating is performed in this state, the pain during perforating can be reduced.

Further, according to the invention, the energy of a laser pulse beam that is suitable according to the water amount of a skin can be set. Therefore, test perforating is not performed, or a user himself does not set a laser pulse beam, and blood sampling with little pain can be performed according to differences among individuals, daily fluctuation, and time fluctuation.

Furthermore, according to the above invention, it is possible to notify a user of the maintenance time of the laser perforating device, and it is possible to increase the reliability as the perforating device. Otherwise, it is possible to notify a user of the maintenance time according to the number of times of use, and it is possible to charge a medical cost, etc. according to the number of times of use within a certain period to a person who bears the medical cost, such as a diabetic patient.

### <Brief Description of the Drawings>

Fig. 1 is a top view of a component concentration measuring apparatus in a first embodiment according to the invention.
Fig. 2 is an internal configuration diagram when the component concentration measuring apparatus in the first embodiment according to the invention is seen from the side,
Fig. 3 is a lower internal configuration diagram when the component concentration measuring apparatus in the first embodiment according to the invention is seen from the top.
Fig. 4 is a perspective view of an insertion holder in the first embodiment according to the invention.
Fig. 5 is a plan view and a sectional view of a slide sheet in the first embodiment according to the invention.
Fig. 6 is a plan view and a sectional view of a biosensor in the first embodiment according to the invention.
Fig. 7 is an enlarged view of a laser perforating spot during laser perforating.
Fig. 8 is an enlarged view of the laser perforating spot during the laser perforating.
Fig. 9 is a plan view and a sectional view of a biosensor in a second embodiment according to the invention.
Fig. 10 is a plan view and a sectional view of a biosensor in a third embodiment according to the invention.
Fig. 11 is a plan view and a sectional view of a biosensor in a fourth embodiment according to the invention.
Fig. 12 is a configuration diagram of an analyzing unit of the biosensor in the first embodiment according to the invention.
Fig. 13 is a perspective view of an insertion holder in the fifth embodiment according to the invention.
Fig. 14 is a plan view and a sectional view of a biosensor in a fifth embodiment according to the invention.
Fig. 15 is an exploded perspective view of a biosensor for laser perforating in a sixth embodiment of the invention.
Fig. 16 is a general view of the biosensor for laser perforating in the sixth embodiment of the invention.
Fig. 17 is a sectional view when the biosensor for laser perforating in the sixth embodiment of the invention is cut at a central portion in a longitudinal direction of Fig. 16.
Fig. 18 is a view showing the relationship between the sensor, a finger, and a laser beam when the biosensor for laser perforating in the sixth embodiment of the invention is used.
Fig. 19 is a view showing a situation during laser perforating in detail when the biosensor for laser perforating in the sixth embodiment of the invention is used,
Fig. 20 is a plan view and a sectional view of a biosensor for laser perforating in a seventh embodiment of the invention.
Fig. 21 is a plan view and a sectional view of a biosensor for laser perforating in an eighth embodiment of the invention.
Fig. 22 is a view illustrating how to use the biosensor for laser perforating in the sixth embodiment of the invention.
Fig. 23 is a plan view and sectional views of a biosensor in a ninth embodiment according to the invention.
Fig. 24 is a plan view and a sectional view of another biosensor in a tenth embodiment according to the invention.
Fig. 25 is a view illustrating how to recognizing another slide sheet in the ninth embodiment according to the invention.
Fig. 26 is a plan view and a sectional view of another biosensor in an eleventh embodiment according to the invention.
Fig. 27 is a perspective view of a laser perforating device 1 according to a twelfth embodiment of the invention.
Fig. 28 is a schematic view of a perforating adapter 507 the laser perforating device 1 according to the twelfth embodiment of the invention.
Fig. 29 is a schematic view of a perforating adapter 514 the laser perforating device 1 according to the twelfth embodiment of the invention.
Fig. 30 is a schematic view of laser irradiation conditions of a laser perforating device according to a thirteenth embodiment of the invention.
Fig. 31 is an enlarged view of a perforated hole of a skin in the laser irradiation conditions of the laser perforating device according to the thirteenth embodiment of the invention.
Fig. 32 is a schematic view of other laser irradiation conditions of a laser perforating device according to the thirteenth embodiment of the invention.
Fig. 33 is a schematic view of a laser oscillator 620 to be carried on the laser perforating device according to the thirteenth embodiment of the invention.
Fig. 34 is a perspective view of the laser perforating device 1 according to the thirteenth embodiment of the invention (1).
Fig. 35 is a perspective view of a laser perforating device 1 according to a fourteenth embodiment of the invention.
Fig. 36 is a flow chart of the operation of the laser perforating device 1 according to the fourteenth embodiment of the invention.
Fig.37 is a view showing an example of a periodic sound to be used by the laser perforating device 1 according to the fourteenth embodiment of the invention.
Fig. 38 is a block diagram of the laser perforating device 1 according to the fourteenth embodiment of the invention.
Fig. 39 is a schematic view of a perforating cap 507 where a water-amount measuring sensor is set, in a laser perforating device 1 of a fifteenth embodiment of the invention.
Fig. 40 is a sectional view of a state where the perforating cap 7 according to the fifteenth embodiment of the invention is applied to a skin.
Fig. 41 is a sectional view of the vicinity of the perforating cap 507 according to the fifteenth embodiment of the invention.
Fig. 42 is a view showing the flow of use of the laser perforating device 1 according to the fifteenth embodiment of the invention.
Fig. 43 is an explanatory view of a case where laser pulse beam energy is corrected on the basis of water amount measurement results of a skin in the embodiment of the invention.
Fig. 44 is a view showing an example in which sensor electrodes 821 for measurement of water amount, and a humidity sensor 822 are arranged on the surface of the laser perforating device 1 in the embodiment of the invention.
Fig. 45 is an enlarged view of a laser perforating spot during laser piercing in the embodiment of the invention.
Fig. 46 is a schematic view of an insertion holder in a sixteenth embodiment of the invention.
Fig. 47 is a schematic view of the insertion holder in the sixteenth embodiment of the invention.
Fig. 48 is a schematic view of a biosensor in the sixteenth embodiment of the invention.
Fig. 49 is a schematic view of the insertion holder in the sixteenth embodiment of the invention.
Fig. 50 is a view showing an oscillation state of a laser pulse beam in a seventeenth embodiment of the invention.
Fig. 51a is an enlarged view of a laser perforating spot after irradiation of a first laser pulse beam in the seventeenth embodiment of the invention, and an enlarged view (b) of the laser perforating spot after irradiation of a second laser pulse beam.
Fig. 52 is a plan view and a sectional view of a biosensor in an eighteenth embodiment of the invention.
Fig. 53 is an enlarged view (a) of a laser perforating spot during laser perforating in the eighteenth embodiment of the invention, and an enlarged view (b) of the laser perforating spot after the laser perforating.
Fig. 54 is an enlarged view of a laser perforating spot during laser perforating in a nineteenth embodiment of the invention.
Fig. 55 is a schematic view of a laser perforating device in a twentieth embodiment of the invention.
Fig. 56 is a flow chart of the operation of the laser perforating device in the twentieth embodiment of the invention.
Fig. 57 is a schematic view of a light waveform in the twentieth embodiment of the invention.
Fig. 58 is a general view of a conventional laser device.
Fig. 59 is an exploded perspective view of a conventional biosensor.
Fig. 60 is a flow chart showing the operation of the conventional laser perforating device.
Fig. 61 is a schematic view of a skin for explaining laser perforating.

### <Reference Numerals>

1: COMPONENT CONCENTRATION MEASURING APPARATUS
2,102: MAIN BODY
3: DISPLAY
4: DISPLAY SWITCHING BUTTON
5: LASER OPERATION BUTTON
6: SLIDE SHEET
7,207: INSERTION HOLDER
8: BIOSENSOR
9: LASER DEVICE
10: BATTERY
11: ELECTRIC CIRCUIT
12, 105: FOCUSING LENS
13: ANALYZING OPTICAL DEVICE
14, 213: CYLINDRICAL BODY
15, 214: CUTOUT
16: PROTRUSION
17, 313: LASER BEAM
18, 107, 314: FINGER
19: THROUGH HOLE
20: LASER PERFORATING
21: PLUME
22, 32: OPENING
31, 51, 100, 251, 301, 51: SUBSTRATE
33, 54, 63, 65, 254, 304, 325, 454: FILM
52: FILTER
53, 255, 305, 455: SPECIMEN REAGENT
55, 117, 256, 306, 456: SPACER
56, 257, 307, 457; SPECIMEN REAGENT SUPPLY PASSAGE
57, 119, 258, 308, 458: COVER
58, 559, 459: VENT
59: SUBSTRATE OPENING
60: SPACER OPENING
61: COVER OPENING
62: SECOND OPENING
64: THIRD OPENING
66: LIGHT-EMITTING ELEMENT
67: LIGHT-RECEIVING ELEMENT
101: SOLID-STATE LASER OSCILLATOR
103: CONTROLLER
104: LENS hood
106: PLATFORM
108: FINGER REST
110: INLET
111: OUTLET
112, 113: LEAD
114: MEASURING ELECTRODE
115: COUNTER ELECTRODE
116: INSULATING LAYER
118: SPATIAL PORTION
218: FIRST ELECTRODE
215: FIRST PROTRUSION
216: SECOND PROTRUSION
217: THIRD PROTRUSION
425: SWITCH

### <Best Mode for Carrying out the Invention>

### (First Embodiment)

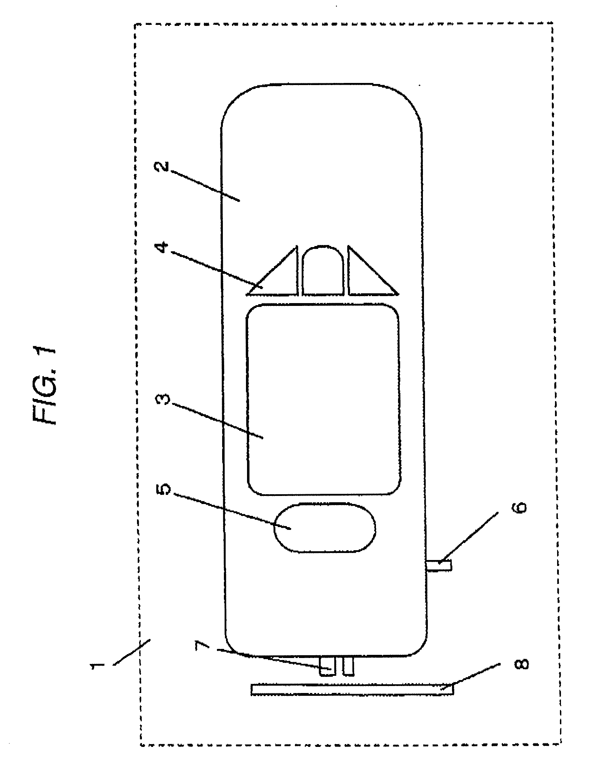

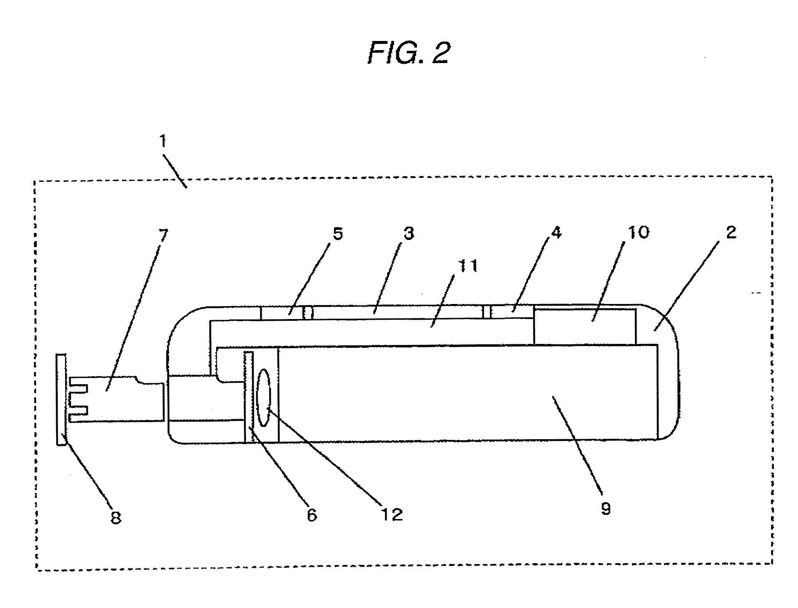

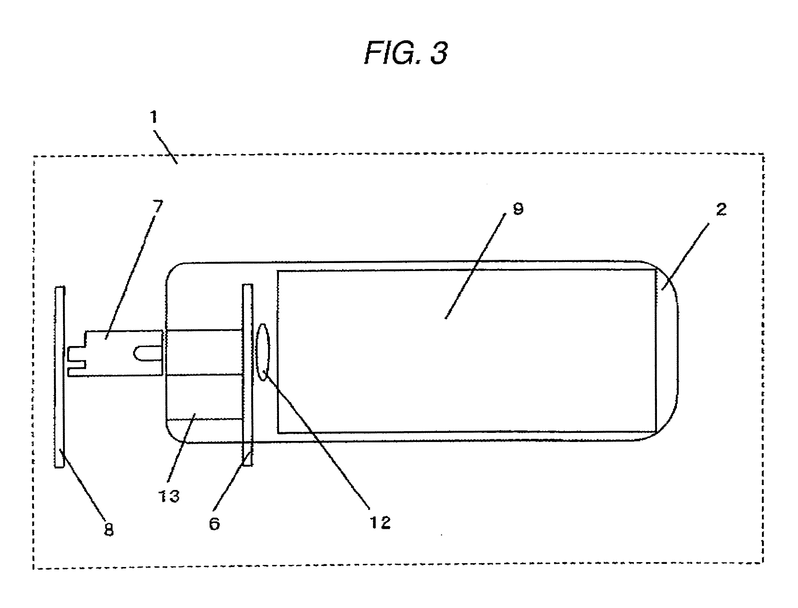

Hereinafter, a biosensor and a component concentration measuring apparatus in a first embodiment according to the invention will be described with reference to the drawings. Fig. 1 is a top view of a component concentration measuring apparatus in a first embodiment according to the invention, Fig. 2 is an internal configuration diagram of the component concentration measuring apparatus when Fig. 1 is seen from the side, and Fig. 3 is a lower internal configuration diagram of the component concentration measuring apparatus when Fig. 1 is seen from the top.

In Fig. 1, the component concentration measuring apparatus 1 is constituted from a display 3, display switching buttons 4 of the display 3, and a laser operation button 5, which are provided in a main body 2, and further, a slide sheet 6 replaceable with respect to the main body 2, an insertion holder 7 fitted into the main body 2, and a biosensor 8. Although Fig. 1 shows that the insertion holder 7 and the biosensor 8 are separated, the insertion holder 7 and the biosensor 8 are integrated together by fitting during operation.

In Fig. 2 that is an exploded view of an internal configuration when Fig. 1 is seen from the side, the same reference numerals as those of Fig. 1 designate the same components as those of Fig. 1. A laser device 9, a chargeable battery 10, an electric circuit 11, and a focusing lens 12 are arranged inside the main body 2. In Fig. 3 that is an internal configuration diagram when Fig. 1 is seen from the top, the same reference numerals as those of Figs. 1 and 2 designate the components as those of Figs. 1 and 2. An analyzing optical device13 for analysis of the biosensor 8, the laser operation button 5, the display 3, the display switching buttons 3, the battery 4, and the laser device 9 are connected with the electric circuit 11 by wiring lines that are not shown. Similarly, the laser device 9 is connected with the battery 10 by wiring lines that are not shown.

### (Insertion Holder)

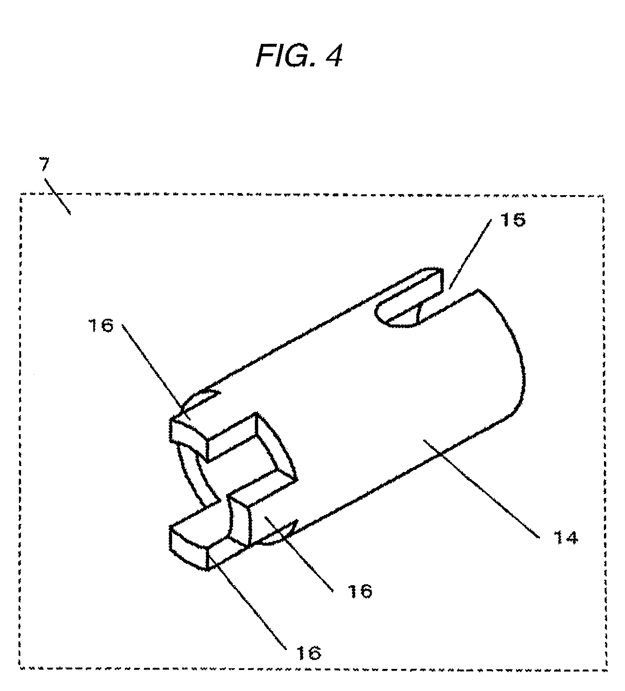

Fig. 4 is a detailed perspective view of the insertion holder 7. In the insertion holder 7, a cutout 15 is arranged at one end of a cylindrical body 14, and protrusions 16 are arranged in three directions at the other end of the cylindrical body.

As the cylindrical body 14, various plastic materials are available, and they are polyethylene, polyvinyl chloride, polypropylene, polystyrene, polyvinyl acetate, ABS resin, AS resin, acrylic resin, polyacetal, polyimide resin, polycarbonate, modified polyphenylene ether (PPE), polybutylene terephthalate (PPB), polyarylate, polysulfone, polyphenylene sulfide, polyether ether ketone, fluororesin, or the like. In this embodiment, AS resin was used. A material with low zeta potential on a plastic surface is preferable.

The outside dimension of the insertion holder 7 may be within such a range that the biosensor 8 can be inserted, and is preferably 3 to 25 mm. Further, the wall thickness of the cylindrical body 14 is 0.2 to 3 mm, and the length thereof is 5 to 30 mm. In this embodiment, the insertion holder is formed in a cylindrical shape. However, a polyhedral tubular body or the like is available.

### (Slide Sheet)

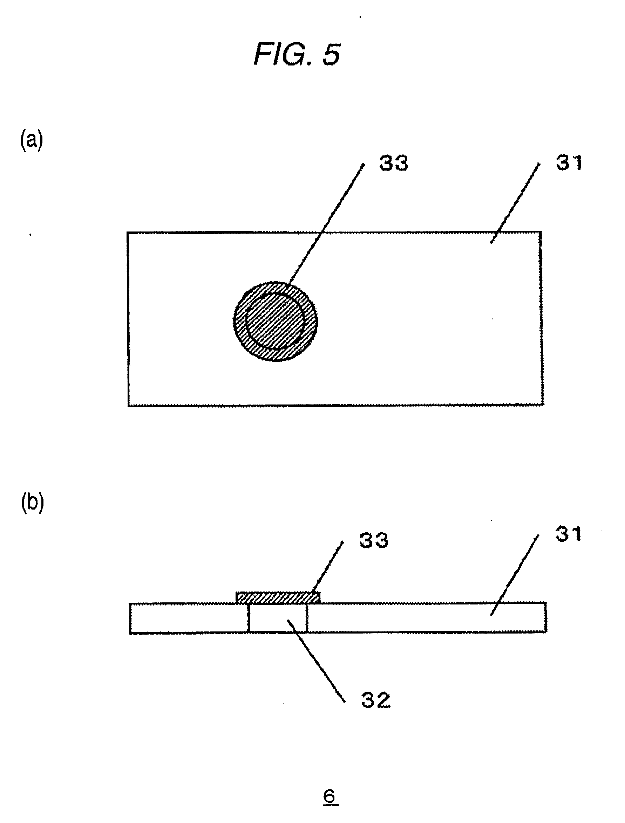

Fig. 5 is a detailed view of the slide sheet 6. Fig. 5(a) is a plan view, and Fig. 5(b) is a sectional view when being cut at a central portion in a longitudinal direction.

The substrate 31 is made of polyethylene terephthalate, polyester, polyolefin, polyamide, polyether, polyamideimide, polystyrene, polycarbonate, poly-ρ-phenylene sulfide, polyvinyl chloride, or the like. In this embodiment, polyethylene terephthalate was used. The substrate 31 has an opening 32, and the opening 32 is covered with a film 33. As the film 33, polyamide, polyester, polyimide, fluorine system, vinyl chloride, polyethylene, polyolefin, polyvinyl alcohol, polypropylene, and the like are available. The slide sheet 6 is 0.1 to 1 mm in the whole thickness, is 5 to 30 mm in the length of a short side, and is 30 to 60 mm in the length of a long axis.

### (Biosensor)

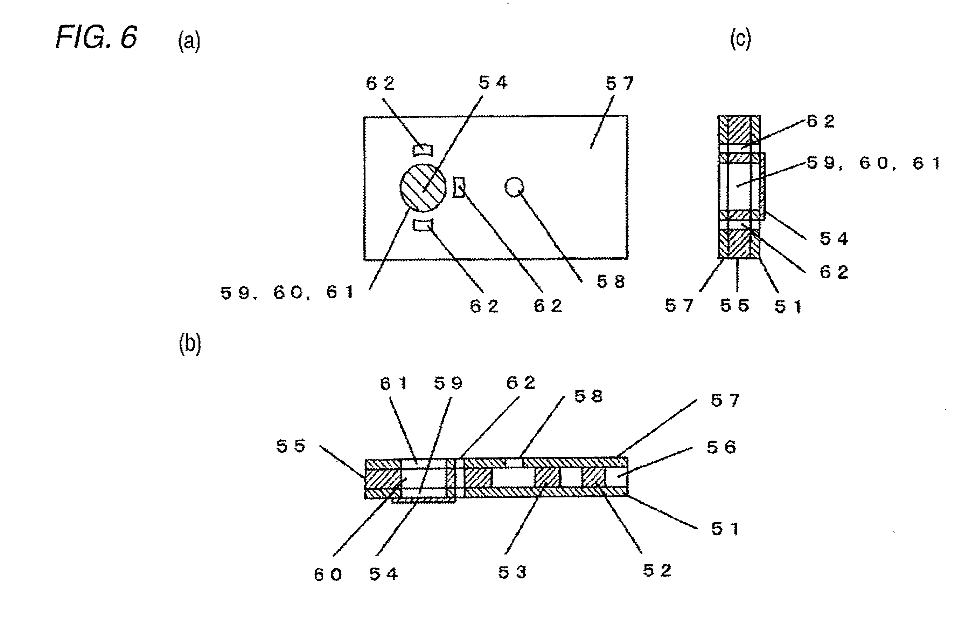

Fig. 6 is a detailed view of the biosensor 8. Fig. 6(a) is a plan view, Fig. 6(b) is a sectional view when being cut at a central portion in a longitudinal direction, and Fig. 6(c) is a sectional view when being cut in a central portion in a lateral direction.

A substrate 51 is made of an insulating material including polyethylene terephthalate or the like, and a cover 57 is made of an insulating material, and a air hole 58 is substantially circular. The material of the cover 57 is preferably a plastic film, and includes polyester, polyolefin, polyamide, polyether, polyamideimide, polystyrene, polycarbonate, poly-ρ-phenylene sulfide, polyvinyl chloride, and the like. Further, the cover has a thickness that it is almost transparent with respect to visible light to near-infrared light. In this embodiment, polycarbonate was used. Further, copolymers, blend materials, and bridged materials of them can be used for the cover 57, and the cover having a thickness of 0.01 mm to 0.5 mm can be used.

Also, a spacer 55 that has a cutout portion for forming a specimen reagent supply passage 56 that supplies a specimen reagent, a filter 52 for separating a corpuscle component, and a reagent layer 53 that is impregnated with the reagent are sandwiched between the cover 57 and the substrate 51, and the cover is arranged integrally with the substrate 51. The reagent layer 53 is formed by coating a reagent containing an enzyme, an electron acceptor, amino acid, sugar alcohol, a water-soluble polymer, and the like.

Openings that pass through the substrate 51, the spacer 55, and the cover 57, respectively, are a substrate opening 59, a spacer opening 60, and a cover opening 61. A film 54 is adhered to the face of the substrate 51 opposite the spacer 55 so as to cover the substrate opening 59. Further, three second openings 62 are provided. The second openings 62 are provided so as to pass through the substrate 51, the spacer 55, and the cover 57, respectively, and some or all of them are provided so as to coincide with each other.

The whole thickness of the biosensor 8 is 0.1 to 1 mm, the length of a short side thereof is 5 to 30 mm, and the length of a long axis thereof is 30 to 60 mm.

Next, detailed description about operation will be made.

### (Preparation)

First, a user inserts the slide sheet 6 into the main body 2. Next, similarly, the insertion holder 7 is inserted into the main body 2 by fitting. The insertion holder 7 is provided with the cutout 15, and this cutout 15 is provided so that the user may not mistake an insertion direction. Although the cutout was used herein, other shapes, such as a protrusion or plurality of cutouts or protrusions, which give asymmetry to the insertion holder 7, are available.

Next, the user inserts the biosensor 8 into the insertion holder 7 by means of fitting. The second openings 62 of the biosensor 8 are arranged so that they can fit on the protrusions 16 of the insertion holder 7. Since the second openings 62 are asymmetrically arranged in the biosensor 8, the user can insert the biosensor without mistaking the insertion direction. Referring to Fig. 6, the three second openings 62 are arranged. However, the invention is not limited thereto, and various arrangements that satisfy the above object can be used. The sectional shape of the openings can also be various shapes, such as circular, oblong, square, polygonal, and elliptical shapes, other than a partial cutout of a double circle. In a case where the arrangement and shape of the second openings 62 of the biosensor 8 are changed, it is needless to say that the number, arrangement, and shape of the protrusions 16 of the insertion holder 7, are suitably changed.

Subsequently, the user applies a skin so that a predetermined laser perforating spot may come to the substrate opening 59 on the film 54 of the biosensor 8. At this time, the protrusions 18 of the insertion holder 7 protrude by about 1 to 3 mm from the biosensor 8, and the protrusions will push the skin. This will stimulate the periphery of the predetermined laser perforating spot. For this reason, it is possible to mitigate the stimulus that the user feels during laser perforating.

### (Laser Perforating)

After the user applies the skin to the biosensor 8, thereby completing the preparation of laser perforating, the user pushes the laser operation button 5 to perform laser perforating. The laser device 9 oscillates a laser beam using a signal from the electric circuit 11 by the laser operation button 5, and the electric power of the battery 10. The oscillated laser beam is focused by the focusing lens 12, and perforates the user's skin.

As the wavelength of the laser beam, a wavelength with a high absorption coefficient of a skin is preferable, and a 3 µm band (photoexcitation laser or mid-infrared semiconductor laser of an erbium-doped medium), or a 9 to 10 µm band (discharge excitation laser or mid-infrared semiconductor laser of a carbon dioxide gas medium) is available.

Further, the focusing diameter of a laser beam at a skin is preferably φ0.5 mm or less, and is more preferably φ0.15 mm or less. Although the focusing shape is preferably a circular shape, an elliptical shape the length of a long axis of which is set to 0.5 mm or less is also available. The laser beam preferably oscillates in pulses and, the time width of the waveform of the pulses is preferably 1 µs or more and 400 µs or less. Irradiation of the laser beam can be irradiation of a single pulse beam or irradiation of multiple pulse beams.

As the focusing lens 12, focusing lenses obtained by performing non-reflective coating (dielectric multilayer film) on a substrate made of calcium fluoride (CaF2), yttrium aluminum garnet (YAG), germanium (Ge), zinc selenide (ZnSe), anhydrous synthetic quartz, and a fluoride mould material are available, and the focal distance thereof is preferably 5 to 30 mm. In this embodiment, a calcium-fluoride substrate was used. Further, the focusing lens 12 is substantially circular, and the outside dimension thereof is 6 to 30 mm.

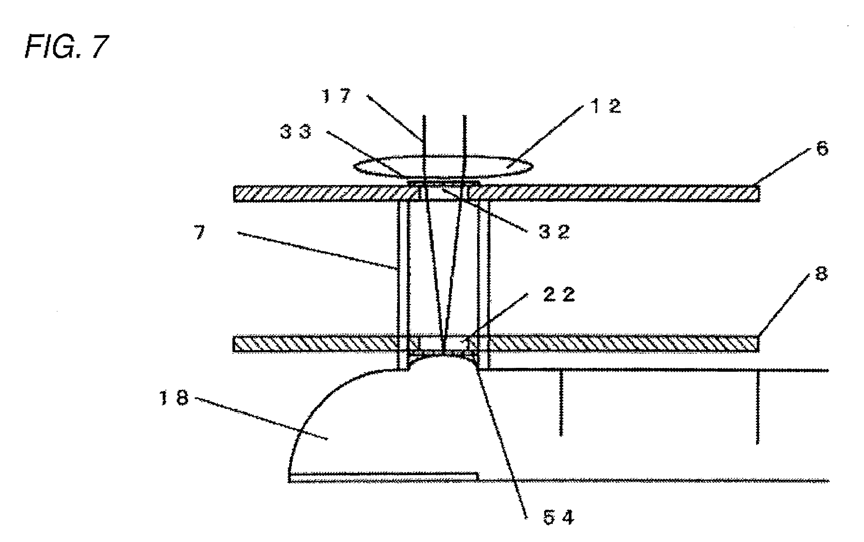

Fig. 7 is an enlarged view of a laser perforating spot during laser perforating. A laser beam is focused on a finger 18 of the user who performs laser perforating by the focusing lens 12, and the locus thereof is a locus 17 of a laser beam. The user's finger 18 touches the film 54 of the biosensor 8, and the laser beam 17 from the main body propagates while the beam diameter thereof is gradually tapered like Fig. 7. The laser beam 17 is set so as to propagate through the film 33 of the slide sheet 6, the opening 32, the inside of the insertion holder 7, the opening 22 of the biosensor 8, and a film 54, and to be focused on the user's finger 18.

The opening diameters of the openings 59, 60, and 61 that constitute the opening 32 of the slide sheet 6, the internal diameter of the insertion holder 7 and the opening 22 of the biosensor 8 may be such diameters that the laser beam 17 is not substantially shielded, and all the diameters are not necessarily the same. Further, the minimum diameter of the openings is 1 mm. Moreover, referring to Fig. 6, the openings are circular. However, various shapes, such as rectangular, square, polygonal, and elliptical shapes, are possible.

The laser beam 17 is partially absorbed by the film 33, but a through hole is not formed in the film, while the laser beam 17 is almost focused at the film 54, and the energy density thereof is high. Thus, the laser beam is absorbed by the film to heat and evaporate the film, thereby forming a through hole. Subsequently, the laser beam is absorbed by the skin of the user's finger 18 to heat and evaporate the skin, thereby eventually damaging a capillary vessels of a dermis to allow blood sampling.

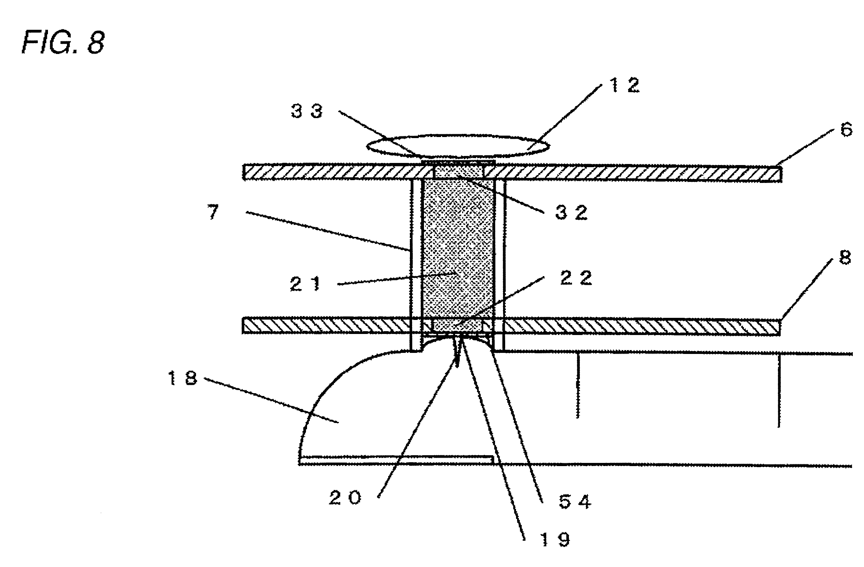

Fig. 8 is an enlarged view of the laser perforating spot immediately after the laser perforating. Since the laser perforating forms a through hole 19 in the film 54, and forms a laser-perforated hole 20 in the skin of the user's finger 18, plume 21 is generated as the tissue of the skin evaporates,

Like Fig. 8, the plume 21 that is generated as the tissue of the skin evaporates goes toward the opening 22 of the biosensor 8, the inside of the insertion holder 7, and the opening where the film 33 is formed, via the through hole 19 formed by irradiating the film 54 with a laser beam. Since the through hole 19 of the film 54 is extremely small similarly to the laser-perforated hole 20 of the skin even after the user lifts his/her finger 18 from the biosensor 8, the amount of the plume that flows back is extremely small, and consequently, the user can avoid smelling the odor of the plume 21.

The component concentration measuring apparatus of this embodiment in which the inside of a glove box where air tightness from the outside is secured is filled with nitrogen gas was used within the glove box to perform laser perforating. When the gas inside the glove box was evacuated into an evaluation box by an exhauster and was investigated a gas detector tube that detects a sulfide compound after the laser perforating, the level of detected quantity was equal even compared with a case where laser perforating is not performed.

As the base material of the film 54, polyamide, polyester, polyimide, fluorine system, vinyl chloride, polyethylene, polyolefin, polyvinyl alcohol, polypropylene, and the like are available. It is more preferable that the film 54 has a higher absorption coefficient with respect to the wavelength of the laser beam 17. In this embodiment, polycarbonate was used. Since not only the absorption coefficient of the base material but the thickness thereof and the absorption coefficient of an additive contribute to the absorption coefficient, it is preferable to adjust the thickness and the additive in order to properly adjust the absorption coefficient.

As the base material of the film 33, polyamide, polyester, polyimide, fluorine system, vinyl chloride, polyethylene, polyolefin, polyvinyl alcohol, polypropylene, and the like are available. In this embodiment, a cycloolefin polymer was used. It is more preferable that the film 33 has a lower absorption coefficient with respect to the wavelength of the laser beam 17. Since not only the absorption coefficient of the base material but the thickness thereof and the absorption coefficient of an additive contribute to the absorption coefficient, it is preferable to adjust the thickness and the additive in order to properly adjust the absorption coefficient.

The film 54, the film 33, and the inside of the insertion holder 7 also have a deodorizing function against abnormal odor components of the plume 21. Volatile substances that are generated as amino acids that are components of protein that constitutes the tissue of a skin are decomposed drift within the plume 21. A stratum corneum that is a main object during laser perforating is composed of the fibrous tissue of protein keratin, and fibers are bonded by cystine bonds that are bonds of a sulfur portion of amino acid cystine that is contained relatively much. As these amino acids are decomposed during evaporation, volatile sulfur compounds or nitrogen compounds are generated, and particularly, a person feels the sulfur compounds as an abnormal odor.

Hydrogen sulfides or methyl mercaptans are mentioned as main sulfur compounds. The hydrogen sulfides can be deodorized by a chemical adsorbent, and composite oxides of manganese, copper, and cobalt can be used. In addition, similarly, by using oxides, hydroxides, composite oxides, or its mixture, containing any of manganese, copper, zinc, and cobalt, an adsorbent that has a powerful chemical adsorbent action against the hydrogen sulfides can be obtained.

The chemical adsorbent chemically adsorbs the hydrogen sulfides finally in the form of sulfates or as simple sulfur substances. Further, the chemical adsorbent also has an intermediation action that converts methyl mercaptans that are the same sulfur-based odor into dimethyl disulfides having a higher threshold value. For this action, a person can feel an effect equivalent to deodorization. The chemical adsorbent has a size of about 0.1 to 1 µm in size, and the shape of the chemical adsorbent is not particularly limited.

Moreover, since the dimethyl disulfides can be removed by a physical adsorbent action by making a physical adsorbent carried, it is possible to remove the sulfur compounds in general. Hydrophobic zeolite having a large portion of silica can be used as the physical adsorbent. In addition, even if zeolite, sepiolite, silica, alumina, and the like are used, the same effect is obtained.

These chemical adsorbent and physical adsorbent can be implemented by being added to the films 33 and 54, or the base material of the insertion holder 7, or by being coated on the films 33 and 54 or the inside of the insertion holder 7. Further, it is also preferable to add and coat these adsorbents to/on the substrate 51, the spacer 55, or the cover 57.

Further, since the film 54 contacts the user's finger 18, it is preferable that antibacterial properties are given to the film. Various antibacterial agents can be used, and can be implemented by coating or kneading onto/into a base material. The antibacterial agents include Ag, Cu, Zn, Ni, Co or their alloys, TiO2, ZnO, WO3, and the like.

In addition, the antibacterial properties are useful by being implemented not only on the film 54 of the biosensor 8 but on the external surface of the biosensor 8, the insertion holder 7, and the slide sheet 6.

In this embodiment, an antibacterial agent in which silver is carried in zirconium phosphates was added to a coating material so as to be 0.5 wt%, and was then coated on a substrate. Furthermore, on the external surface of the substrate, the above antibacterial agent of 0.5 wt% was added to resin that constitutes the substrate. In addition, in both the processing methods, the antibacterial agent of 0.5 wt% to 1.0 Wt% has an antibacterial effect, and is economical.

The user detaches a skin from the biosensor 8 and pushes the vicinity of a perforating spot to squeeze out blood that is a specimen sample by 0.5 to several micro litters. Thereafter, by making a blood sampling spot touched with the specimen reagent supply passage 56 at a side of the biosensor 8, the air hole 58 acts, whereby blood is collected in the biosensor 8 by a capillary phenomenon. As for the blood, a corpuscle component in the blood is captured by the filter 52 on the way, and only a plasma component reaches the specimen reagent 53.

The filter 52 is made of a fibrous material, such as a glass fiber or a non-woven tissue, and is configured in a mesh shape. Depending on the interval between fibers, components in a liquid to be analyzed, having a size more than the interval, can be captured. The components to be captured are a component that hinders a reaction between an analyte in the specimen reagent 53, and a reagent, and a component that has a color except the analyte. The interval between fibers is about several micrometers to several tens of micrometers, and preferably 1 to 5 µm. The length in the direction of the specimen reagent supply passage 56 is about several millimeters, and is preferably 1 to 5 mm.

The enzyme and chromogen of the enzyme colorimetric method that are well-known to persons skilled in the art are set in the specimen reagent 53 in a state where they are coated, adhered, or printed on the substrate 51, or they are integrated with a filter paper, a film, and other carriers by coating, printing, impregnation, or kneading. The size of the specimen reagent 53 is preferably a square or rectangle whose one side is 3 mm or more.

For the blood that is a liquid to be analyzed that has reached the specimen reagent 53, in a case where glucose is given as an analyte, a reaction of glucose + oxygen + water - (glucose oxidase) → gluconic acid + hydrogen peroxide is taken place between the glucose and an enzyme in the specimen reagent 53, and further a reaction of hydrogen peroxide + chromogen - (peroxidase) → hydrogen peroxide + quinone-based pigment is taken place between the reaction production and a chromogen. By a series of the reactions, the specimen reagent 53 shows coloration corresponding to the glucose concentration that is an analyte.

Pyranose oxidase can also be used instead of the glucose oxytase. Instead of the peroxidase, hemoglobin, iron thiocyanate, iron ferrocyanide, potassium iodide, ammonium molybdate, and the like can be used.

The chromogen may be those having absorption in a visible region in the case of color development. Chromogens based on o-anisidine, benzidine, o-tolidine, tetramethyl benzidine, and leuko; Trinder-based reagents obtained by combining 4-amino antipyrine and phenol, or their derivatives and aniline derivatives; and the like can be used.

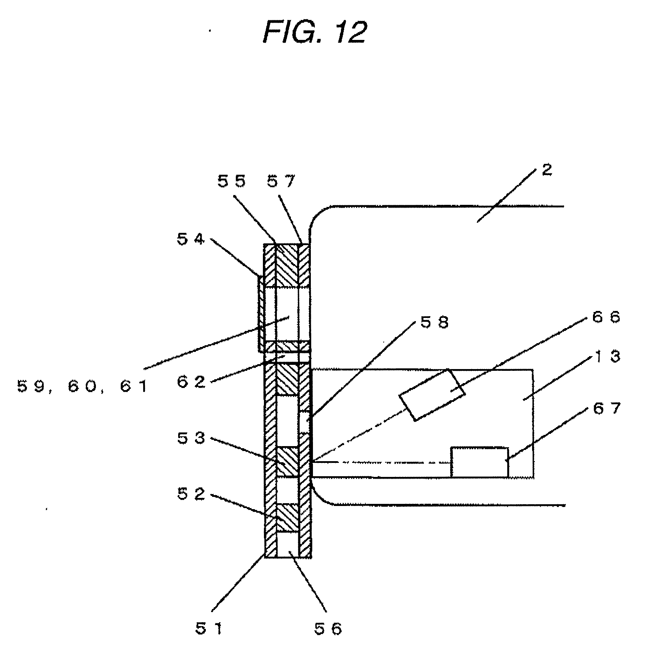

Fig. 12 is a configuration diagram of the analyzing optical device 13 for measuring the coloration state of the specimen reagent 53. The light of a light-emitting element 66 that is electrically connected with the electric circuit 11 passes through an opening provided in the main body 2, and enters the specimen reagent 53 via a cover 57 that is substantially transparent to the light of the light-emitting element 66 of the biosensor 8 substantially, and similarly, reflected light is detected by a light-receiving element 67 that is electrically connected with the electric circuit 11.

A window material that is transparent to the wavelength of the light emitted from the light-emitting element 66 for the purpose of preventing entering of dust or the like from the outside can also be set in the opening of the analyzing optical device 13 of the main body 2. As a window material, polycarbonate, polymethylmethacrylate, MS resin, polypropylene, ABS resin, polystyrene, epoxy resin, polyarete, polysulfone, polyethersulfone, nylon resin, polybutylene terephthalate, fluororesin, poly-4-methylpentene-1, phenoxy resin, polyimide resin, olefin/maleimide copolymers, phenol resin, non-bromine-based flame-resistant PC, cycloolefin polymers, methacrylic resin, triacetyl cellulose, and various glass materials, such as silicon oxide (SiO2) and BK7, can be used.

The degree of coloration is converted into a concentration depending on a reflecting ratio by a simple color of visible light or near-infrared light, or an intensity ratio by a plurality of beams of such light. As the light source, a combination of a lamp and a spectrum filter, a light-emitting diode (LED), and a laser diode (LD) can be used. In this embodiment, analysis was performed with the reflected light of the light-emitting diode having a wavelength of 610 nm, using glucose oxytase, an enzyme of peroxydase, and quinone pigment.

The concentration of a substance to be measured in the blood obtained by analysis is displayed on the display 3, and is recognized by the user. The electric circuit 11 further includes a memory unit that is not shown, and analysis results are recorded along with measurement date and time or a user code. The user can again recognize past measurement results afterward, using the display switching button 4.

As described above, the component concentration measuring apparatus according to the first embodiment is adapted such that a main body is provided with a laser device, a focusing lens, an optical device for determining color reactions of a biosensor, an electric circuit, a display capable of displaying analysis results, and a chargeable battery, a slide sheet and an insertion holder are inserted into the main body on the optical axis of a laser beam, and the biosensor is fitted into the insertion holder. Thereby, since a user does not smell an abnormal odor in a plume generated during laser perforating, and a test paper to be touched with a user's sampling spot is replaced at every sampling, there is no fear of infection, and since the user can perform sampling and analysis with one instrument, simple and easy use is achieved.

Further, a film to be perforated with a laser beam on the optical axis of a laser beam is provided in the biosensor that performs componential analysis by the color reaction of a specimen reagent. Thereby, a plume generated during laser perforating is discharged through the perforating hole from a user's skin, and the plume generated during laser perforating is discharged through the perforating from the user's skin. As a result, the plume does not leak toward the user, so that the user can be prevented from smelling an abnormal odor in the plume generated during laser perforating.

Moreover, a deodorizing function is provided on a film of the slide sheet on the optical axis of the laser beam, at least an inner surface of the insertion holder similarly on the optical axis of the laser beam, and a film of the biosensor similarly on the optical axis of the laser beam. Thereby, an abnormal odor in a plume generated during laser perforating can be eliminated.

### (Second Embodiment)

Hereinafter, a biosensor according to a second embodiment in the invention will be described in detail. In addition, only points that are different from the first embodiment will be described.

In the first embodiment, the user used the slide sheet 6 of Fig. 5 and the biosensor 8 of Fig. 6 that are different in structure. In this embodiment, the biosensor 8 of Fig. 6 is used as the slide sheet 6. The openings 59, 60, and 61 and film 54 of the biosensor 8 of Fig. 6 are arranged on the optical axis of a laser beam so as to correspond to the opening 32 and film 33 of a slide sheet 6 of Fig. 5.

During laser perforating, since the film 54 of the biosensor 8 to be used as the slide sheet 6 is on the side of the focusing lens rather than the focusing point of the focusing lens, the energy density of a laser beam on the film is lower, and since a through hole is not formed by the laser beam, the biosensor after being used as the slide sheet can be used again as a biosensor. Accordingly, since the user can reduce the number of types of consumable articles to manage, convenience can be improved.

### (Third Embodiment)

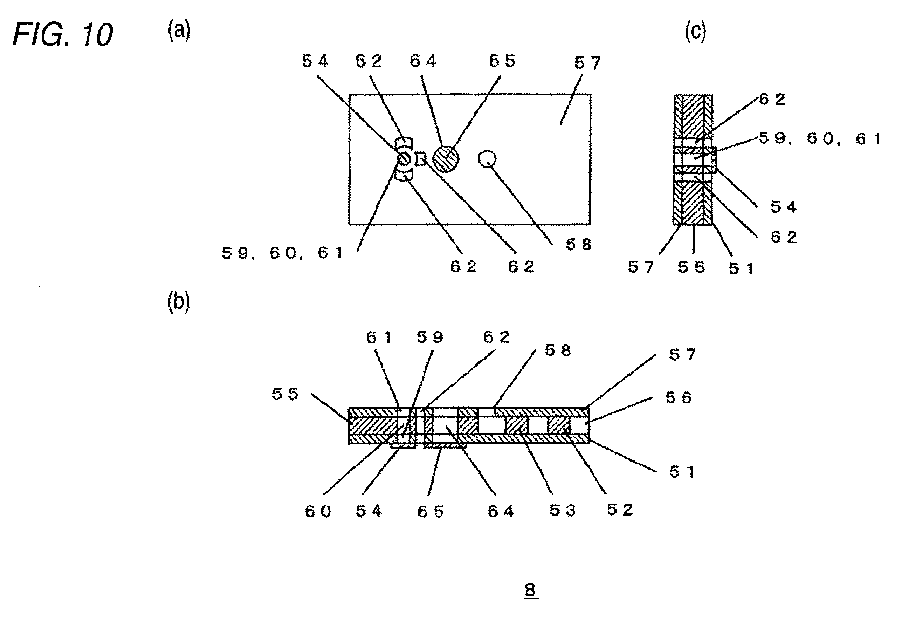

Hereinafter, a biosensor according to a third embodiment in the invention will be described in detail. In addition, only points that are different from the previous embodiments will be described. A biosensor is shown in Fig. 10. Fig. 10(a) is a plan view, Fig. 10(b) is a sectional view when being cut at a central portion in a longitudinal direction, and Fig. 10(c) is a sectional view when being cut in a central portion in a lateral direction. A third opening 64 and a film 65 when the biosensor is used as a slide sheet are further provided. When the biosensor is used as a slide sheet, the third opening 64 and film 65 are arranged on the optical axis of a laser beam, and the film 65 is configured so that a through hole may not be formed in the film even if laser beams are radiated onto the film multiple times.

As the film 65, various kinds of plastics or resins, such as polyamide, polyester, polyimide, fluorine system, vinyl chloride, polyethylene, polyolefin, polyvinyl alcohol, and polypropylene are available. Further, films obtained by performing non-reflective coating (dielectric multilayer film) on calcium fluoride (CaF2), yttrium aluminum garnet (YAG), germanium (Ge), zinc selenide (ZnSe), anhydrous synthetic quartz, and a fluoride mould material are also available. In this embodiment, a cycloolefin polymer was used.

It is more preferable that the film 65 has a lower absorption coefficient with respect to the wavelength of a laser beam. Since not only the absorption coefficient of the base material but the thickness thereof and the absorption coefficient of an additive contribute to the absorption coefficient, it is preferable to adjust the thickness and the additive in order to properly adjust the absorption coefficient. Further, similar to the first embodiment, it is preferable that a deodorizing function and antibacterial properties are given to the film 65.

Accordingly, a user who usually purchases 25 to 50 sheets of biosensors as one unit can use one of them only for a slide sheet. As a result, deterioration of analysis performance of a specimen reagent caused by exposing a biosensor to the air for a prolonged period of time is prevented, and it is not necessary to perform replacement of a slide sheet every time. Thus, user's convenience can be improved.

### (Fourth Embodiment)

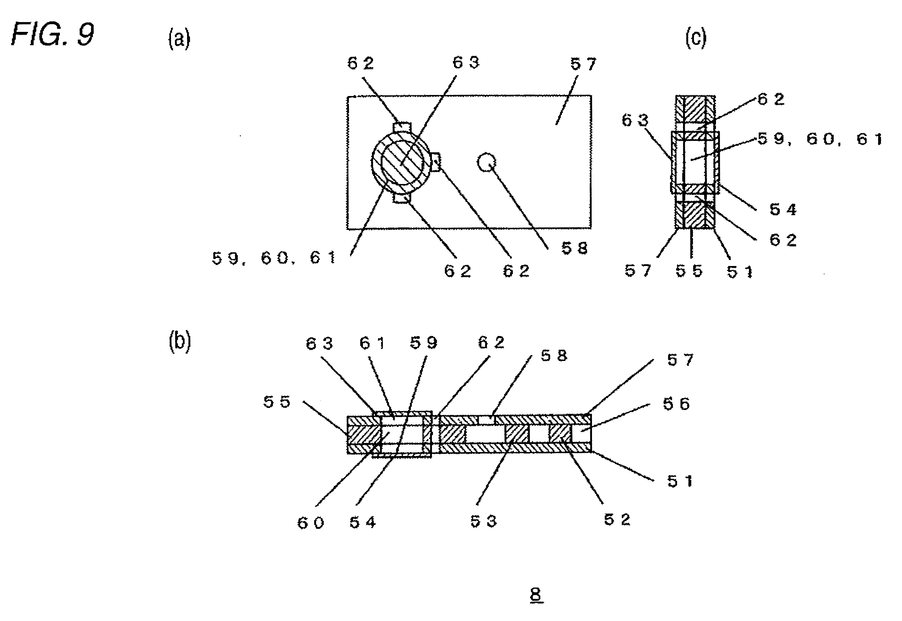

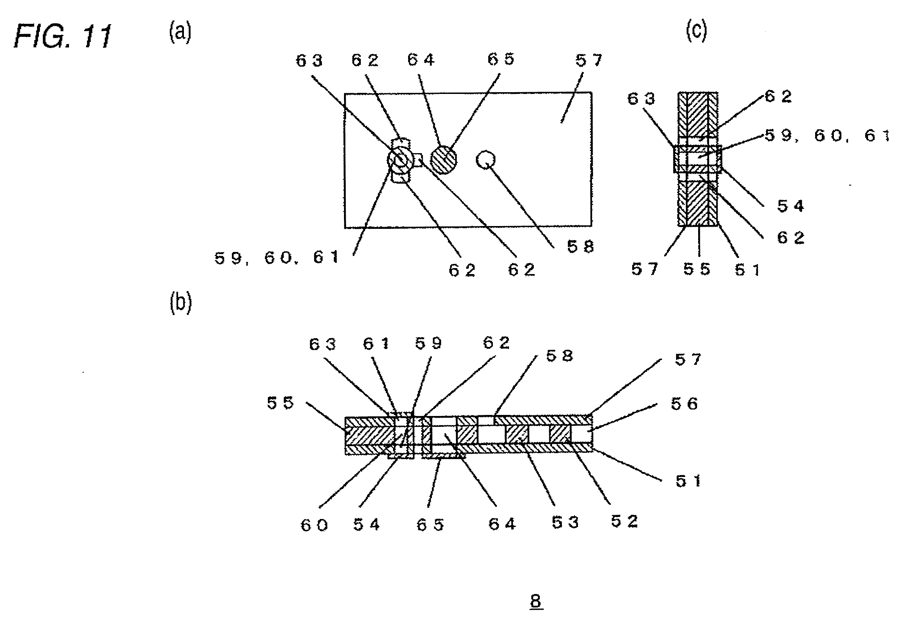

Hereinafter, a biosensor in a fourth embodiment according to the invention is shown in Figs. 9 and 11. Fig. 9 shows a biosensor obtained by giving changes to Fig. 6 that is the biosensor in the first embodiment. Fig. 9(a) is a plan view, Fig. 9(b) is a sectional view when being cut at a central portion in a longitudinal direction, and Fig. 9(c) is a sectional view when being cut in a central portion in a lateral direction. Further, Fig. 11 shows a biosensor obtained by giving changes to Fig. 10 that is the biosensor in the third embodiment. Fig. 11(a) is a plan view, Fig. 11(b) is a sectional view when being cut at a central portion in a longitudinal direction, and Fig. 11(c) is a sectional view when being cut in a central portion in a lateral direction. Hereinafter, only points that are different from the previous embodiments will be described.

In the biosensors of Figs. 9 and 11, in addition to the film 54 provided on one side of the openings 59, 60, and 61, a film 63 is further provided on the other side of the openings. As the film 63, various kinds of plastics or resins, such as polyamide, polyester, polyimide, fluorine system, vinyl chloride, polyethylene, polyolefin, polyvinyl alcohol, and polypropylene are available. Further, films obtained by performing non-reflective coating (dielectric multilayer film) on calcium fluoride (CaF2), yttrium aluminum garnet (YAG), germanium (Ge), zinc selenide (ZnSe), anhydrous synthetic quartz, and a fluoride mould material are also available. In this embodiment, a cycloolefin polymer was used. It is more preferable that the film 63 has a lower absorption coefficient with respect to the wavelength of a laser beam. Since not only the absorption coefficient of the base material but the thickness thereof and the absorption coefficient of an additive contribute to the absorption coefficient, it is preferable to adjust the thickness and the additive in order to properly adjust the absorption coefficient. Further, similar to the first embodiment, it is preferable that a deodorizing function and antibacterial properties are given to the film 63.

In the first embodiment, a plume containing abnormal odor components generated during laser perforating drifts into a space formed from the openings 59, 60, and 61 of the biosensor 8, the inside of the insertion holder 7, and the slide sheet 6, via a through hole to be formed in the film 54 that is on the side of a human body during laser perforating. By these deodorizing functions, a user is kept from smelling an odor of the plume.

In this embodiment, a plume containing abnormal odor components generated during laser perforating drifts into a space formed by the openings 59, 60, and 61 of the biosensor 8 and the film 63 in which a through hole is not formed during laser perforating, via a through hole to be formed in the film 54 of the biosensor 8 during laser perforating. The abnormal odor components are eliminated by deodorizing components of the film 54 and the film 63, thereby keeping a user from smelling an abnormal odor of the plume, so that convenience can be improved.

In addition, as the specimen sample, those sampled percutaneously, such as blood and interstitial fluid, can be used, and as the analyte, the blood glucose level (glucose concentration), or various kinds of serological or biochemical items can be used.

### (Fifth Embodiment)

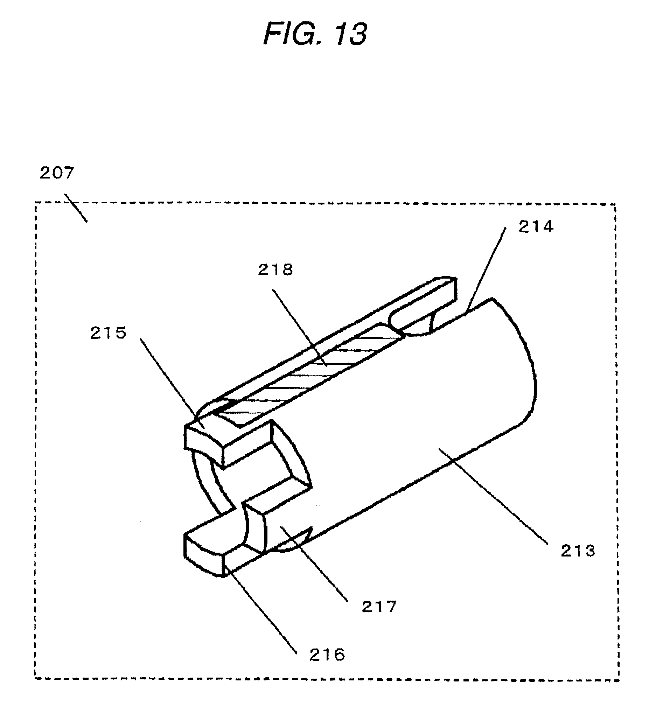

Hereinafter, a fifth embodiment according to the invention will be described. Fig. 13 is a detailed perspective view of an insertion holder 207. In Fig. 13, reference numeral 213 represents a cylindrical body, reference numeral 214 represents a cutout of one end of the cylindrical body 213, reference numeral 215 represents a first protrusion of one end of the cylindrical body 213, reference numeral 216 represents a second protrusion of one end of the cylindrical body 213, reference numeral 217 represents a third protrusion of one end of the cylindrical body 213, and reference numeral 218 represents a first electrode that extends toward the first protrusion 215 on an outer wall of the cylindrical body 213. Further, although not shown, a second electrode that extends toward the second protrusion 216 on the outer wall of the cylindrical body 213 is arranged.

As the cylindrical body 213, various plastic materials are available, and they are polyethylene, polyvinyl chloride, polypropylene, polystyrene, polyvinyl acetate, ABS resin, AS resin, acrylic resin, polyacetal, polyimide resin, polycarbonate, modified polyphenylene ether (PPE), polybutylene terephthalate (PPB), polyarylate, polysulfone, polyphenylene sulfide, polyether ether ketone, fluororesin, or the like. In this embodiment, AS resin was used. A material with low zeta potential on a plastic surface is preferable. The first electrode 218 and the second electrode may be made of a material having conductivity. In this embodiment, copper was used. Preferably, metallic materials, such as copper and aluminum, and materials in which metallic materials are subjected to metal plating and rust-preventive treatment are available.

The outside dimension of the insertion holder 207 may be within such a range that the biosensor 8 can be inserted, and is preferably 3 to 25 mm. Further, the wall thickness of the cylindrical body 213 is 0.2 to 3 mm, and the length thereof is 5 to 30 mm. In this embodiment, the insertion holder was formed in a cylindrical shape. However, a polyhedral tubular body or the like is available.

### (Biosensor)

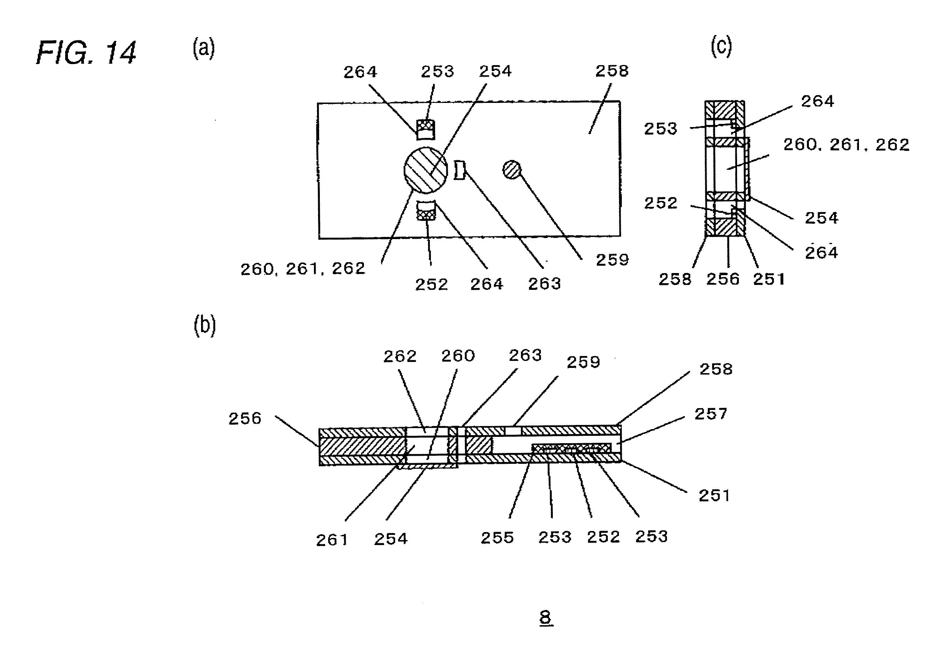

Fig. 14 is a detailed view of the biosensor 8 according to this embodiment. Fig. 14(a) is a plan view, Fig. 14(b) is a sectional view when being cut at a central portion in a longitudinal direction, and Fig. 14(c) is a sectional view when being cut in a central portion in a lateral direction.

Reference numeral 251 represents an insulating substrate made of polyethylene terephthalate or the like. On the surface of the substrate 251, an electrode couple 253 and a measuring electrode 252 that are made of noble metals, such as gold and palladium, or electrical conductive materials, such as carbon, are formed by a screen printing method or a sputtering vapor-deposition method.

Reference numeral 258 represents an insulating cover, and reference numeral 259 represents a substantially circular air hole. The material of the cover 258 is preferably a plastic film, and includes polyester, polyolefin, polyamide, polyether, polyamideimide, polystyrene, polycarbonate, poly-ρ-phenylene sulfide, polyvinyl chloride, and the like. In this embodiment, polyethylene terephthalate was used. Further, copolymers, blend materials, and bridged materials of them can be used for the cover 258, and the cover having a thickness of 0.01 mm to 0.5 mm can be used.

Also, a spacer 256 that has a cutout portion for forming a specimen reagent supply passage 257 that supplies a specimen reagent, and a reagent layer 255 that is impregnated with the reagent are sandwiched between the cover 258 and the substrate 251, and the cover is arranged integrally with the substrate 251.

The spacer 256 is arranged so as to cover the electrode couple 253 and the measuring electrode 252 on the substrate 251, the sample supply passage 257 is formed by an oblong cutout portion provided in the middle of a front edge of the spacer 256, and the reagent layer 255 is formed by coating a reagent containing an enzyme, an electron acceptor, amino acid, sugar alcohol, a water-soluble polymer, and the like on the electrode couple 253 and the measuring electrode 252 that are exposed from the cutout portion of the spacer 256.

Moreover, openings that pass through the substrate 251, the spacer 256, and the cover 258, respectively, are a substrate opening 260, a spacer opening 261, and a cover opening 262. Also, a film 254 is adhered to the face of the substrate 251 opposite the spacer 256 so as to cover the substrate opening 260. The whole thickness of the biosensor is 0.1 to 1 mm, the length of a short side thereof is 5 to 30 mm, and the length of a long axis thereof is 30 to 60 mm,

Further, a second opening 263 and third openings 264 are provided, and the measuring electrode 252 and the electrode couple 253 are exposed to portions in the openings 264. The second opening 263 and the third openings 264 are provided so as to pass through the substrate 251, the spacer 256, and the cover 258, respectively, and some or all of them are provided so as to coincide with each other.

Next, detailed description about operation will be made.

### (Preparation)

First, a user inserts the slide sheet 6 into the main body 2. Next, similarly, the insertion holder 207 is inserted into the main body 2 by fitting. The insertion holder 207 is provided with the cutout 214, and this cutout 214 is provided so that the user may not mistake an insertion direction. Although the cutout was used herein, other shapes, such as a protrusion or plurality of cutouts or protrusions, which give asymmetry to the insertion holder 207, are available.

Next, the user inserts the biosensor 8 into the insertion holder 207 by means of fitting. The second opening 263 and the third openings 264 of the biosensor 8 are arranged so that they can fit on the first protrusion 215, the second protrusion 216, and the third protrusion 217 of the insertion holder 207. Since the second opening 263 is asymmetrically arranged in the biosensor 8, the user can insert the biosensor without mistaking the insertion direction. Referring to Fig. 14, one second opening 263 is arranged. However, the invention is not limited thereto, and various arrangements that satisfy the above object can be used. The sectional shape of the third openings 264 and the second opening 263 can also be various shapes, such as circular, oblong, square, polygonal, and elliptical shapes, other than a partial cutout of a double circle. In a case where the arrangement and shape of the second opening 263 or third openings 264 of the biosensor 8 are changed, it is needless to say that the number, arrangement, and shape of protrusions including the first protrusion 215, the second protrusion 216, and the third protrusion 216 of the insertion holder 207, are suitably changed.

When the biosensor 8 is inserted into the insertion holder 207 by fitting, the measuring electrode 252 and the electrode couple 253 in the third openings 264 of the biosensor 8 will be respectively and electrically connected with the first electrode 218 and the second electrode (not shown) at the outer periphery of the cylindrical body 213 of the insertion holder 207. Furthermore, although not shown in Figs. 1 to 3, terminals for the first electrode 218 and second electrode are arranged on the side of the main body 2 side so that the first electrode 218 and second electrode of the insertion holder 207 that are inserted into the main body 2 by fitting may be electrically connected with the electric circuit 11 within the main body 2. If the biosensor 8 and the main body 2 are put into a mutually electrically connected state, the component concentration measuring apparatus 1 will be in a preparatory state of laser perforating and component concentration analysis from the electric circuit 11, and the fact that the apparatus has reached that state will be displayed on the display 3, thereby enabling a user to recognize the fact.

Subsequently, the user applies a skin so that a predetermined laser perforating spot may come to the substrate opening 260 on the film 254 of the biosensor 8. At this time, the protrusions, i.e., the first protrusion 215, the second protrusion 216, and the third protrusion 217 of the insertion holder 207 protrude by about 1 to 3 mm from the biosensor 8, and the protrusions will push the skin. This will stimulate the periphery of the predetermined laser perforating spot. For this reason, it is possible to mitigate the stimulus that the user feels during laser perforating.

### (Laser Perforating)

After the user applies the skin to the biosensor 8, thereby completing the preparation of laser perforating, the user pushes the laser operation button 5 to perform laser perforating. The laser device 9 oscillates a laser beam using a signal from the electric circuit 11 by the laser operation button 5, and the electric power of the battery 10. The oscillated laser beam is focused by the focusing lens 12, and perforates the user's skin.

As the wavelength of the laser beam, a wavelength with a high absorption coefficient of a skin is preferable, and a 3 µm band (photoexcitation laser or mid-infrared semiconductor laser of an erbium-doped medium), or a 9 to 10 µm band (discharge excitation laser or mid-infrared semiconductor laser of a carbon dioxide gas medium) is available. Further, the focusing diameter of a laser beam at a skin is preferably φ0.5 mm or less, and is more preferably φ0.15 mm or less. Although the focusing shape is preferably a circular shape, an elliptical shape the length of a long axis of which is set to 0.5 mm or less is also available. The laser beam preferably oscillates in pulses and, the time width of the waveform of the pulses is preferably 1 µs or more and 400 µs or less. Irradiation of the laser beam can be irradiation of a single pulse beam or irradiation of multiple pulse beams.

As the focusing lens 12, focusing lenses obtained by performing non-reflective coating (dielectric multilayer film) on a substrate made of calcium fluoride (CaF2), yttrium aluminum garnet (YAG), germanium (Ge), zinc selenide (ZnSe), anhydrous synthetic quartz, and a fluoride mould material are available, and the focal distance thereof is preferably 5 to 30 mm. In this embodiment, a calcium-fluoride substrate was used. Further, the focusing lens 12 is substantially circular, and the outside dimension thereof is 6 to 30 mm.

After the laser perforating, the user detaches a skin from the biosensor 8 and pushes the vicinity of a perforating spot to squeeze out blood that is a specimen sample by 0.5 to several micro litters. Thereafter, by making a blood sampling spot touched with the specimen reagent supply passage 257 at a side of the biosensor 8, the air hole 259 acts, whereby blood is collected in the biosensor 8 by the capillary phenomenon. As for the sampled blood, a reagent and a substance to be measured in the sampled blood react with each other in the reagent layer 255. As for charges generated in the reaction, the amount of the charges is analyzed by the electric circuit 11 via the measuring electrode 252, the electrode couple 253, and an electrode of the insertion holder 207. The concentration of the substance to be measured in the blood obtained by the analysis is displayed on the display 3, and is recognized by a user. The electric circuit 11 further includes a memory unit that is not shown, and analysis results are recorded along with measurement date and time or user codes. The user can again recognize past measurement results afterward, using the display switching button 4.

According to such a component concentration measuring apparatus of the embodiment of the invention, a main body is provided with a laser device, a focusing lens, an electric circuit capable of analyzing components, a display capable of displaying analysis results, and a chargeable battery, a slide sheet and an insertion holder are inserted into the main body on the optical axis of a laser beam, and the biosensor is fitted into the insertion holder. Thereby, since a user does not smell an abnormal odor in a plume generated during laser perforating, and a test paper to be touched with a user's sampling spot is replaced at every sampling, there is no fear of infection, and since the user can perform sampling and analysis with one instrument, simple and easy use is achieved.

Further, a film to be perforated by a laser beam on the optical axis of a laser beam is provided in the biosensor. Thereby, a plume generated during laser perforating is discharged through the perforating hole from a user's skin, and the plume generated during laser perforating is discharged through the perforating from the user's skin. As a result, the plume does not leak toward the user, so that the user can be prevented from smelling an abnormal odor in the plume generated during laser perforating.

Moreover, by providing an asymmetrical structure on the side of the insertion holder that is inserted into the main body, a user does not mistake the insertion direction, and thus, simple and easy use becomes possible.

Moreover, by providing a structure wherein the fitting between the biosensor and the insertion holder is performed by the fitting between an opening of the biosensor, and a protrusion of the insertion holder, a user does not mistake the fitting direction, and thus, simple and easy use becomes possible.

Moreover, by providing a structure wherein the fitting between the biosensor and the insertion holder is performed by the fitting between an opening of the biosensor, and a protrusion of the insertion holder, a user does not mistake the fitting direction, and slip-out hardly occurs. Thus, simple and easy use becomes possible.

Moreover, by providing a structure in which a measuring electrode and an electrode couple of the biosensor are electrically connected with individual electrodes provided on the surface of the insertion holder by the fitting between the biosensor and the insertion holder. Thereby, a user can perform component analysis without removing a test paper and remounting it for component analysis again during the component analysis of a body fluid sampled by laser perforating, and thus simple and easy use becomes possible.

Moreover, a deodorizing function is provided on a film of the slide sheet on the optical axis of a laser beam, at least an inner surface of the insertion holder similarly on the optical axis of the laser beam, and a film of the biosensor similarly on the optical axis of the laser beam. Thereby, an abnormal odor in a plume generated during laser perforating can be eliminated.

Moreover, by providing an antibacterial function in the biosensor, the insertion holder, and the slide sheet, any infection when used by a user can be prevented.

In addition, as the specimen sample, those sampled percutaneously, such as blood and interstitial fluid, can be used, and as the analyte, the blood glucose level (glucose concentration), or various kinds of serological or biochemical items can be used.

Further, in a case where the blood glucose level (glucose concentration) is used as an analyte as an example of those capable of being used as a reagent, employable combination of an enzyme, in some cases, coenzyme and a mediator includes: glucose oxidase + potassium ferricyanide or ferricynium; and glucose deoxidase + pyrroloquinoline quinone or nicotinamide adenine dinucleotide + phenanthroline quinone, osmium complex, or potassium ferricyanide.

### (Sixth Embodiment)

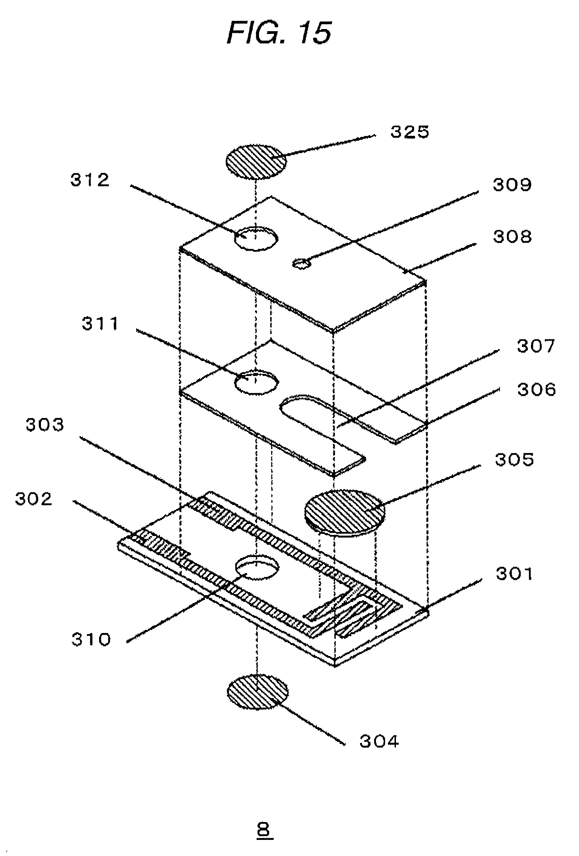

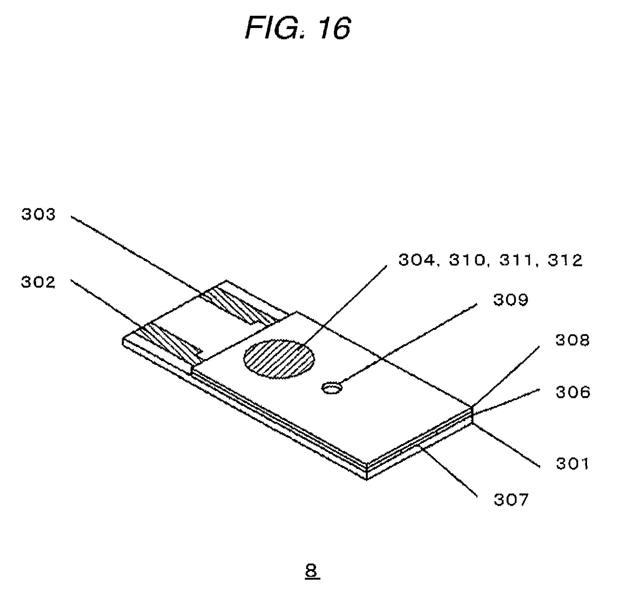

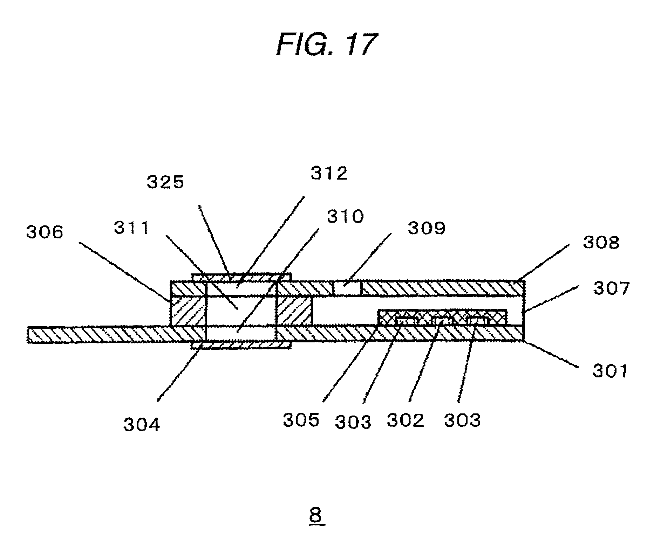

A biosensor for laser perforating of a sixth embodiment of the invention is shown in Figs. 15 to 17. Fig. 15 is an exploded perspective view of the biosensor, Fig. 16 is a general view of the biosensor, and Fig. 17 is a sectional view when the sensor of Fig. 16 is cut at a central portion in a longitudinal direction.

A substrate 301 is made of an insulating material including polyethylene terephthalate or the like. On the surface of the substrate 301, an electrode couple 303 and a measuring electrode 302 that are made of noble metals, such as gold and palladium, or electrical conductive materials, such as carbon, are formed by a screen printing method or a sputtering vapor-deposition method.

A cover 308 is made of an insulating material, and an air hole 309 is substantially circular. The material of the cover 308 is preferably a plastic film, and includes polyester, polyolefin, polyamide, polyether, polyamideimide, polystyrene, polycarbonate, poly-ρ-phenylene sulfide, polyvinyl chloride, and the like.

In this embodiment, polycarbonate was used.

Further, copolymers, blend materials, and bridged materials of them can be used for the cover 308, and the cover having a thickness of 0.01 mm to 0.5 mm can be used.

Also, a spacer 306 that has a cutout portion for forming a specimen reagent supply passage 307 that supplies a specimen reagent, and a reagent layer 305 that is impregnated with the reagent are sandwiched between the cover 308 and the substrate 301, and the cover is arranged integrally with the substrate 301.

The spacer 306 is arranged so as to cover the electrode couple 303 and the measuring electrode 302 on the substrate 301, the sample supply passage 307 is formed by an oblong cutout portion provided in the middle of a front edge of the spacer 306, and the reagent layer 305 is formed by coating a reagent containing an enzyme, an electron acceptor, amino acid, sugar alcohol, a water-soluble polymer, and the like on the electrode couple 303 and the measuring electrode 302 that are exposed from the cutout portion of the spacer 306.

Moreover, openings that pass through the substrate 301, the spacer 306, and the cover 308, respectively, are a substrate opening 310, a spacer opening 311, and a cover opening 312. Also, a film 304 is adhered to the face of the substrate 301 opposite the spacer 306 so as to cover the substrate opening 310. Further, a film 325 is adhered to the face of the cover 308 opposite the spacer 306 so as to cover the cover opening 312. The whole thickness of the biosensor is 0.1 to 1 mm, the length of a short side thereof is 5 to 30 mm, and the length of a long axis thereof is 30 to 60 mm.

Next, operation will be described in detail.

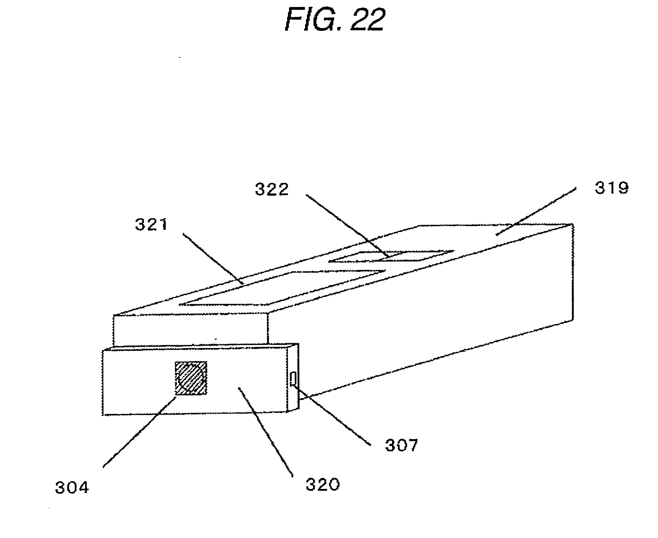

Fig. 22 is a view for explaining how to use. In Fig. 22, reference numeral 319 represents a main body, reference numeral 321 represents a display means on the main body 319, reference numeral 322 represents an operation means on the main body 319, and reference numeral 320 represents a biosensor for laser perforating (hereinafter sometimes referred to as a sensor). Inside the main body 319, a laser oscillator that is not shown, a focusing means of a laser beam oscillated from the laser oscillator, which is similarly not shown, and an electric board for operating and controlling the laser oscillator, which is similarly not shown, are arranged.

As the wavelength of the laser beam, a wavelength with a high absorption coefficient of a skin is preferable, and a 3 µm band, or a 9 to 10 µm band is available. Further, the focusing diameter of a laser beam at a skin is preferably φ0.5 mm or less, and is more preferably φ0.15 mm or less. Although the focusing shape is preferably a circular shape, an elliptical shape the length of a long axis of which is set to 0.5 mm or less is also available. The laser beam preferably oscillates in pulses and, the time width of the waveform of the pulses is preferably 1 µs or more and 400 µs or less. Irradiation of the laser beam can be irradiation of a single pulse beam or irradiation of multiple pulse beams.

Furthermore, although not shown, the main body 319 holds the biosensor for laser perforating. In the main body, a conversion circuit that applies a constant voltage to the biosensor for laser perforating, and converts a current corresponding to the concentration of an analyte in a specimen sample at this time into a voltage, an A-D converter that converts the converted voltage into a digital value, and a memory that records the measured value, and an electric board for operating and controlling these elements are arranged. Further, although not shown, a power source that supplies power to the above elements is arranged.

Measurement values, values stored in a memory, the state of an apparatus, date and time, and the like are displayed on the display means 321. The operation means 322 is, for example, a push button, and performs an oscillation operation of a laser beam, a start operation of measurement, display a switching operation of the display means 321, and the like.

A user arranges the sensor 320 so that the sensor may touch the whole surface of the main body 319. The user puts his/her finger on the sensor 320 so that a predetermined perforating spot may come to almost the center of the film 304 of the sensor 320. By operating the operation means 322 to oscillate a laser beam from the laser oscillator, perforating for blood sampling is performed on the skin of the finger. After the perforating, the user first detaches his/her finger from the sensor 320 and pushes the vicinity of a perforating spot to squeeze out blood that is a specimen sample by 0.5 to several micro litters. Thereafter, by making a blood sampling spot touched with the specimen reagent supply passage 307 at a side of the sensor 320, blood is collected in the sensor 320. Analysis results are displayed on the display means 321, and are recognized by the user.

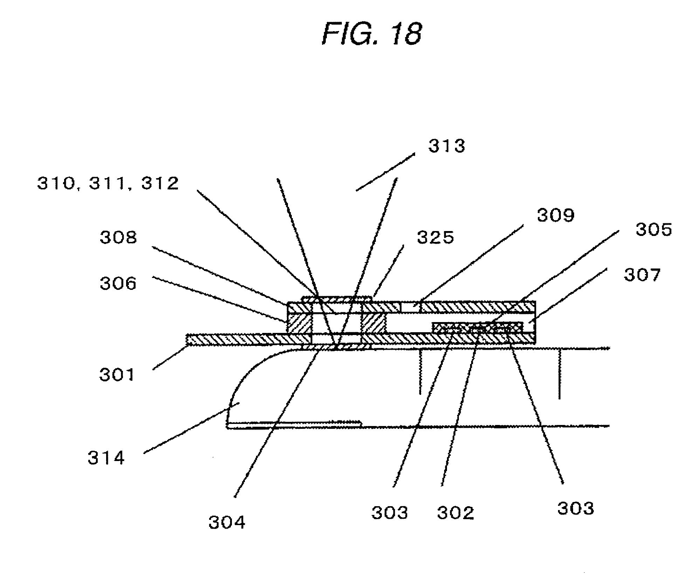

Fig. 18 is a view showing the relationship between the sensor, a finger, and a laser beam during use. In Fig. 18, a finger 314 of a user who performs laser perforating, and a locus 313 of a laser beam focused by the focusing means are shown. The user's finger 314 touches the film 304 of the sensor 320, and the laser beam 313 from the main body propagates while the beam diameter thereof is gradually tapered like Fig. 18. The laser beam 313 is set so as to propagate through the cover opening 312 of the sensor 320, the spacer opening 311, and the substrate opening 310, and to be focused on the user's finger 314.

The opening diameters of the openings 310, 311, and 312 may be such diameters that the laser beam 313 is not substantially shielded, and all the diameters are not necessarily the same. Further, the minimum diameter of the openings is 1 mm. Moreover, referring to Figs. 15 to 17, the openings are circular. However, various shapes, such as rectangular, square, polygonal, and elliptical shapes, are possible.

The laser beam 313 is partially absorbed by the film 325, but a through hole is not formed in the film, while the laser beam is focused at the film 304 to heat and evaporate the film, thereby forming a through hole. Subsequently, the laser beam is absorbed by the skin of the user's finger 314 to heat and evaporate the skin, thereby eventually damaging a capillary vessels of a dermis to allow blood sampling.

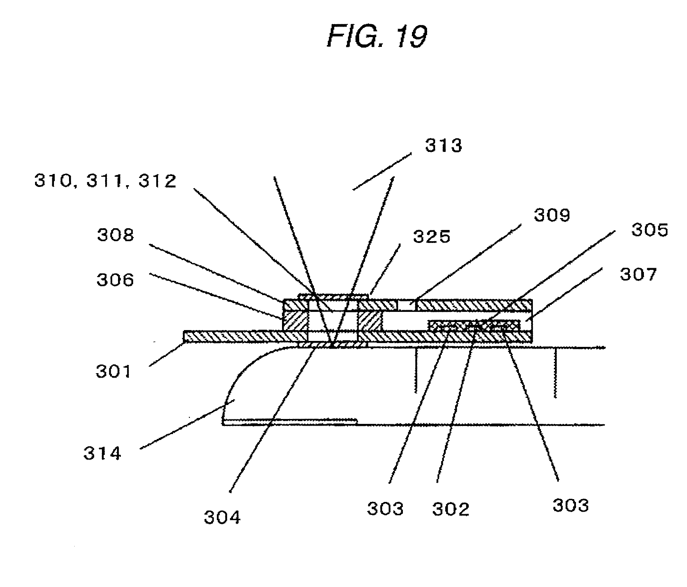

Fig. 19 is a view shown a situation during laser perforating in detail. In Fig. 19, a through hole 315 is formed in the film 304 with a laser beam. Similarly, a laser-perforated hole 316 is formed in the skin of the user's finger 314. A plume 317 is generated when the tissue of the skin evaporates during laser perforating.

Like Fig. 19, the plume 317 that is generated as the tissue of the skin evaporates goes toward the substrate opening 310, the spacer opening 311, the cover opening 312, and the opening where the film 325 is formed, via the through hole 315 formed by irradiating the film 304 with a laser beam. Since the through hole 315 of the film 304 is extremely small similarly to the laser-perforated hole 316 of the skin even after the user lifts his/her finger 314 from the sensor, the amount of the plume that flows back is extremely small, and consequently, the user can avoid smelling the odor of the plume 317.

As the base material of the film 304, nylon, polyester, polyimide, fluorine system, vinyl chloride, polystyrene, polyethylene, polyolefin, polyvinyl alcohol, polypropylene, and the like are available. In this embodiment, polyethylene was used.

It is more preferable that the film 304 has a higher absorption coefficient with respect to the wavelength of the laser beam 313. Since not only the absorption coefficient of the base material but the thickness thereof and the absorption coefficient of an additive contribute to the absorption coefficient, it is preferable to adjust the thickness and the additive in order to properly adjust the absorption coefficient.

As the base material of the film 325, nylon, polyester, polyimide, fluorine system, vinyl chloride, polystyrene, polyethylene, polyolefin, polyvinyl alcohol, polypropylene, and the like are available. In this embodiment, polystyrene was used.

It is more preferable that the film 325 has a lower absorption coefficient with respect to the wavelength of the laser beam 313. Since not only the absorption coefficient of the base material but the thickness thereof and the absorption coefficient of an additive contribute to the absorption coefficient, it is preferable to adjust the thickness and the additive in order to properly adjust the absorption coefficient.

The film 304 and the film 325 also have a deodorizing function against abnormal odor components of the plume 317. Volatile substances that are generated as amino acids that are components of protein that constitutes the tissue of a skin are decomposed drift within the plume 317. A stratum corneum that is a main object during laser perforating is composed of the fibrous tissue of protein keratin, and fibers are bonded by cystine bonds that are bonds of a sulfur portion of amino acid cystine that is contained relatively much. As these amino acids are decomposed during evaporation, volatile sulfur compounds or nitrogen compounds are generated, and particularly, a person feels the sulfur compounds as an abnormal odor.

Hydrogen sulfides or methyl mercaptans are mentioned as main sulfur compounds. The hydrogen sulfides can be deodorized by a chemical adsorbent, and composite oxides of manganese, copper, and cobalt can be used. In addition, by using oxides, hydroxides, composite oxides, or its mixture, which contain any of manganese, copper, zinc, and cobalt, similarly, an adsorbent that has a powerful chemical adsorbent action against the hydrogen sulfides can be obtained.

The chemical adsorbent chemically adsorbs the hydrogen sulfides finally in the form of sulfates or as simple sulfur substances. Further, the chemical adsorbent also has an intermediation action that converts methyl mercaptans that are the same sulfur-based odor into dimethyl disulfides having a higher threshold value. For this action, a person can feel an effect equivalent to deodorization. The chemical adsorbent has a size of about 0.1 to 1 µm in size, and the shape of the chemical adsorbent is not particularly limited.

Moreover, since the dimethyl disulfides can be removed by a physical adsorbent action by making a physical adsorbent carried, it is possible to remove the sulfur compounds in general. Hydrophobic zeolite having a large portion of silica can be used as the physical adsorbent. In addition, even if zeolite, sepiolite, silica, alumina, and the like are used, the same effect is obtained.

These chemical adsorbent and physical adsorbent can be implemented by being added to base materials of the films, or by being coated on the film. Further, it is also preferable to add and coat these adsorbents to/on the substrate.

Further, since the film 304 contacts the user's finger 314, it is preferable that antibacterial properties are given to the film. Various antibacterial agents can be used, and can be implemented by coating or kneading onto/into a base material. The antibacterial agents include Ag, Cu, Zn, Ni, Co or their alloys, TiO2, ZnO, WO3, and the like. In this embodiment, an antibacterial agent in which silver is carried in zirconium phosphates was kneaded into the film 304 so as to be 0.5 wt%.

According to such a biosensor for laser perforating of the sixth embodiment of the invention, films are provided in the sensor, and at one side thereof, and deodorizing function are given to the films. Thereby, a plume including an odor generated during laser perforating can be prevented from leaking to outside, and odor components can be eliminated.

### (Seventh Embodiment)

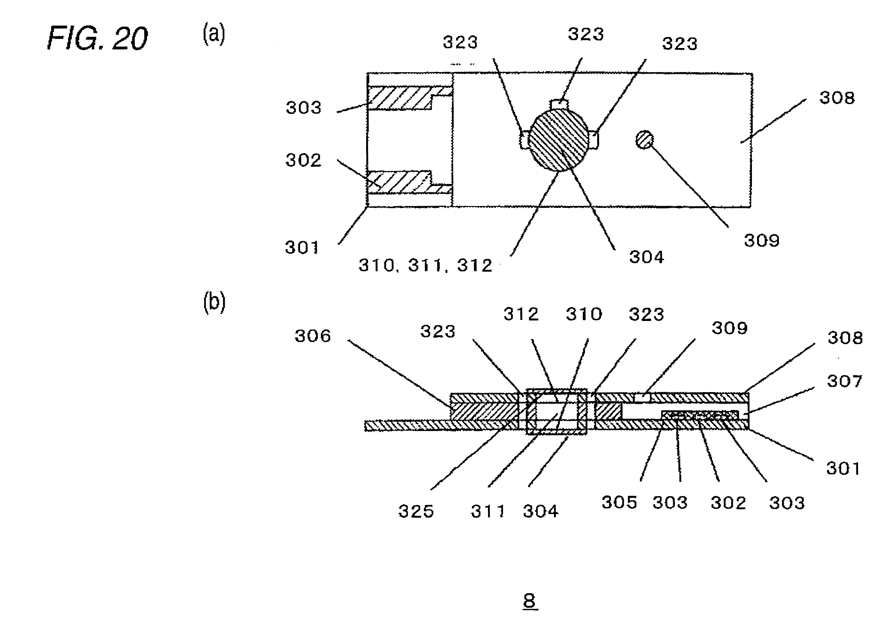

Next, a biosensor for laser perforating of a seventh embodiment of the invention is shown in Fig. 20. Fig. 20(a) is a plan view, and Fig. 20(b) is a sectional view when being cut at a central portion in a longitudinal direction of Fig. 20(a). The seventh embodiment is different from the sixth embodiment only in that three second openings 323 are newly provided. The second openings 323 are provided so as to pass through the substrate 301, the spacer 306, and the cover 308, respectively, and some or all of them are provided so as to coincide with each other.

The second openings 323 operate as follows. In the sixth embodiment, the sensor and the main body are connected together by contact. However, in the seventh embodiment, a protrusion is provided in the main body so as to coincide with the second openings 323. A user can insert the sensor into the protrusion, thereby connecting it with the main body. For this reason, the relative distance between the laser oscillator and the sensor can be determined, and the shape of a laser-perforated hole can be made stable.

Further, the second openings 323 are arranged in asymmetrical positions in the direction of a short side of the sensor. For this reason, a user can insert the biosensor without mistaking the insertion direction. Moreover, the protrusion of the main body that protrudes from the second openings 323 will stimulate the vicinity of a predetermined laser perforating spot when the user applies his/her finger to the sensor. For this reason, it is possible to mitigate the stimulus that the user feels during laser perforating.

In addition, referring to Fig. 20, the three second openings 323 are arranged. However, the invention is not limited thereto, and various arrangements that satisfy the above object can be used. The sectional shape of the second openings 323 can also be various shapes, such as circular, oblong, square, polygonal, and elliptical shapes, other than a partial cutout of a double circle.

As described above, according to the biosensor for laser perforating of the seventh embodiment of the invention, new openings are provided apart from the opening covered with the filter. Thereby, the relative distance between the laser oscillator and the sensor can be determined, and the shape of a laser-perforated hole can be made stable. Furthermore, the connection direction between the sensor and the main body can be made exact, and a stimulus during laser perforating can be mitigated depending on a stimulus caused by the protrusion that protrudes from the opening.

### (Eighth embodiment)

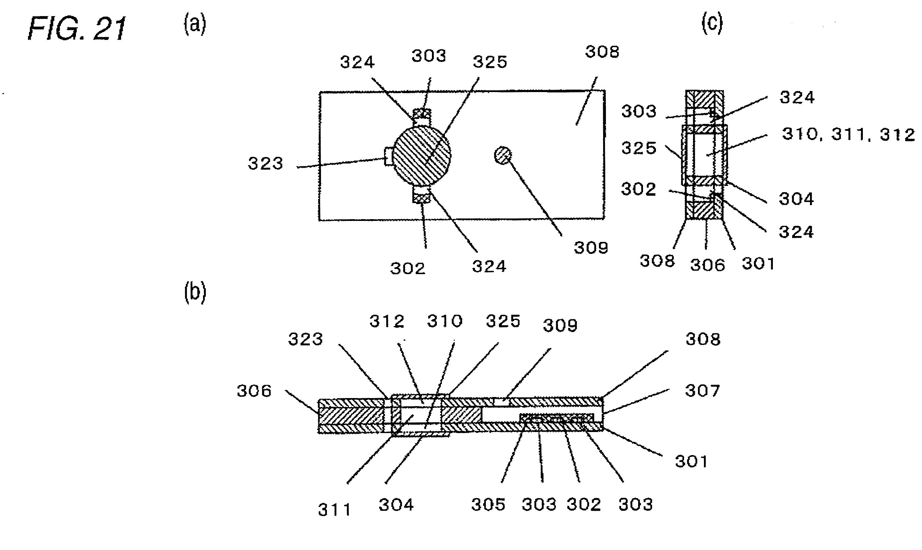

Next, a biosensor for laser perforating of an eighth embodiment of the invention is shown in Fig. 21. Fig. 21(a) is a plan view, Fig. 21(b) is a sectional view when being cut at a central portion in a longitudinal direction of Fig. 21(a), and Fig. 21(c) is a sectional view when being cut in a central portion in a lateral direction of Fig. 21(a).

The eighth embodiment is different from the sixth embodiment in that a second opening 323 and third openings 324 are newly provided, and the measuring electrode 302 and the electrode couple 303 that are have been exposed to the surface have moved into the openings 324, and is different from the seventh embodiment in that two of the second openings 323 become the third openings 324, and the measuring electrode 302 and the electrode couple 303 that have been exposed to the surface are provided so as to be exposed into the third openings 324, respectively. The second opening 323 and the third openings 324 are provided so as to pass through the substrate 301, the spacer 306, and the cover 308, respectively, and some or all of them are provided so as to coincide with each other.

The second opening 323 and the third openings 324 operate as follows. In the sixth embodiment, the sensor and the main body are connected together by contact. However, in the eighth embodiment, protrusions are provided in the main body so as to coincide with the second opening 323 and the third openings 324. In the protrusions of them that coincide with the third openings 324, electrodes for electrically analyzing the concentration of an analyte in a specimen sample within the main body extend. Thereby, a user can insert the sensor into the protrusions, thereby electrically and mechanically connecting it with the main body. For this reason, the relative distance between the laser oscillator and the sensor can be determined, and the shape of a laser-perforated hole can be made stable. Further, the insertion can be electrically detected, and a user is enabled to recognize an insertion error. Thus, the user's convenience for use improves.

Further, the second opening 323 and the third openings 324 are arranged in asymmetrical positions in the direction of a short side of the sensor. For this reason, a user can insert the biosensor without mistaking the insertion direction. Moreover, the protrusions of the main body that protrudes from the second opening 323 and the third openings 324 will stimulate the vicinity of a predetermined laser perforating spot when the user applies his/her finger to the sensor. For this reason, it is possible to mitigate the stimulus that the user feels during laser perforating.

In addition, referring to Fig. 21, the second opening 323 and the third openings 324 are arranged in a total of three spots. However, the invention is not limited thereto, and various arrangements that satisfy the above object can be used. The sectional shape of the second opening 323 and the third openings 324 can also be various shapes, such as circular, oblong, square, polygonal, and elliptical shapes, other than a partial cutout of a double circle.

As described above, according to the biosensor for laser perforating of the eighth embodiment of the invention, new openings are provided apart from the opening covered with the filter, and analyzing electrodes are exposed into the openings. Thereby, the relative distance between the laser oscillator and the sensor can be determined, and the shape of a laser-perforated hole can be made stable. Further, since the state of the insertion can be electrically detected and confirmed, a user is enabled to recognize an insertion error. Furthermore, the connection direction between the sensor and the main body can be made exact, and a stimulus during laser perforating can be mitigated depending on a stimulus created by the protrusions that protrudes from the openings.

In addition, as the specimen sample, those sampled percutaneously, such as blood and interstitial fluid, can be used, and as the analyte, the blood glucose level (glucose concentration), or various kinds of serological or biochemical items can be used.

Further, in a case where the blood glucose level (glucose concentration) is used as an analyte as an example of those capable of being used as a reagent, employable combination of an enzyme, in some cases, coenzyme and a mediator includes: glucose oxidase + potassium ferricyanide or ferricynium; and glucose deoxidase + pyrroloquinoline quinone or nicotinamide adenine dinucleotide + phenanthroline quinone, osmium complex, or potassium ferricyanide.

### (Ninth Embodiment)

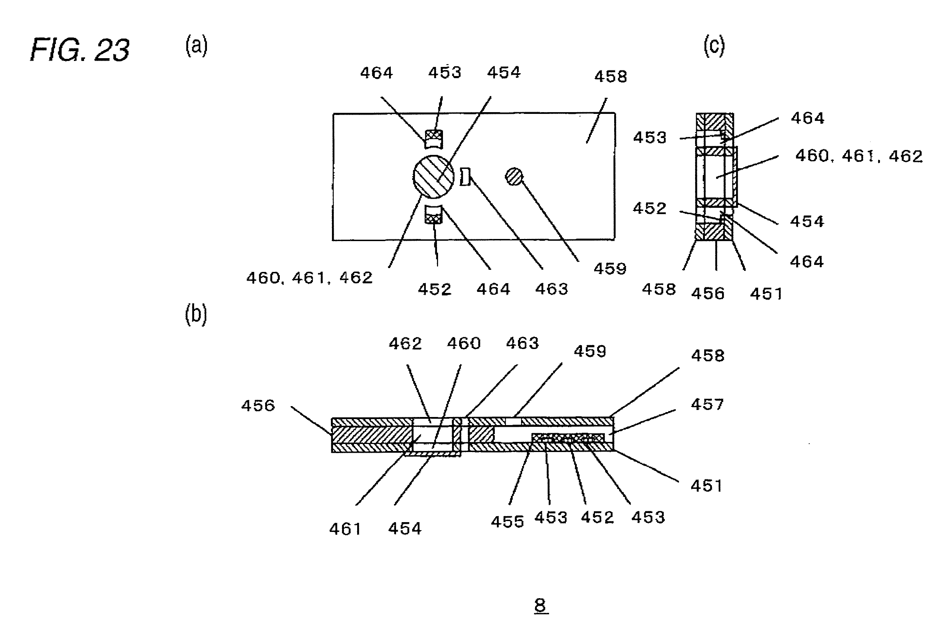

Fig. 23 is a detailed view of the biosensor 8 according to this embodiment. Fig. 23(a) is a plan view, Fig. 23(b) is a sectional view when being cut at a central portion in a longitudinal direction, and Fig. 23(c) is a sectional view when being cut in a central portion in a lateral direction.

On the surface of a substrate 451 in which polyethylene terephthalate, polyester, polyolefin, polyamide, polyether, polyamideimide, polystyrene, polycarbonate, poly-ρ-phenylene sulfide, polyvinyl chloride, ant the like are available, and polyethylene terephthalate is used in this embodiment, an electrode couple 453 and a measuring electrode 452 that are made of noble metals, such as gold and palladium, or electrical conductive materials, such as carbon, are formed by a screen printing method or a sputtering vapor-deposition method.

An insulating cover 458 has a substantially circular air hole 459. The material of the cover 458 is preferably a plastic film, and includes polyester, polyolefin, polyamide, polyether, polyamideimide, polystyrene, polycarbonate, poly- p-phenylene sulfide, polyvinyl chloride, and the like. In this embodiment, polyethylene terephthalate was used. Further, copolymers, blend materials, and bridged materials of them can be used for the cover 458, and the cover having a thickness of 0.01 mm to 0.5 mm can be used.

Also, a spacer 456 that has a cutout portion for forming a specimen reagent supply passage 457 that supplies a specimen reagent, and a reagent layer 455 that is impregnated with the reagent are sandwiched between the cover 458 and the substrate 451, and the cover is arranged integrally with the substrate 451.

The spacer 456 is arranged so as to cover the electrode couple 453 and the measuring electrode 452 on the substrate 451, the sample supply passage 457 is formed by an oblong cutout portion provided in the middle of a front edge of the spacer 456, and the reagent layer 455 is formed by coating a reagent containing an enzyme, an electron acceptor, amino acid, sugar alcohol, a water-soluble polymer, and the like on the electrode couple 453 and the measuring electrode 452 that are exposed from the cutout portion of the spacer 456.

Moreover, openings that pass through the substrate 451, the spacer 456, and the cover 458, respectively, are a substrate opening 460, a spacer opening 461, and a cover opening 462. Also, a film 454 is adhered to the face of the substrate 451 opposite the spacer 456 so as to cover the substrate opening 460. The whole thickness of the biosensor is 0.1 to 1 mm, the length of a short side thereof is 5 to 30 mm, and the length of a long axis thereof is 30 to 60 mm.

Further, a second opening 463 and third openings 464 are provided, and the measuring electrode 452 and the electrode couple 453 are exposed to portions in the openings 464. The second opening 463 and the third openings 464 are provided so as to pass through the substrate 451, the spacer 456, and the cover 458, respectively, and some or all of them are provided so as to coincide with each other.

Next, detailed description about operation will be made.

### (Mounting of Slide Sheet)

First, a user inserts the slide sheet 6 into the main body 2. In the invention, the biosensor 8 can be used as the slide sheet 6. By the openings 460, 461, and 462 and film 454 of the biosensor 23 of Fig. 23, the function to prevent any contamination (clouding) of the focusing lens 12 caused by an evaporant during laser perforating, which is the object of the slide sheet 6, is exhibited. Therefore, since a user does not need to prepare the slide sheet 6 separately from the biosensor 8 indispensable to component concentration measurement, user's convenience can be improved.

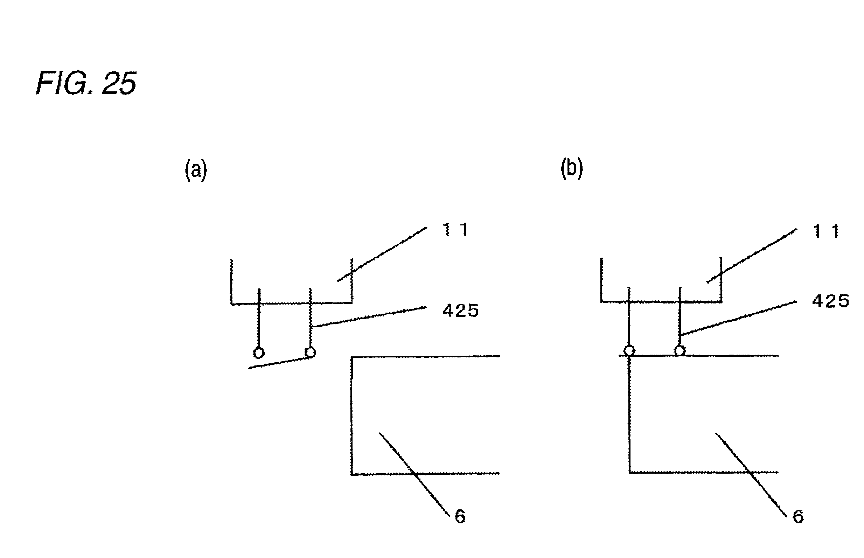

Fig. 25 is a schematic configuration diagram for allows the main body 2 to recognize the mounting of the slide sheet 6. A switch 425 that is electrically connected with the electric circuit 11 within the main body 2 is configured, and the switch 425 is open when the slide sheet 6 is not inserted ((a) of Fig. 25). When the slide sheet 6 is inserted into the main body 2, the switch 425 is brought into a closed state, and consequently, the electric circuit 11 can recognize the insertion of the slide sheet 6 ((b) of Fig. 25).

### (Tenth Embodiment)

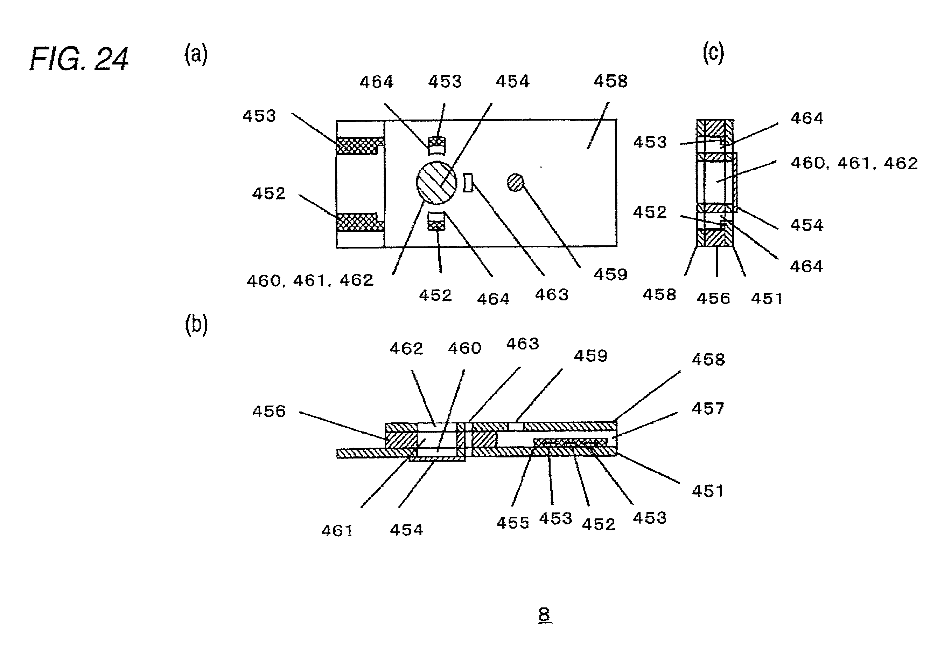

Now, a method of recognizing the mounting of one slide sheet 6 is a method using an electrode on the slide sheet 6. Fig. 24 shows a biosensor obtained by modifying the same slide sheet 6 as the biosensor 8 of Fig. 23. Fig. 24(a) is a plan view, Fig. 24(b) is a sectional view when being cut at a central portion in a longitudinal direction, and Fig. 24(c) is a sectional view when being cut in a central portion in a lateral direction. This biosensor is different from that of Fig. 23 in that the cover 458 and the spacer 456 are configured so that the measuring electrode 452 and the electrode couple 453 may be exposed at the end of the biosensor. Although not shown, the electrical connection between the electric circuit 11 inside the main body 2, and the measuring electrode 452 and the electrode couple 453 are established by insertion of the slide sheet 6. Accordingly, the insertion of the slide sheet 6 can be detected. Although Fig. 24 shows a configuration in which the measuring electrode 452 and the electrode couple 453 are used, electrodes independent from the measuring electrode 452 and the electrode couple 453 may be configured for detection of insertion.

### (Mounting of Insertion Holder)

Next, similarly, the insertion holder 207 (refer to Fig. 13) is inserted into the main body 2 by fitting. The insertion holder 207 is provided with the cutout 214, and this cutout 214 is provided so that the user may not mistake an insertion direction. Although the cutout was used herein, other shapes, such as a protrusion or plurality of cutouts or protrusions, which give asymmetry to the insertion holder 207, are available.

### (Mounting of Biosensor)

Next, the user inserts the biosensor 8 into the insertion holder 207 by means of fitting. The second opening 463 and the third openings 464 of the biosensor 8 are arranged so that they can fit on the first protrusion 215, the second protrusion 216, and the third protrusion 217 of the insertion holder 207. Since the second opening 463 is asymmetrically arranged in the biosensor 8, the user can insert the biosensor without mistaking the insertion direction. Referring to Fig. 23, one second opening 463 is arranged. However, the invention is not limited thereto, and various arrangements that satisfy the above object can be used. The sectional shape of the third openings 464 and the second opening 463 can also be various shapes, such as circular, oblong, square, polygonal, and elliptical shapes, other than a partial cutout of a double circle. In a case where the arrangement and shape of the second opening 463 or third openings 464 of the biosensor 8 are changed, it is needless to say that the number, arrangement, and shape of protrusions including the first protrusion 215, the second protrusion 216, and the third protrusion 216 of the insertion holder 207, are suitably changed.

When the biosensor 8 is inserted into the insertion holder 207 by fitting, the measuring electrode 452 and the electrode couple 453 in the third openings 464 of the biosensor 8 will be respectively and electrically connected with the first electrode 218 and the second electrode (not shown) at the outer periphery of the cylindrical body 213 of the insertion holder 207. Furthermore, although not shown in Figs. 1 to 3, terminals for the first electrode 218 and second electrode are arranged on the side of the main body 2 side so that the first electrode 218 and second electrode of the insertion holder 207 that are inserted into the main body 2 by fitting may be electrically connected with the electric circuit 11 within the main body 2. If the biosensor 8 and the main body 2 are put into a mutually electrically connected state, the component concentration measuring apparatus 1 will be in a preparatory state of laser perforating and component concentration analysis from the electric circuit 11, and the fact that the apparatus has reached that state will be displayed on the display 3, thereby enabling a user to recognize the fact.

Subsequently, the user applies a skin so that a predetermined laser perforating spot may come to the substrate opening 460 on the film 454 of the biosensor 8. At this time, the protrusion of the insertion holder 207, the first protrusion 215, the second protrusion 216, and the third protrusion 217 protrude by about 1 to 3 mm from the biosensor 8, and the protrusions will push the skin. This will stimulate the periphery of the predetermined laser perforating spot. For this reason, it is possible to mitigate the stimulus that the user feels during laser perforating.

### (Laser Perforating)

After the user applies the skin to the biosensor 8, thereby completing the preparation of laser perforating, the user pushes the laser operation button 5 to perform laser perforating. The laser device 9 oscillates a laser beam using a signal from the electric circuit 11 by the laser operation button 5, and the electric power of the battery 10. The oscillated laser beam is focused by the focusing lens 12, and perforates the user's skin.

As the wavelength of the laser beam, a wavelength with a high absorption coefficient of a skin is preferable, and a 3 µm band (photoexcitation laser or mid-infrared semiconductor laser of an erbium-doped medium), or a 9 to 10 µm band (discharge excitation laser or mid-infrared semiconductor laser of a carbon dioxide gas medium) is available. Further, the focusing diameter of a laser beam at a skin is preferably φ0.5 mm or less, and is more preferably φ0.15 mm or less. Although the focusing shape is preferably a circular shape, an elliptical shape the length of a long axis of which is set to 0.5 mm or less is also available. The laser beam preferably oscillates in pulses and, the time width of the waveform of the pulses is preferably 1 µs or more and 400 µs or less. Irradiation of the laser beam can be irradiation of a single pulse beam or irradiation of multiple pulse beams.

As the focusing lens 12, focusing lenses obtained by performing non-reflective coating (dielectric multilayer film) on a substrate made of calcium fluoride (CaF2), yttrium aluminum garnet (YAG), germanium (Ge), zinc selenide (ZnSe), anhydrous synthetic quartz, and a fluoride mould material are available, and the focal distance thereof is preferably 5 to 30 mm. In this embodiment, a calcium-fluoride substrate was used. Further, the focusing lens 12 is substantially circular, and the outside dimension thereof is 6 to 30 mm.

### (Measurement of Component Concentration)

After the laser perforating, the user detaches a skin from the biosensor 8 and pushes the vicinity of a perforating spot to squeeze out blood that is a specimen sample by 0.5 to several micro litters. Thereafter, by making a blood sampling spot touched with the specimen reagent supply passage 457 at a side of the biosensor 8, the air hole 459 acts, whereby blood is collected in the biosensor 8 by the capillary phenomenon. As for the sampled blood, a reagent and a substance to be measured in the sampled blood react with each other in the reagent layer 455. As for charges generated in the reaction, the amount of the charges is analyzed by the electric circuit 11 via the measuring electrode 452, the electrode couple 453, and an electrode of the insertion holder 207. The concentration of the substance to be measured in the blood obtained by the analysis is displayed on the display 3, and is recognized by a user. The electric circuit 11 further includes a memory unit that is not shown, and analysis results are recorded along with measurement date and time or user codes. The user can again recognize past measurement results afterward, using the display switching button 4.

In this embodiment, after the biosensor 8 was first used as the slide sheet 6, the biosensor that was used as the slide sheet 6 in the previous measurement was used as the biosensor 8 in the next component concentration measurement. Further, since a user replaces biosensors at every component concentration measurement and performs the measurement several times on a day, the user prepares biosensors 8 in units of several tens of sheets. Thus, it is also useful to use one of several tens of biosensors only for an slide sheet.

### (Eleventh Embodiment)

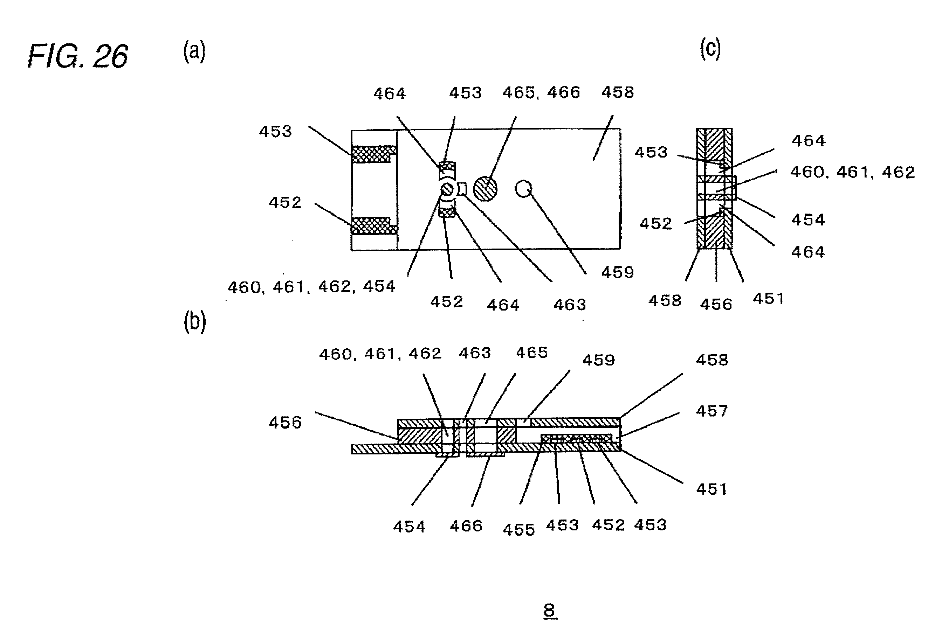

Moreover, it is also possible to provide a biosensor with separate opening and film for a slide sheet like Fig. 26. Fig. 26(a) is a plan view, Fig. 26(b) is a sectional view when being cut at a central portion in a longitudinal direction, and Fig. 26(c) is a sectional view when being cut in a central portion in a lateral direction. This biosensor is different from the biosensors of Figs. 23 and 24 in which openings 465 passing through the substrate 451, the spacer 456, and the cover 458, and a film 466 is disposed. The openings 465 may have such a diameter that a laser beam is not substantially shielded, and the minimum diameter thereof is 1 mm. Moreover, referring to Fig. 26, the openings are circular. However, various shapes, such as rectangular, square, polygonal, and elliptical shapes, are possible. Although the shapes of the openings 465 in the substrate 451, the spacer 456, and the cover 458 are the same, they may be different shapes such that a laser beam is not substantially shielded.

The film 466 was selected from the same base material group as the film 454. In this embodiment, a cycloolefin polymer was used. It is desirable to increase the transmission property of the film 466 to a laser beam so that a through hole may not be formed therein particularly even if the film is irradiated with the laser beam several times. Moreover, similar to the previous film 454, it is needless to say that that it is useful to give a deodorizing function and antibacterial properties to the film 466.

According to such a component concentration measuring apparatus of the embodiment of the invention, a main body is provided with a laser device, a focusing lens, an electric circuit capable of analyzing components, a display capable of displaying analysis results, and a chargeable battery, a slide sheet and an insertion holder are inserted into the main body on the optical axis of a laser beam, the biosensor is fitted into the insertion holder, and the slide sheet is also enabled to be used as the biosensor. Thereby, since a user does not smell an abnormal odor in a plume generated during laser perforating, and there is also no contamination of optical components, which are focusing means, by a plume, it is possible to achieve simple and easy use that the user can manage a sheet and a test paper with one kind of consumable articles.

Further, a film that is not perforated with a laser beam when used as a slide sheet and that is perforated with a laser beam when used as a biosensor is provided on the optical axis of the laser beam in a slide sheet that also has the function of a biosensor. Thereby, since a user does not smell an abnormal odor in a plume generated during laser perforating, and there is also no contamination of optical components, which are focusing means, by a plume, it is possible to achieve simple and easy use that the user can manage a sheet and a sensor with one kind of consumable articles.

Moreover, a film that is not perforated with a laser beam on the optical axis of the laser beam when used as a biosensor and a film that is not perforated with a laser beam on the optical axis of the laser beam when used as a slide sheet are provided in a slide sheet that also has the function of a biosensor. Thereby, since a user does not smell an abnormal odor in a plume generated during laser perforating, and there is also no contamination of optical components, which are focusing means, by a plume, it is possible to achieve simple and easy use that the user can manage a sheet and a test paper with one kind of consumable articles.

Moreover, by providing a function to detect insertion of a slide sheet into the main body, user's forgetting of the insertion of a slide sheet can be prevented, and accordingly, any contamination of the focusing lens by a plume can be prevented reliably.

Moreover, by making a function to detect insertion of a slide sheet into the main body performed by the operation of a switch by the insertion of the slide sheet, user's forgetting of the insertion of a slide sheet can be prevented, and accordingly, any contamination of the focusing lens by a plume can be prevented reliably.

Moreover, by making a function to detect insertion of a slide sheet into the main body performed by electrical connection with an electrode provided in the slide sheet, user's forgetting of the insertion of a slide sheet can be prevented, and accordingly, any contamination of the focusing lens by a plume can be prevented reliably.

Moreover, a deodorizing function is provided on a film of the slide sheet having the function of a biosensor on the optical axis of a laser beam, at least an inner surface of the insertion holder similarly on the optical axis of the laser beam, and a film of the biosensor similarly on the optical axis of the laser beam. Thereby, an abnormal odor in a plume generated during laser perforating can be eliminated.

Moreover, by providing an antibacterial function in a slide sheet having the function of a biosensor, and the insertion holder, any infection when used by a user can be prevented.

In addition, as the specimen sample, those sampled percutaneously, such as blood and interstitial fluid, can be used, and as the analyte, the blood glucose level (glucose concentration), or various kinds of serological or biochemical items can be used.

Further, in a case where the blood glucose level (glucose concentration) is used as an analyte as an example of those capable of being used as a reagent, employable combination of an enzyme, in some cases, coenzyme and a mediator includes: glucose oxidase + potassium ferricyanide or ferricynium; and glucose deoxidase + pyrroloquinoline quinone or nicotinamide adenine dinucleotide + phenanthroline quinone, osmium complex, or potassium ferricyanide.

### (Twelfth Embodiment)

Hereinafter, an example of a laser perforating device in which a perforating adapter according to a twelfth embodiment of the invention is mountably provided will be described.

The laser perforating device according to the invention is composed of a laser oscillator, a display that displays a power source, a battery, and operation status, a setting button that sets an operation mode, and a main body in which an operation switch that starts perforating operation is provided, and a replaceable perforating adapter. As for the perforating adapter, in a hollow body having an axis parallel to a laser optical axis, a focusing lens protective film is provided in an opening of the hollow body on the side of a focusing lens, and a perforating film is provided on the side where a skin that is a perforating spot is pressed during laser perforating, in the opposite opening of the hollow body.

By adopting such configuration, a closed space is formed by the perforating film, the hollow body, and the focusing lens protective film. By forming a hole in the perforating film with a laser beam, and laser-perforating a skin through this hole, a generated plume can be confined in the closed space, and an abnormal odor can be removed by a deodorizing function provided in the perforating film, the hollow body, and the focusing lens protective film. Further, since an antibacterial function is also provided, there is also no fear of infection. Moreover, since this perforating adapter is replaceable, infection can be prevented by replacing the adapter at every use.

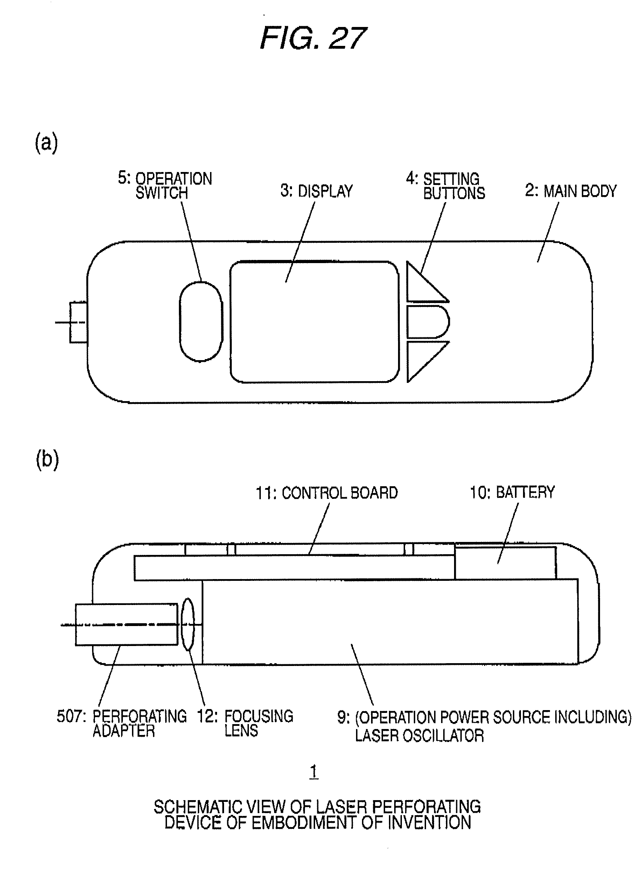

Fig. 27(a) is a top view of the laser perforating device 1 according to the embodiment of the invention, and Fig. 27(b) is a schematic sectional view showing the internal structure of the laser perforating device. The laser perforating device 1 is composed of a main body 2, a laser oscillator 9 including an operation power source, a focusing lens 12, a perforating adapter 507, a control board 11, a battery 10, an operation switch 5, a setting button 4, and a display 3. The battery 10, the control board 11 and the laser oscillator 9, the control board 11 and the laser oscillator 9, the setting button 4, the display 3, and the operation switch 5 are connected electrically and in signal.

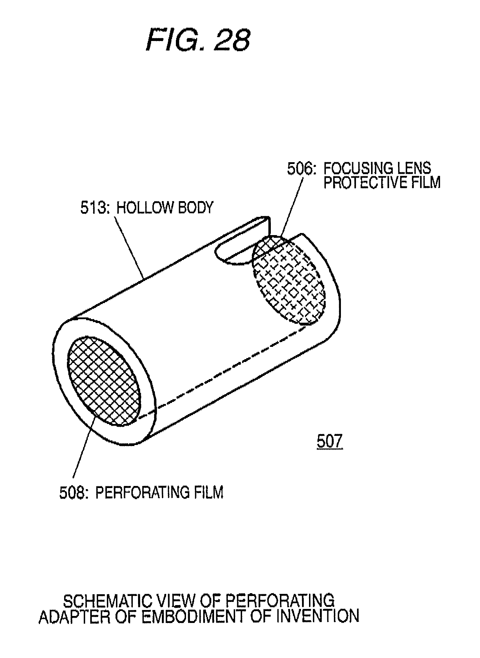

Fig. 28 is a schematic view of the perforating adapter 507 in the laser perforating device 1 of the embodiment of the invention. The perforating adapter 507 has a shape based on the cylindrical body 513. As the material of the adapter, various plastic materials are available, and they are polyethylene, polyvinyl chloride, polypropylene, polystyrene, polyvinyl acetate, ABS resin, AS resin, acrylic resin, polyacetal, polyimide resin, polycarbonate, modified polyphenylene ether (PPE), polybutylene terephthalate (PPB), polyarylate, polysulfone, polyphenylene sulfide, polyether ether ketone, fluororesin, or the like. A material with low zeta potential on a plastic surface is preferable.

A perforating film 508 (first film) is provided in an opening that is a portion where a person's skin (finger, etc.) touches. The perforating film 508 absorbs a laser beam and is perforated. As the base material of the film 508, nylon, polyester, polyimide, fluorine system, vinyl chloride, polyethylene, polyolefin, polyvinyl alcohol, polypropylene, and the like are available.

An opening on the side of the main body is mounted with a focusing lens protective film 506 (second film). The focusing lens protective film 506 does not absorb a laser beam, but transmit the laser beam. Since this film is not perforated by absorption of the laser beam unlike the perforating film 508 (first film), it has a role of preventing a piece of tissue that has evaporated from a skin during laser perforating from adhering to the focusing lens 12, and contaminating the focusing lens 12. As the base material of the focusing lens protective film 506, nylon, polyester, polyimide, fluorine system, vinyl chloride, polyethylene, polyolefin, polyvinyl alcohol, polypropylene, and the like are available.

Next, the operation will be described. First, when a user pushes the operation switch 5, the laser perforating device 1 begins to start. Next, the user inputs the operating conditions of the laser perforating device 1 using the setting button 4. After the input, the user inserts the perforating adapter 507 into the main body 2, and presses his/her finger that is a perforating spot against the perforating adapter 507.

After the completion of the preparation, when "standby" is displayed on the display 3, and the user pushes the operation switch 5 once again, the laser oscillator 9 oscillates a laser pulse beam, the oscillated laser beam is focused by the focusing lens 12, and is focused on a skin of a user's finger while its beam diameter is made small within the hollow perforating adapter 507. Then, the laser beam is absorbed by the skin, and the skin is heated and evaporated, and thereby perforated. By the perforating, the epidermis of the skin and the uppermost surface of the dermis thereof evaporate, a capillary vessels within, for example, a dermal papilla of the dermis is wounded, and blood oozes out. Then, the blood oozes out to the surface of the skin through the perforated hole.

At this time, any hole is not formed in the focusing lens protective film 506, while a laser beam is absorbed by the perforating film 508 where the skin touches, thereby forming a hole. Through this hole, the previous laser perforating is performed. A plume generated by the evaporation of the skin during laser perforating is diffused into the hollow body 513 through the hole of the perforating film 508.

Volatile substances that are generated as amino acids that are components of protein that constitutes the tissue of a skin are decomposed drift within the plume. A stratum corneum that is a main object during laser perforating is composed of the fibrous tissue of protein keratin, and fibers are bonded by cystine bonds that are bonds of a sulfur portion of amino acid cystine that is contained relatively much. As these amino acids are decomposed during evaporation, volatile sulfur compounds or nitrogen compounds are generated, and particularly, a person feels the sulfur compounds as an abnormal odor.

Thus, a deodorizing function against abnormal odor components of the plume is given to the inside of the perforating adapter 507. Hydrogen sulfides or methyl mercaptans are mentioned as main sulfur compounds. The hydrogen sulfides can be deodorized by a chemical adsorbent, and composite oxides of manganese, copper, and cobalt can be used. In addition, by using oxides, hydroxides, composite oxides, or its mixture, which contain any of manganese, copper, zinc, and cobalt, similarly, an adsorbent that has a powerful chemical adsorbent action against the hydrogen sulfides can be obtained.

The chemical adsorbent chemically adsorbs the hydrogen sulfides finally in the form of sulfates or as simple sulfur substances. Further, the chemical adsorbent also has an intermediation action that converts methyl mercaptans that are the same sulfur-based odor into dimethyl disulfides having a higher threshold value. For this action, a person can feel an effect equivalent to deodorization.

Moreover, since the dimethyl disulfides can be removed by a physical adsorbent action by making a physical adsorbent carried, it is possible to remove the sulfur compounds in general. Hydrophobic zeolite having a large portion of silica can be used as the physical adsorbent. In addition, even if zeolite, sepiolite, silica, alumina, and the like are used, the same effect is obtained.

These chemical adsorbent and physical adsorbent are added to the base material of the focusing lens protective film 506, perforating film 508, or perforating adapter 507. Otherwise, the adsorbents can be coated on the focusing lens protective film 506, the perforating film 508, or the inside of the perforating adapter 507.

Further, since the perforating film 508 contacts the user's finger, it is preferable that antibacterial properties (antibacterial properties) are given to the film. The antibacterial properties can be realized by coating or kneading onto/into a base material. The antibacterial agents include Ag, Cu, Zn, Ni, Co or their alloys, TiO2, ZnO, WO3, and the like. In addition, the antibacterial properties are useful by being implemented not only on the perforating film 508 but on the external surface of the hollow body 513 of the perforating adapter 507 or the focusing lens protective film 506.

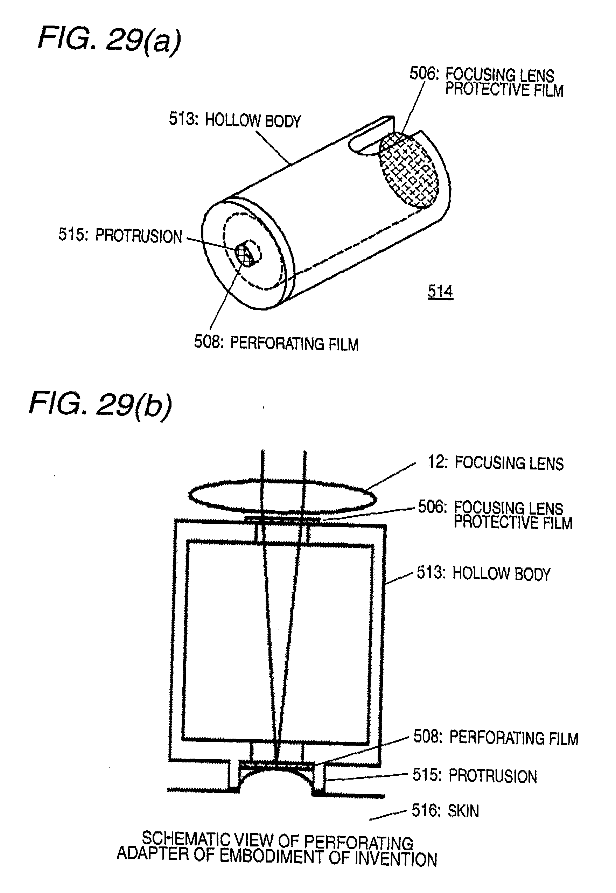

Fig. 29(a) is a schematic view of another perforating adapter 514 in the laser perforating device 1 of the embodiment of the invention. As for the perforating adapter 514, the diameter of the opening against which the skin is pressed is controlled by a protrusion 515 formed around the perforating film 508. Since the diameter and depth that realize optimal pressing against the skin is set depending on the shape of the protrusion 515, reliable blood sampling is allowed.

Fig. 29(b) is an enlarged view of a laser perforating spot during laser perforating. A user applies the perforating adapter 514 to a predetermined laser perforating spot of a skin 516. At this time, the protrusion 515 protrudes about 1 to 3 mm from the perforating film 508, and this protrusion 515 will pushes the skin 516. This will stimulate the periphery of the predetermined laser perforating spot. For this reason, it is possible to mitigate the stimulus that the user feels during laser perforating.

As described above, according to the laser perforating device 1 of this embodiment, a closed space can be formed by the perforating film 508, the hollow body 513, and the focusing lens protective film 506. Then, by forming a hole in the perforating film 508 with a laser beam, and laser-perforating a skin through this hole, a generated plume can be confined in the closed space.

Further, an abnormal odor can be removed by a deodorizing function provided in the perforating film 508, the hollow body 513, and the focusing lens protective film 506. Also, since an antibacterial function is also provided, there is no fear of infection. Moreover, since the perforating adapter 507 or 514 is replaceable, infection can be prevented by replacing the adapter at every use.

### (Thirteenth Embodiment)

Hereinafter, a laser irradiation method of perforating (laser-perforating) the skin of a human body with a laser beam as the thirteenth embodiment of the invention will be described. After the laser irradiation method is first described, a laser perforating device will be described.

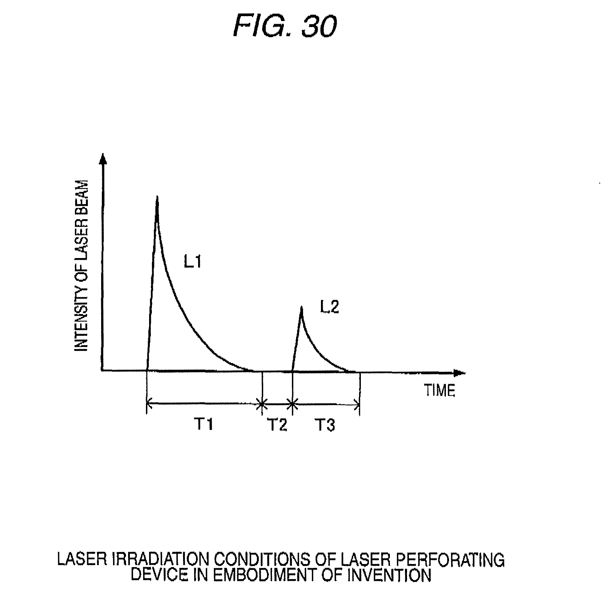

Fig. 30 is a schematic view of laser irradiation conditions of the laser perforating device according to the thirteenth embodiment of the invention. In this drawing, the axis of abscissa represents time, and the axis of ordinate represents laser beam intensity. A left laser pulse beam L1 is a laser pulse beam for perforating epidermis, and a right laser pulse beam L2 is a laser pulse beam for perforating dermis.

The full time width T1 of the laser pulse beam L1 for perforating epidermis are 100 to 400 µs. The full time width T3 of the laser pulse beam L2 for perforating dermis are 50 to 300 µs. The difference (T1 - T3) between the full time widths T1 and T3 of the laser pulse beams L1 and L2 is preferably 50 to 200 µs. In addition, the shorter the interval T2 between the laser pulse beam L1 and the laser pulse beam L2, the better. The interval is preferably 500 ms or less, and more preferably 1 ms or less.

As for laser pulse beam energy, the total of the energy obtained when a unit area (1 cm²) is irradiated with the two laser pulse beams L1 and L2 is preferably 100 to 300 J. For example, supposing an irradiation area is 0.1 mm (= 0.01 cm) in diameter, the area becomes 7.85 x 10⁻⁵ cm² (= 0.005 x 0.005 x π). 7.85 x 10⁻⁵ cm² x 100 to 300 J/cm² = 7.85 to 23.6 mJ obtained by multiplying the area by energy density 100 to 300 J/cm² per irradiation unit area becomes the total energy of the laser pulse beams L1 and the L2.

Further, the energy of a laser pulse beam for perforating dermis is preferably 10 to 40% of the total of the energy of a laser pulse beam for perforating epidermis and the energy of a laser pulse beam for perforating dermis. That is, in a case where the total energy of the laser pulse beams L1 and the L2 is 20 mJ, the energy of the laser pulse beam L2 for perforating dermis is preferably 10 to 40% (2 to 8 mJ) of the total energy.

Moreover, the focusing diameter of a laser pulse beam is 0.15 mm or less, and is preferably 0.1 mm or less. Further, the shorter the time interval T2 between the laser pulse beams L1 and L2, the better. The interval is 500 ms or less, and preferably 1 ms or less.

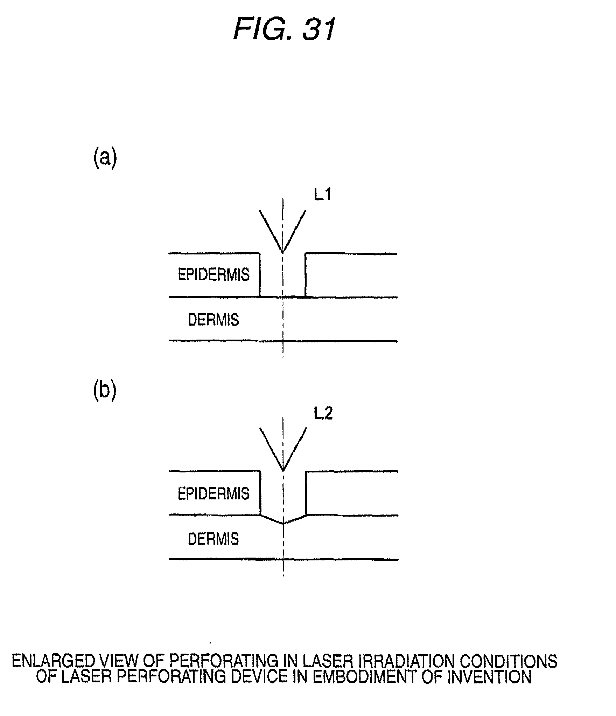

Fig. 31 is an enlarged view of a perforating hole of a skin in laser irradiation conditions of the laser perforating device according to the embodiment of the invention. When the skin is irradiated with the first laser pulse beam L1 for perforating epidermis, a columnar perforating hole is formed in epidermis like Fig. 31(a).

Next, when the same position as the first laser pulse beam L1 is irradiated with the second laser pulse beam L2 for perforating dermis, a mortar-shaped perforating hole is formed in the dermis like Fig. 31(b). At this time, a capillary vessels within the dermis is wounded, and blood oozes out. The oozed blood oozes out onto the skin via the laser-perforated hole of the epidermis. The depth of the laser-perforated hole to the dermis is 0.05 to 0.3 mm, and is preferably 0.05 to 0.25 mm.

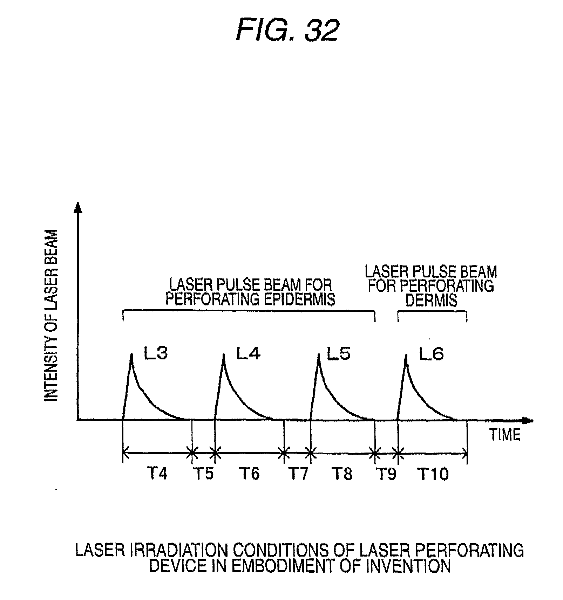

Fig. 32 shows other laser irradiation conditions according to the thirteenth embodiment of the invention. The first laser pulse beam L1 for perforating the epidermis shown in Fig. 30 is further divided into a plurality of laser pulse beams L3, L4, and L5. The time widths T4, T6, and T8 of the laser pulse beams L3, L4, and L5 for perforating the epidermis are 100 to 400 µs, respectively.

Further, the total of the energy obtained when a unit area (1 cm²) is irradiated with the laser pulse beams L3, L4, L5, and L6 is preferably 5 to 100 J (energy density per irradiation unit area is 5 to 100 J/cm²). The shorter the time intervals T5, T7, and T9 of the laser pulse beams L3, L4, L5, and L6, the better. The intervals are 500 ms or less, and preferably 1 ms or less.

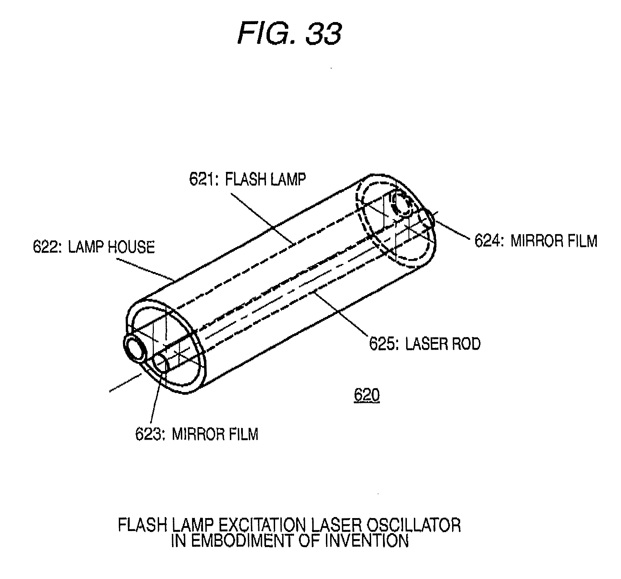

Fig. 33 is a schematic view of a laser oscillator 620 to be carried on the laser perforating device according to the embodiment of the invention. The laser oscillator 620 is composed of a laser rod 625 at ends of which mirror films 623 and 624 for a laser beam resonator are formed, a flash lamp 621 for exciting a laser beam, and a lamp house 622 for efficiently guiding the light of the flash lamp 621 to the laser rod 625. In addition, there is a power source (not shown) for operating the flash lamp 621. In addition, the laser rod 625 and the two mirror films 623 and 624 constitutes the laser beam resonator.

The white light emitted from the flash lamp 621 is reflected directly or inside the lamp house 622, and a wavelength with absorption of the laser rod 625 is absorbed by the laser rod 625. Then, an active medium within the laser rod 625 is excited, and inverted distribution is formed. Spontaneous emission light propagates inside the laser rod 625, and is reflected by the mirror films 623 and 624 at both end faces of the laser rod 625, reciprocates in a mode that is an eigensolution within a light resonator, and is amplified by induced emission, and is extracted as a laser beam from one mirror film 623 where reflectance is slightly lowered.

The flash lamp 621 emits white light as Xe gas inside of a glass tube discharges electricity by high-voltage charges charged in a capacitor by preliminary ionization by a trigger electrode. In order to oscillate a plurality of laser pulse beams, the flash lamp 621 should emit light multiple times. This is enabled, for example, by a method of making light emitted multiple times by gate-controlling high-voltage charges that are once charged by a semiconductor gate switch (IGBT), and by performing gate-controlling while charging is performed. By examining a gate control pattern, it is possible to control the number of pulses of each laser pulse beam, full time width, light energy, and pulse interval.

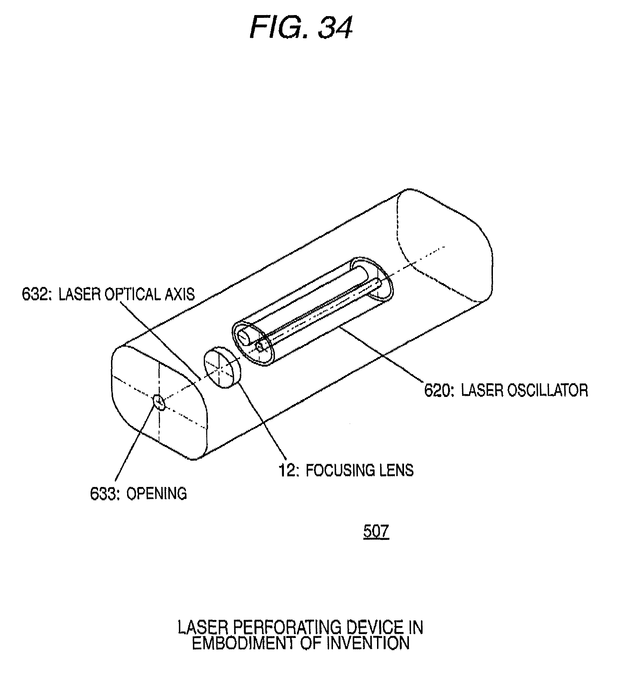

Fig. 34 is a schematic view of the laser perforating device 1 according to the embodiment of the invention. The inside of a main body is provided with a laser oscillator 620, a power source that is not shown, and a focusing lens 12 on a laser optical axis 632 of a laser oscillator 620. An opening 633 is provided in a focusing point of the focusing lens 12 on the laser optical axis 632 of the main body. By applying a finger to the opening and performing laser perforating, blood can be sampled from the finger.

As described above, according to the laser perforating method of this embodiment, a laser pulse beam for perforating dermis is radiated to the same position as a laser pulse beam for perforating epidermis, and a mortar-shaped perforating hole is formed in the dermis. Therefore, the dermis is not perforated deeply, any scar does not remain, pain during perforating is little, and stable blood sampling can be performed.

Further, the time width of a laser pulse beam for perforating epidermis is equal to or larger than that of a laser pulse beam for perforating dermis, and the energy of the laser pulse beam for perforating epidermis is larger than that of the laser pulse beam for perforating dermis. Therefore, the depth of a cross-sectional shape of a conical laser-perforated hole in the dermis can be controlled to necessary minimum, any scar does not remain, pain during perforating is little, and stable blood sampling can be performed.

### (Fourteen Embodiment)

First, the contents of study until the invention has been contrived will be described. The present inventors have found out the following fact as a result of studying pains during blood sampling. That is, perforating of a person's skin for blood sampling is performed directly by a needle, and indirectly by a laser beam. If an interaction with a human body occurs, a reaction against an action is given to the human body. Further, a sensing mechanism of a pain is a nerve reaction by a stimulus to a free nerve terminal of dermis on the side of epidermis that detects a pain. A pain is sensed when a nerve reaction amount is above a pain detection threshold value.

That is, in order to realize perforating with little pain, the nerve reaction amount is made below a pain detection threshold value, or a means that makes the pain detection threshold value sufficiently high enough is considered. In order to make the nerve reaction amount small, it is desirable to perform perforating in a place where there is no free nerve terminal so as not to stimulate other free nerve terminals. However, a fingertip that is a general blood sampling spot has a high density of free nerve terminals, and it is not easy to avoid this.

Meanwhile, it was proved that a pain detection threshold value can be controlled to some extent from the outside. That is, it was found out that the action that lowers the pain detection threshold value changes with person's consciousness to a stimulus. The consciousness can be lowered by concentrating person's consciousness in addition to the timing of perforating.

Specifically, it was found out that, when the laser perforating device is used while a periodic sound, such as an operation sound of a pump, or an engine sound, is given to a user, user's consciousness is concentrated on the periodic sounds other than the timing of perforating, and a sense of pain by a stimulus during laser perforating is lowered.

In addition, any kinds of periodic sounds may be prepared, and may be changed randomly every time. Further, the timing from the generation of a periodic sound to laser perforating may be changed randomly every time. This is because, if a user always hears the same periodic sound, it is impossible to concentrate user's consciousness on the periodic sound, and if the timing to laser perforating is always the same, the user may be conscious of the timing of laser perforating unconsciously.

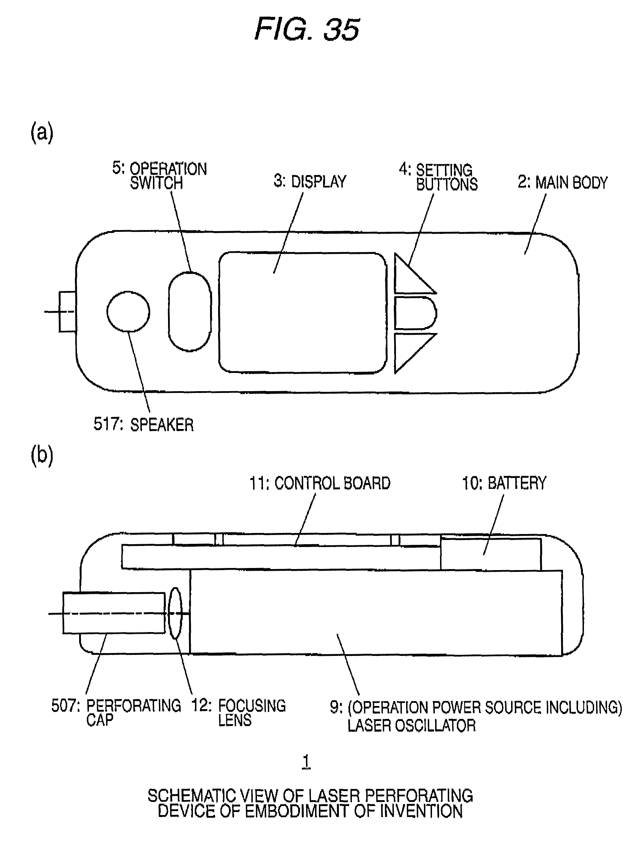

Fig. 35 is a schematic view of the laser perforating device 1 according to the embodiment of the invention. The laser perforating device 1 is composed of a main body 2, a laser oscillator 9 including an operation power source, a focusing lens 12, a perforating cap 507, a control board 11, a battery 10, an operation switch 5, a setting button 4, a display 3, and a speaker 517. The battery 10, the control board 11 and the laser oscillator 9, the control board 11 and the laser oscillator 9, the setting button 4, the display 3, the operation switch 5, and the speaker 517 are connected electrically and in signal. The speaker 517 generates a periodic sound on the basis of a control signal generated inside as described below.

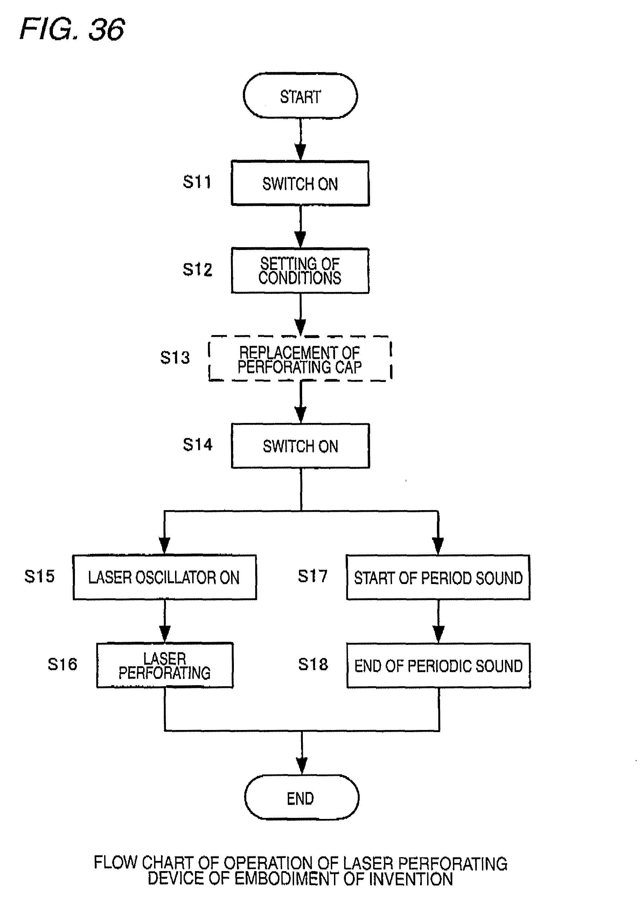

Fig. 36 is a flow chart of the operation of the laser perforating device 1 according to the embodiment of the invention. The operation will be described according to the flow chart. First, when a user pushes the operation switch 5 (Step S11), the laser perforating device 1 begins to start. Next, the user inputs conditions, such as laser perforating conditions, by the setting button 4 (Step S12).

After the setting, the user presses his/her finger that is a perforating spot against the perforating cap 507. At this time, the perforating cap 507 can be replaced (Step S13). Next, when the user pushes the operation switch 5 once again (Step S14), a periodic sound begins to sound from the speaker 517 (Step S17), and the operation power source of the laser oscillator 9 is brought into a standby state (Step S15).

Next, the laser oscillator 9 oscillates a laser pulse beam with certain timing, the oscillated laser beam is focused by the focusing lens 12, and is focused on a skin of a user's finger while its beam diameter is made small within the hollow perforating cap 507. Then, the laser beam is absorbed by the skin, and the skin is heated and evaporated, and thereby perforated (Step S16).

By the perforating, the epidermis of the skin and the uppermost surface of the dermis thereof evaporate, a capillary vessels within, for example, a dermal papilla of the dermis is wounded, and blood oozes out. Then, the blood oozes out to the surface of the skin through the perforated hole. The periodic sound disappears after a certain fixed period after the laser perforating (Step S18).

According to the laser perforating device 1 of this embodiment, user's consciousness is concentrated on a periodic sound. Thus, a user cannot wait for the timing of laser perforating, and can perform laser perforating in a state where the threshold value of a sense of pain is low. Therefore, the pain can be alleviated.

Further, there are several kinds of periodic sounds, and the timing from the generation (Step S17) of a periodic sound to the laser perforating (Step S16) is changed randomly every time. Thus, a user is not accustomed to this periodic sound or timing, but can concentrate his/her consciousness on something other than the laser perforating.

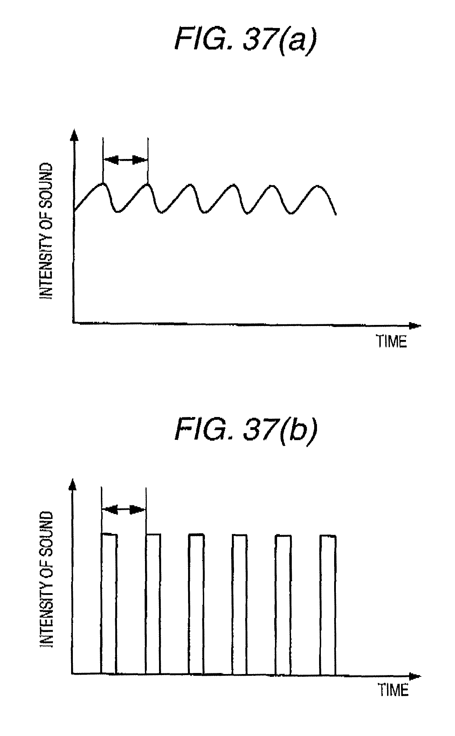

Fig. 37 is a view showing an example of a periodic sound to be used by the laser perforating device 1 according to the embodiment of the invention. Here, the periodic sound means a sound that is repeated in monotone, such as a pump or engine sound, or a certain range of sound within repeated tempo, such as a metronome sound.

Fig. 37(a) is a view showing the intensity of a sound repeated in monotone, such as a pump or engine sound. The sound repeated in monotone is a low-frequency sound the center frequency of which is 20 to 100 Hz, and is more preferably 40 to 70 Hz. Accordingly, a user can direct his consciousness to the sound without feeling dispersant.

Fig. 37(b) is a view showing the intensity of a certain range of sound with repeated tempo, such as a metronome sound. As other sounds with repeated tempo, there are an ON/OFF sound of a mechanical switch, a sound that strikes a keyboard, and the like. The repeated tempo is within a range of 60 to 208, and more preferably 120 to 180 if it is expressed in scales of (shows how many beats for one minute) of a metronome. Accordingly, a user can direct his consciousness to the tempo without feeling irritation.

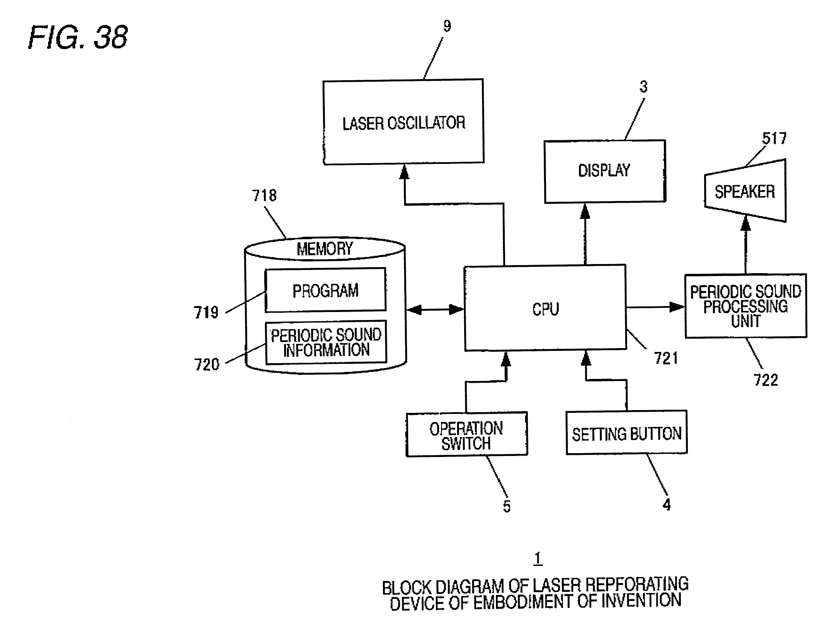

Fig. 38 is a block diagram showing an internal configuration of the laser perforating device 1 of this embodiment. As shown in this drawing, the laser perforating device 1 is provided with a laser oscillator 9 that is a perforating means, a display 3, an operation switch 5, a setting button 4, a CPU 721 a memory 718 that stores a program 719 for making the CPU 721 perform ambient sound processing, and periodic sound information 720, a periodic sound processing unit 722 for generating a periodic sound, and a speaker 517 that is a sound source.

In the memory 718, a plurality of periodic sounds, such as a sound that is repeated in monotone, such as a pump or engine sound, and a certain range of sound within repeated tempo, such as a metronome sound, are stored as the periodic sound information 720. When the operation switch 5 is pushed after laser perforating conditions, etc. are set from the setting button 4, according to the program 719 stored in the memory 718, the CPU 721 reads from the memory 718 a predetermined item of the periodic sound information 720 corresponding to a random value generated according to a random function, and transmits it to the periodic sound processing unit 722 to cause a predetermined periodic sound to be generated from the speaker 517.

Thereafter, the CPU 721 makes a skin to be irradiated with a laser pulse beam from the laser oscillator 9 after the lapse of random time generated according to a random function, thereby performing perforating. As such, in the laser perforating device 1 of this embodiment, a random periodic sound is generated during perforating, and the timing from the generation of the periodic sound to laser perforating is changed randomly. Thus, user's consciousness can be diverted from perforating. Further, it is difficult for a user to wait for the timing of laser perforating. Thereby, the pain during perforating can be alleviated.

As described above, according to the laser perforating device 1 of this embodiment, a periodic dummy sound is generated, user's consciousness is diverted to the sound, and a user is made not to be conscious of perforating timing. Thus, laser perforating can be performed in a state where a threshold level that the user feels pain.

Further, a random periodic sound is generated during perforating, and the timing from the generation of the periodic sound to laser perforating is changed randomly. Thus, user's consciousness can be diverted from perforating. Further, it is difficult for a user to wait for the timing of laser perforating. Thereby, the pain during perforating can be alleviated.

### (Fifteenth Embodiment)

Prior to the description of the embodiment of the invention, first, the progress until the invention has been contrived will be described. The laser perforating by a laser pulse beam is performed by perforating the epidermis of a skin to be targeted, damaging a capillary vessels in a dermal papilla in the dermis, making blood ooze out from the capillary vessels, and making blood ooze out of the skin through a hole of the epidermis. Further, the laser perforating of a skin by a laser pulse beam is achieved as the skin is heated and evaporated by absorption of laser pulse beam energy into the skin.

The principal component of a skin is water, and the wavelength of a laser pulse beam having a high absorption coefficient of water is selected. For example, the wavelength of 2.94 µm using Er:YAG solid-state laser media is most efficiently absorbed into water, and perforating is allowed with lowest laser pulse beam energy.

For this reason, factors that form differences among individuals in laser pulse beam energy required for laser perforating include the thickness of the epidermis of a skin and the water amount of the epidermis. Generally, the thickness of the dermis becomes large with aging, men are thicker in dermis than women, and the color of a skin and the thickness of the skin have a relationship that the black skin is the thickest, and the yellow skin is secondly thicker, and the white skin is thinnest. Further, the thickness of a skin also has something to do with the use frequency of person's fingertip, and a musician or sport player who uses his/her fingertip has a thicker skin than ordinary persons.

Moreover, although the water amount of epidermis also has negative correlation with the thickness of the epidermis, it has more random differences among individuals than the thickness of the epidermis. Further, since the water amount on the uppermost surface of the epidermis is balanced with the humidity of the ambient air, there are fluctuation from day to day and fluctuation within a day.

Further, as a result of study by the present inventors, it was proved that, in a case where laser perforating is performed with an extremely smaller focusing diameter (<φ0.15 mm) than a conventional laser perforating device, as the differences among individuals, the water amount has a larger effect on the laser pulse beam energy than the thickness of the epidermis. It was proved that, there was about double difference in the laser pulse beam energy required for laser perforating at the focusing diameter of, for example, φ0.5 mm, whereas a difference was hardly seen in the focusing diameter of φ0.15 mm. It turns out that there is a large difference although the thickness of the epidermis is not quantified. However, there was no difference in the water amount of the epidermis.

As a result of the above studies, the laser perforating device according to the embodiment of the invention is configured so that a water-amount measuring sensor may be provided in the perforating cap that touches user's skin. Accordingly, the water amount of a perforating spot can be measured before perforating. Further, the attributes of a user, such as age, sex, race, and skin type (thick, hard, thin, and soft), are input in advance.

Further, from the data of age, sex, a race, and skin type, setting values are given as compared with laser pulse beam energy setting parameters stored in the memory in the laser perforating device, thereby allowing reliable blood sampling.

Moreover, it is also possible to arrange a humidity sensor inside the laser perforating device or on the surface thereof to measure the humidity of the ambient air to determine a setting value. In addition, a sensor that measures water amount may be arranged on the surface of the laser perforating devices other than a perforating head.

The laser perforating device 1 according to the embodiment of the invention is almost the same as that shown in Fig. 35. The laser perforating device 1 of this embodiment is composed of a main body 2, a laser oscillator 9 including an operation power source, a focusing lens 12, a perforating cap 507, a control board 11, a battery 10, an operation switch 5, a setting button 4, and a display 3. The battery 10, the control board 11 and the laser oscillator 9, the control board 11 and the laser oscillator 9, the setting button 4, the display 3, and the operation switch 5 are connected electrically and in signal. However, in this embodiment, the water-amount measuring sensor is set in the perforating cap 507, and is connected with the control board 11 via the perforating cap 507.

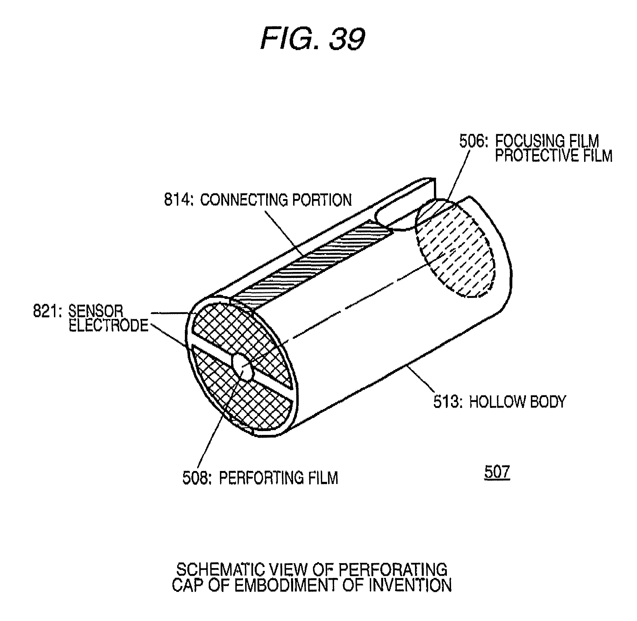

Fig. 39 is a schematic view of the perforating cap 507 where the water-amount measuring sensor is set, in the laser perforating device 1 of the embodiment of the invention. The perforating cap 507 has a shape based on a cylindrical body 513, and an opening of the cap on the side of the main body is mounted with a focusing lens protective film 506. The focusing lens protective film 506 does not absorb a laser beam, but transmit the laser beam. This film has a role of preventing a piece of tissue that has evaporated from a skin during laser perforating from adhering to the focusing lens 12, and contaminating the focusing lens 12.

As the base material of the focusing lens protective film 506, nylon, polyester, polyimide, fluorine system, vinyl chloride, polyethylene, polyolefin, polyvinyl alcohol, polypropylene, and the like are available.

An opening is provided in a portion that touches a person's finger for perforating, and this opening is mounted with the perforating film 508. The perforating film 508 transmits a laser beam, and absorbs the laser beam and is perforated. Also, sensor electrodes 821 for measurement of water amount are set so as to surround the periphery of the perforating film. In the perforating cap 507, the sensor electrodes 821 are fixed to the hollow body 513, and the surfaces of the sensor electrodes are coated with glass that is an insulating material. In addition, a connecting portion 814 electrically connects the sensor electrodes 821 and the apparatus body 2. In addition, the connecting portion 814 can be provided at an outer peripheral face or inner peripheral face of the hollow body 513.

When this perforating cap 507 is applied to a skin, a capacitor in which, particularly, a stratum corneum of epidermis is used as a dielectric body is formed. Although the electrostatic capacity of this capacitor is determined by the dielectric constant of a skin that is the dielectric body, the dielectric constant changes greatly depending on the water amount of the skin. This is because the specific dielectric constant of water is about 80, and is large as compared with the dielectric constant of other substances that form a stratum corneum, for example, about 1.5 that the dielectric constant of protein. Therefore, the electrostatic capacity remarkably reflects the water amount of a skin, and if the water amount of a skin changes, the value of the electrostatic capacity changes greatly.

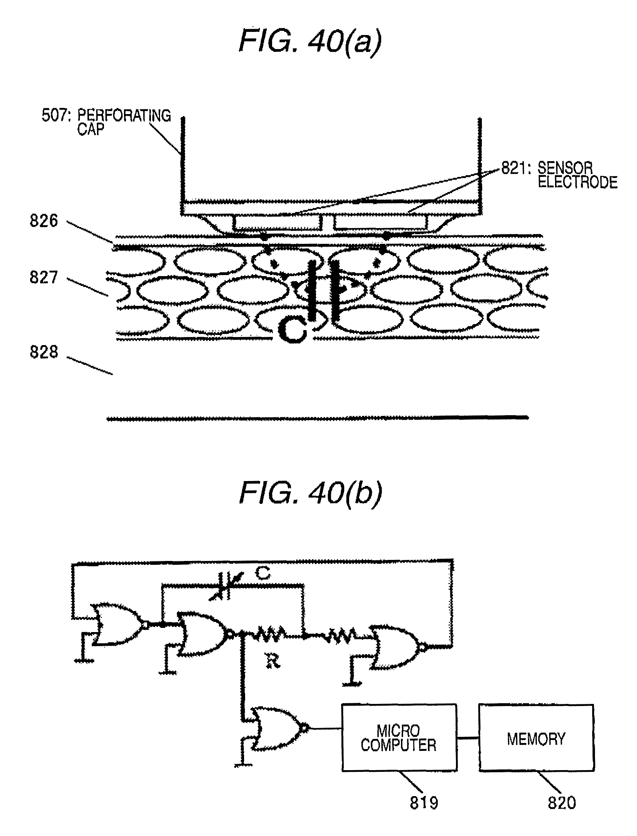

Fig. 40(a) is a sectional view in a state where the perforating cap 507 is applied to a skin. In at the tip of the perforating cap 507, the sensor electrodes 821 are fixed to the substrate, and the surface of the sensor electrode is coated with glass that is an insulating material. When this perforating cap 507 is applied to a skin, as shown in this drawing, the sensor electrodes 821 do not touch the skin directly, but touch the skin via the glass that is an insulating material.

On the other hand, the skin of a person to be tested is composed of layers in the order of a sebum layer 826, a stratum corneum 827, and an epidermis 828 from the surface. When the sensor electrodes 821 are applied to a skin, a capacitor in which the stratum corneum 827 is used as a dielectric body is formed like C shown in this drawing. Although the electrostatic capacity C of this capacitor is determined by the dielectric constant of a skin that is the dielectric body, the dielectric constant changes greatly depending on the water amount of the skin.

An electrostatic capacity detecting means is able to generate a rectangular wave with a frequency determined by electrostatic capacity and a resistance value, and converts a change of this oscillation frequency into the water amount of a skin. Fig. 40(b) is a schematic block diagram for explaining the electrostatic capacity detecting means for obtaining the change of the electrostatic capacity C.

In this drawing, particularly, a portion for detecting electrostatic capacity shows a concrete example of a circuit configuration. Since C in this drawing is the electrostatic capacity between the sensor electrodes 821, and changes with the water amount of a skin, it is described as a variable capacitor. The circuit is a multi-vibrator composed of NOR gates of a C-MOS that is generally known.

This circuit can generate a rectangular wave with a frequency determined by C and R. The circuit is configured so that the change of the electrostatic capacity C may be obtained as a change of an oscillation frequency. This rectangular wave signal is input to a microcomputer 819 as a skin water amount calculating means, the microcomputer 819 converts this frequency into the water amount of a skin, and stores it in a memory 820. Also, the microcomputer 819 adjusts laser pulse beam energy according to the water amount.

Further, the perforating cap 507 is set in a state of being pushed out by a spring for exact measurement of water amount, and is designed so that measurement of water amount may be allowed by push-in. This mechanism is connected with an interlock of the laser perforating device 1, and is adapted so that the distance between the focusing lens 12 and the skin of a finger may be exactly realized with high repeatability. In addition, the perforating cap 507 is detachably held by a cap holder 831 of a main body of the perforating device 1.

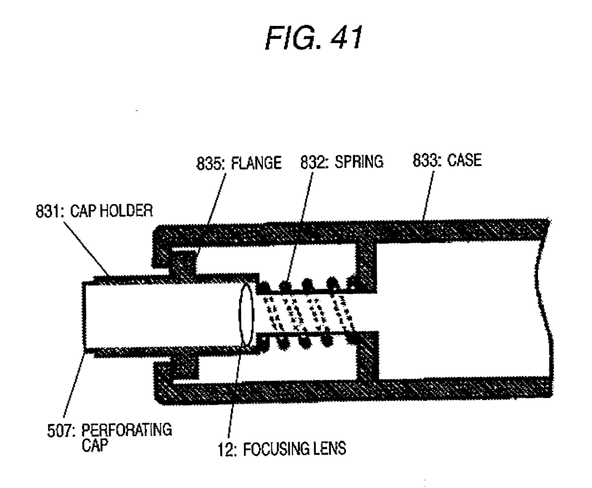

Fig. 41 is a sectional view in the vicinity of the perforating cap 507. The perforating cap 507 is attached to the cap holder 831, and the cap holder 831 is held by a case 833 that constitutes an outer shell via a spring 832 that is an elastic body. By such a configuration, the cap holder 831 is configured so as to slide toward the inside of the case 833 by the elasticity of the spring 832 when a portion of the perforating cap 507 is pressed against a skin.

That is, although the perforating cap 507 is normally pushed outward by the spring 832, it is in a state of being not jumped because a flange 835 touches the case 833. When the perforating cap 507 is pressed against a skin, the perforating cap 507 is dented into the inside of the case, and is kept constant by spring pressure. If this spring pressure is adjusted properly, the sensor electrodes 821 touches a skin by fixed pressing force, and consequently the stability of measurement improves.

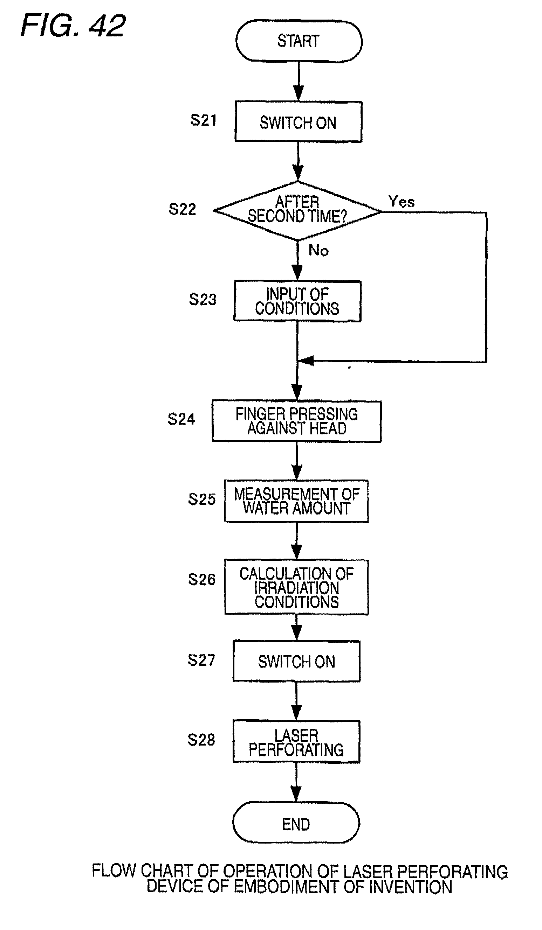

Next, the operation will be described. Fig. 42 shows the flow of use of the laser perforating device 1 according to the embodiment of the invention. First, when a user pushes the operation switch 5 (Step S21), the laser perforating device 1 begins to start. Next, in a case where the user uses the laser perforating device 1 for the first time (No of Step S22), the user inputs attributes, such as age, sex, race, and skin type (thick, hard, thin, and soft), using the setting button 4 (Step S23). After the input, the user strongly presses his/her finger that is a perforating spot into the perforating cap 507, and presses his/her finger against the cap to the fullest (Step S24). Then, measurement of the water amount of a skin is started (Step S25).

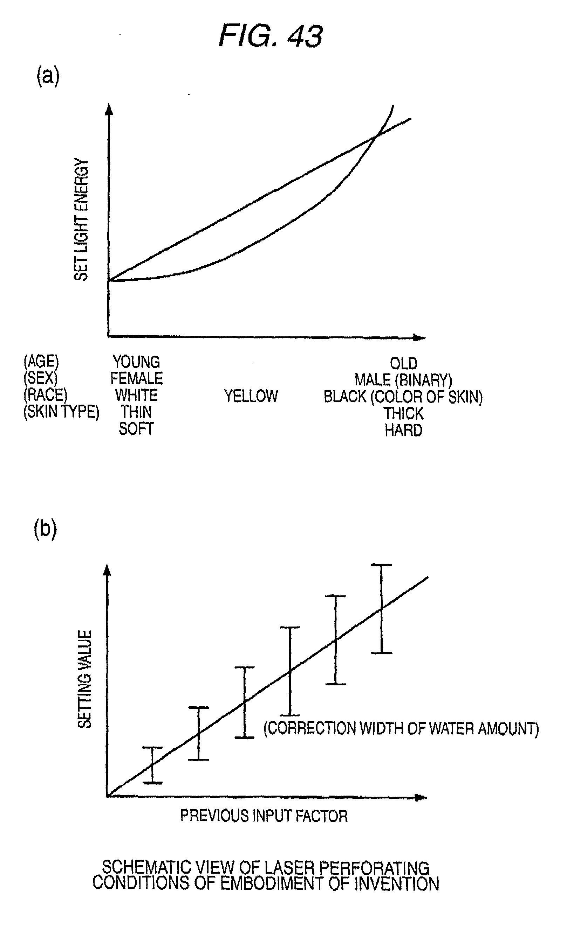

After the measurement of the water amount of the skin, laser pulse beam energy that is a laser perforating condition is set from previous input information and the measurement result of the water amount of the skin (Step S26). The method of thinking of setting is shown in Fig. 43. First, since a proportional function of rough laser pulse beam energy is prepared depending on an input condition, the laser pulse beam energy is set accordingly.

Fig. 43(a) is a schematic view showing how to set the laser pulse beam energy depending on input conditions. As shown in this drawing, the laser pulse beam energy is prepared in advance according to whether the age is low or high, whether the sex is male or female (binary), whether the race is the white race, the yellow race, or the black race (color of a skin), or whether the skin type is thin or thick, or soft or hard. As this relation, proportional, power, and exponential relationship are possible.

Fig. 43(b) is an explanatory view of a case where laser pulse beam energy is corrected on the basis of water amount measurement results of a skin in the embodiment of the invention. Values set according to the above input conditions are corrected (adjusted) in the vertical direction of this drawing on the basis of the water amount measurement results of a skin, and the energy of a laser pulse beam to be radiated is determined. At this time, in a case where there is much water amount, the values are corrected downward, and are corrected so that the laser pulse beam energy may become low.

After the completion of the correction, when "standby" is displayed on the display 3, and the user pushes the operation switch 5 once again (Step S27), the laser oscillator 9 oscillates a laser pulse beam, the oscillated laser beam is focused by the focusing lens 12, and is focused on a skin of a user's finger while its beam diameter is made small within the hollow perforating cap 507. Then, the laser beam is absorbed by the skin, and the skin is heated and evaporated, and thereby perforated (Step S28).

By the perforating, the epidermis of the skin and the uppermost surface of the dermis thereof evaporate, a capillary vessels within, for example, a dermal papilla of the dermis is wounded, and blood oozes out. Then, the blood oozes out to the surface of the skin through the perforated hole. In addition, in a case where the same user uses the biosensor again afterward, since the previous input condition is stored, the process after second use (Yes of Step S22) is started from the measurement of the water amount of a skin.

Accordingly, a user can simply and easily realize reliable blood sampling without being influenced by differences among individuals in laser pulse beam energy, fluctuation from day to day, and fluctuation within a day, and without performing the laser perforating of not sampling blood in advance.

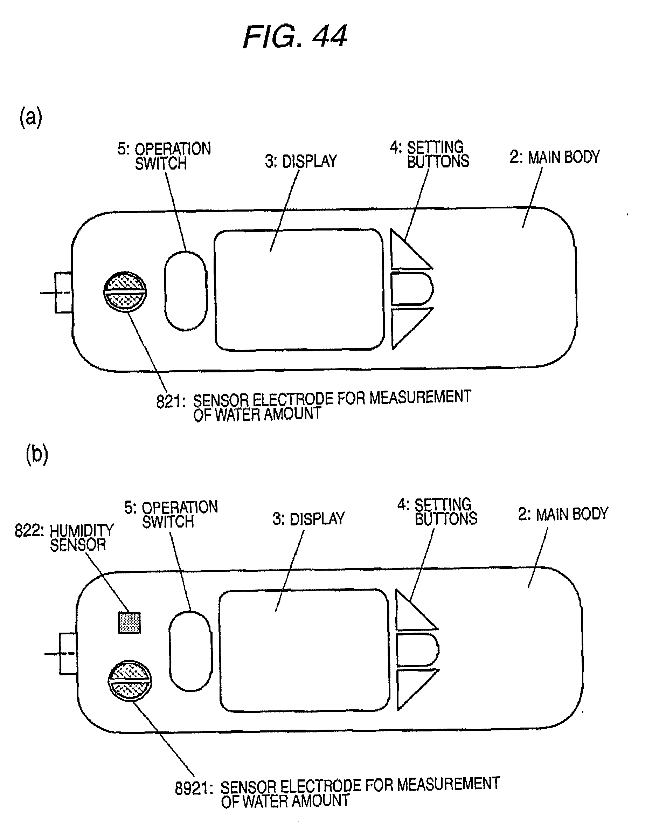

Fig. 44(a) shows an example in which the sensor electrodes 821 that measure water amount are arranged on the surface of the laser perforating device 1 excluding the perforating cap 507, in the laser perforating device 1 according to the embodiment of the invention. In this case, before perforating is performed, a fingertip is applied to the sensor electrodes 821 for measurement of water amount, and water amount is then measured. According to the laser perforating device 1 of this embodiment, since the sensor electrodes 821 that measure water amount are arranged on the surface of the laser perforating device 1, the water amount of a skin can be simply and easily measured.

Moreover, it is also possible to arrange a humidity sensor inside the laser perforating device 1 or on the surface thereof to measure the humidity of the ambient air to determine a setting value. Fig. 44(b) shows an example in which the humidity sensor 822 is arranged on the surface of the laser perforating device 1, in the laser perforating device 1 according to the embodiment of the invention. Since the humidity of the ambient air is closely related to the water amount of a skin, if laser pulse beam energy is corrected according to the humidity of the ambient air, it is possible to exactly set minimum laser pulse beam energy that is suitable for blood sampling.

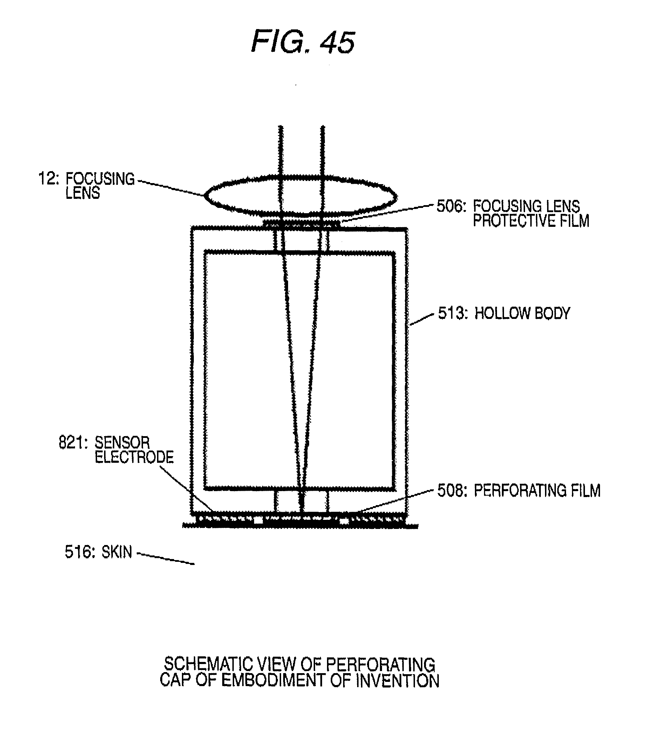

Fig. 45 is an enlarged view of a laser perforating spot during laser perforating. First, when a user pushes the operation switch 5, the laser perforating device 1 begins to start. Next, the user inputs the operating conditions of the laser perforating device 1 using the setting button 4. After the input, the user inserts the perforating cap 507 into the main body 2, and presses his/her finger that is a perforating spot against the perforating cap 507. Then, the water amount of a skin is measured, and laser pulse beam energy that is a laser perforating condition is set from previous input information and the measurement result of the water amount of the skin.

After the completion of the preparation, when "standby" is displayed on the display 3, and the user pushes the operation switch 5 once again, the laser oscillator 9 oscillates a laser pulse beam, the oscillated laser beam is focused by the focusing lens 12, and is focused on a skin of a user's finger while its beam diameter is made small within the hollow perforating cap 507. Then, the laser beam is absorbed by the skin, and the skin is heated and evaporated, and thereby perforated. By the perforating, the epidermis of the skin and the uppermost surface of the dermis thereof evaporate, a capillary vessels within, for example, a dermal papilla of the dermis is wounded, and blood oozes out. Then, the blood oozes out to the surface of the skin through the perforated hole.

At this time, any hole is not formed in the focusing lens protective film 506, while a laser beam is absorbed by the perforating film 508 where the skin touches, thereby forming a hole. Through this hole, the previous laser perforating is performed. A plume generated by the evaporation of the skin during laser perforating is diffused into the hollow body 513 through the hole of the perforating film 508.

Volatile substances that are generated as amino acids that are components of protein that constitutes the tissue of a skin are decomposed drift within the plume. A stratum corneum that is a main object during laser perforating is composed of the fibrous tissue of protein keratin, and fibers are bonded by cystine bonds that are bonds of a sulfur portion of amino acid cystine that is contained relatively much. As these amino acids are decomposed during evaporation, volatile sulfur compounds or nitrogen compounds are generated, and particularly, a person feels the sulfur compounds as an abnormal odor.

Thus, a deodorizing function against abnormal odor components of the plume is given to the inside of the perforating cap 507. Hydrogen sulfides or methyl mercaptans are mentioned as main sulfur compounds. The hydrogen sulfides can be deodorized by a chemical adsorbent, and composite oxides of manganese, copper, and cobalt can be used. In addition, by using oxides, hydroxides, composite oxides, or its mixture, which contain any of manganese, copper, zinc, and cobalt, similarly, an adsorbent that has a powerful chemical adsorbent action against the hydrogen sulfides can be obtained.

The chemical adsorbent chemically adsorbs the hydrogen sulfides finally in the form of sulfates or as simple sulfur substances. Further, the chemical adsorbent also has an intermediation action that converts methyl mercaptans that are the same sulfur-based odor into dimethyl disulfides having a higher threshold value. For this action, a person can feel an effect equivalent to deodorization.

Moreover, since the dimethyl disulfides can be removed by a physical adsorbent action by making a physical adsorbent carried, it is possible to remove the sulfur compounds in general. Hydrophobic zeolite having a large portion of silica can be used as the physical adsorbent. In addition, even if zeolite, sepiolite, silica, alumina, and the like are used, the same effect is obtained.

These chemical adsorbent and physical adsorbent are added to the base material of the focusing lens protective film 506, perforating film 508, or perforating cap 507. Otherwise, the adsorbents can be coated on the focusing lens protective film 506, the perforating film 508, or the inside of the perforating cap 507.

Further, since the perforating film 508 and the sensor electrodes 821 contact the user's finger, it is preferable that antibacterial properties are given to the film. The antibacterial properties can be implemented by coating or kneading onto/into a base material. The antibacterial agents include Ag, Cu, Zn, Ni, Co or their alloys, TiO2 ZnO, WO3, and the like. In addition, the antibacterial properties are useful by being implemented not only on the perforating film 508 and the sensor electrodes 821 but on the external surface of the hollow body 513 of the perforating cap 507 or the focusing lens protective film 506.

As described above, according to the laser perforating device 1 and the laser perforating method according to this embodiment, the energy of a laser pulse beam is adjusted according to the water amount of a skin measured by the sensor 821 for measurement of water amount that measures the water amount of the skin. Thus, it is possible to set laser pulse beam energy that is optimal according to the fluctuation of the water amount of a skin, and it is possible to perform reliable blood sampling without performing a perforating test, and with little pain.

Further, the setting button 4 that inputs user's attributes, such as age, sex, race, or skin type is provided, and the energy of a laser pulse beam is adjusted according to the age, sex, race, or skin type input from the setting button 4. Thus, it is possible to set laser pulse beam energy that is optimal according to the condition of a skin, and it is possible to perform reliable blood sampling with little pain.

### (Sixteenth Embodiment)

The sixteenth embodiment of the invention related to a new-shape insertion body in which a sheet, an insertion body, and a test paper are integrated. When a laser perforating device with no component concentration measurement function is taken into consideration, an integral configuration of a film insertion body and a film is also allowed.

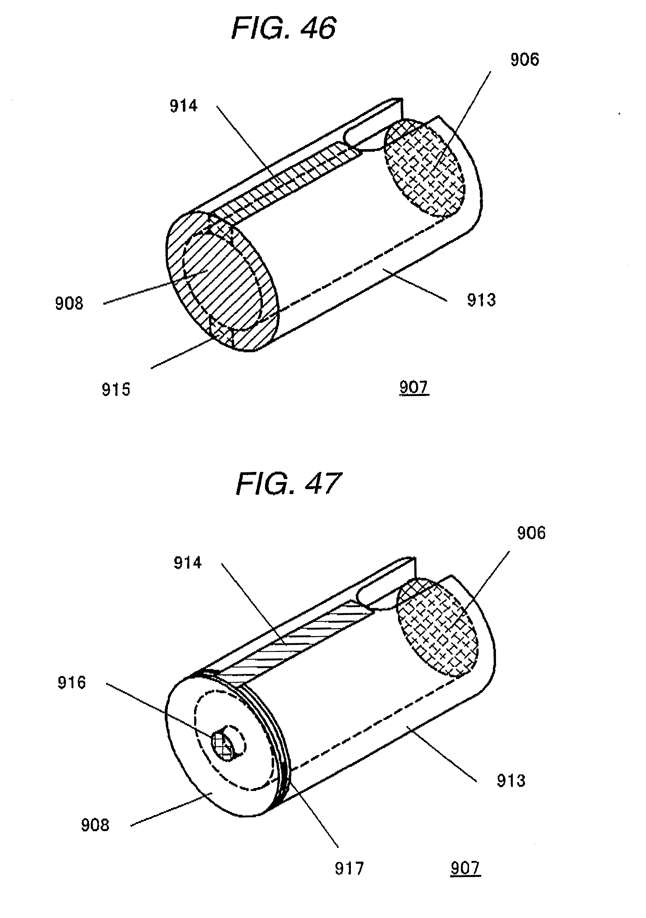

Fig. 46 is a detailed perspective view of a modified insertion holder 907 in the sixteenth embodiment of the invention. In this drawing, reference numeral 913 represents a hollow body, reference numeral 906 represents a film, reference numeral 908 represents a biosensor, reference numeral 914 represents a first electrode, and reference numeral 915 represents a second electrode.

The insertion holder 907 is the same as, for example, the insertion holder 207 according to the fifth embodiment (Fig. 13), and an opening of the insertion holder 907 on the main body is mounted with the film 906. The film 906 is the same as the film 33 of the slide sheet 6.

The biosensor 908 is inserted into an opening of the insertion holder 907 opposite the opening thereof on the side of the main body. The biosensor 908 is the same as the biosensor 8. Although not shown in Fig. 46, the specimen reagent supply passage 56 of the biosensor 8 is configured so as to be at an outer peripheral portion of the insertion holder 907. Further, the measuring electrode 252 and the electrode couple 253 of the biosensor 8 are configured so that they may be connected to the first electrode 914 and the second electrode 915, respectively.

The insertion holder 907 has been constituted by the biosensor 8, the insertion holder 207, and the slide sheet 6 that are three components, but is now constituted by one insertion holder 907. Accordingly, there is an advantage that the number of parts to be replaced when a user uses this device can be only one.

Fig. 47 is a detailed perspective view of a biosensor-patch-type insertion holder according to this embodiment. In this drawing, reference numeral 913 represents a hollow body, reference numeral 906 represents a focusing lens protective film, reference numeral 908 represents a biosensor, reference numeral 914 represents a first electrode, reference numeral 916 represents a fingerrest film, and reference numeral 917 represents a specimen reagent supply passage opening. A set of electrodes that are electrically connected with an analyzing unit of the main body are formed at the outer periphery of the hollow body 913 in order to use the biosensor electrochemically, and are constituted by a first electrode 914 and a second electrode that is not shown.

The focusing lens protective film 906 is formed on the main body side of the insertion holder 907 so as to include a laser beam, and the optical axis of the laser beam. The biosensor 908 is patched on the side of the insertion holder 908 opposite the main body so as to include a laser beam, and the optical axis of the laser beam.

A fingerrest film 916 is formed in a spot against which a skin that is a portion to be irradiated is pressed, in a portion including the laser optical axis of the biosensor 908 and a laser beam. Further, the specimen reagent supply passage opening 917 for supplying blood or the like that has oozed from a skin after laser perforating to the biosensor 908 is formed at a side face of the biosensor 908. The fingerrest film 916 is a film that absorbs a laser beam and is perforated. By pressing a finger against the fingerrest film 916, an irradiated portion can be fixed and can be stably perforated.

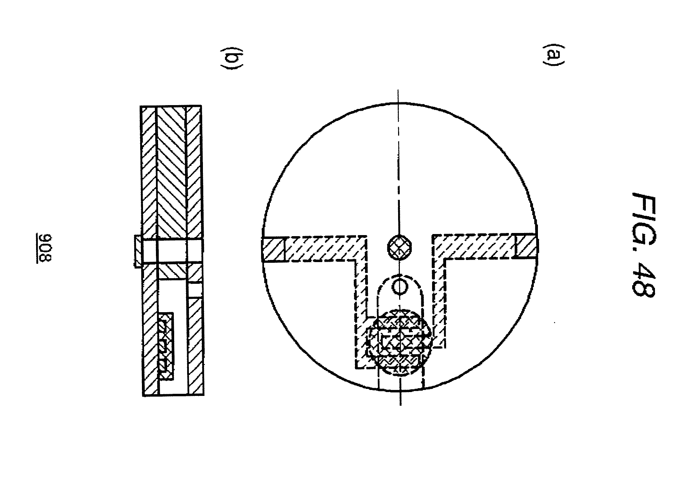

Fig. 48 is a detailed plan view and a central sectional view of the biosensor 908 of Fig. 47 (in addition, the respective portions of the configuration are the same as those of Fig. 14 of the fifth embodiment). In order to electrically connect the biosensor 908 with the first electrode 914 and the second electrode (not shown) the outer periphery of the hollow body 913 of Fig. 47, the respective electrodes on the side of the biosensor 908 are exposed on the outer periphery of the biosensor 908, and the electrodes contacts each other during patching.

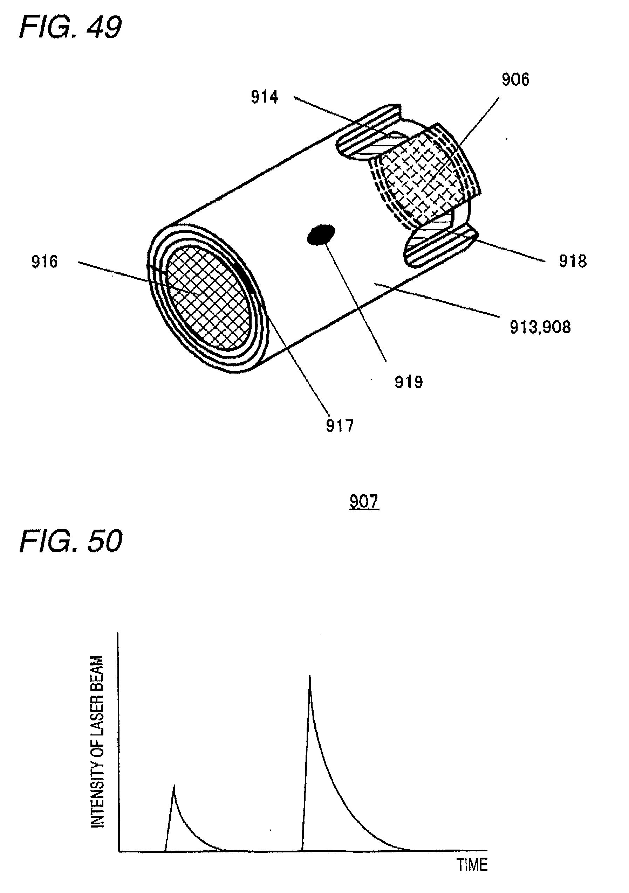

Fig. 49 is a schematic view of a hollow biosensor-type insertion holder 907 in which a biosensor is bent into a hollow body, and their ends are joined, and a focusing lens protective film and a fingerrest film are provided at both ends of the hollow body. In this drawing, reference numerals 913, 908 represents a hollow body and biosensor, reference numeral 906 represents a focusing lens protective film, reference numeral 914 represents a first electrode, reference numeral 916 represents a fingerrest film, and reference numeral 917 represents a specimen reagent supply passage opening, reference numeral 918 represents a second electrode, and reference numeral 919 represents an air hole.

This holder is obtained by making a spacer and a cover of a conventional biosensor (for example, Fig. 59) the same size as an insulating layer, making the first electrode 914 and the second electrode 918 exposed to the spacer and the cover, and providing a cutout so as to be capable of fitting into an insertion holder receiving portion of a main body when a hollow body is bent into a hollow body and is made into as an insertion holder. The electrodes are exposed to the insertion holder side of the insertion holder receiving portion on the side of the main body that is not shown, and when the insertion holder 907 is fitted, electrical connection with the respective electrodes of the biosensor is allowed.

The focusing lens protective film 906 is stuck on the main body side of the biosensor made into a hollow body, and the fingerrest film 916 is stuck on the surface opposite to the main body side. Further, the specimen reagent supply passage opening 917 is provided on the side of the fingerrest film 916, and the air hole 919 is provided at the outer periphery of the insertion holder 907 so that sampled blood may flow through a specimen reagent flow passage by the capillary phenomenon.

### (Seventeenth Embodiment)

A seventeenth embodiment of the invention relates to double pulsing of a laser pulse beam, and particularly, to a perforating method of perforating a test paper film with first weak shot, and perforating a skin with a second strong shot.

Fig. 50 is a view showing an oscillation state of a laser pulse beam in the seventeenth embodiment of the invention. As shown in Fig. 50, laser perforating is performed by a first laser pulse beam and a second laser pulse beam. Here, the light energy and peak light intensity of the first laser pulse beam are set to be weaker than those of the second laser pulse beam.

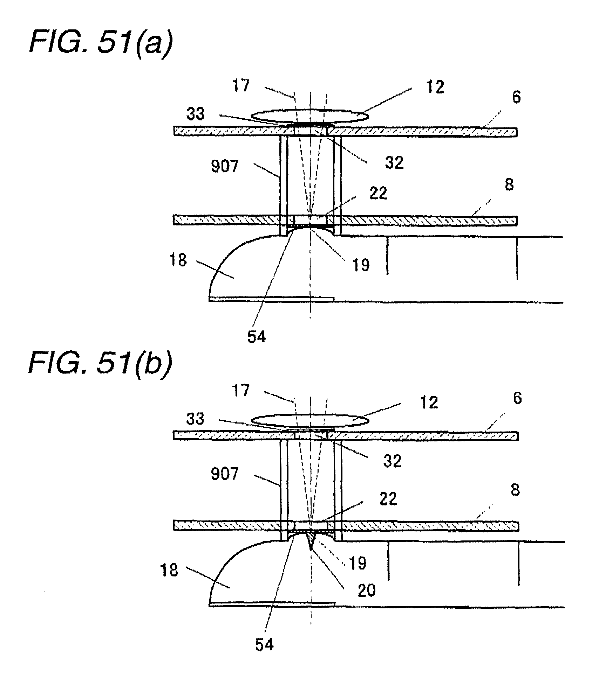

Fig. 51 (a) is an enlarged view of a laser perforating spot after irradiation of a first laser pulse beam in the seventeenth embodiment of the invention, and Fig. 51(b) is an enlarged view of the laser perforating spot after irradiation of a second laser pulse beam. A laser beam is focused on a finger 18 of the user who performs laser perforating by the focusing lens 12, and the locus thereof is a locus 17 of the laser beam.

The user's finger 18 touches the film 54 of the biosensor 8, and the laser beam 17 from the main body propagates while the beam diameter thereof is gradually tapered like Fig. 51 (a). The laser beam 17 is set so as to propagate through the film 33 of the slide sheet 6, the opening 32, the inside of the insertion holder 907, the opening 24 of the biosensor 8, and a film 54, and so as to be focused on the user's finger 18.

The opening diameters of the openings 59, 60, and 61 that constitute the opening 32 of the slide sheet 6, the internal diameter of the insertion holder 907, and the opening 22 of the biosensor 8 may be such diameters that the laser beam 17 is not substantially shielded, and all the diameters are not necessarily the same. Further, the minimum diameter of the openings is 1 mm.

The first laser pulse beam is partially absorbed by the film 33, but a through hole is not formed in the film, while the laser beam 17 is almost focused at the film 54, and the energy density thereof is high. Thus, the laser beam is absorbed by the film to heat and evaporate the film, thereby forming a through hole (Fig. 51 (a)). Subsequently, the second laser pulse beam is absorbed by the skin of the user's finger 18 to heat and evaporate the skin, thereby eventually damaging a capillary vessels of a dermis to allow blood sampling (Fig. 51(b)).

Thus, a through hole is formed in the film 54 by the first laser pulse beam, and when there are differences among individuals in the laser perforating conditions of skins, stable blood sampling is allowed by adjusting the second laser pulse beam.

### (Eighteenth Embodiment)

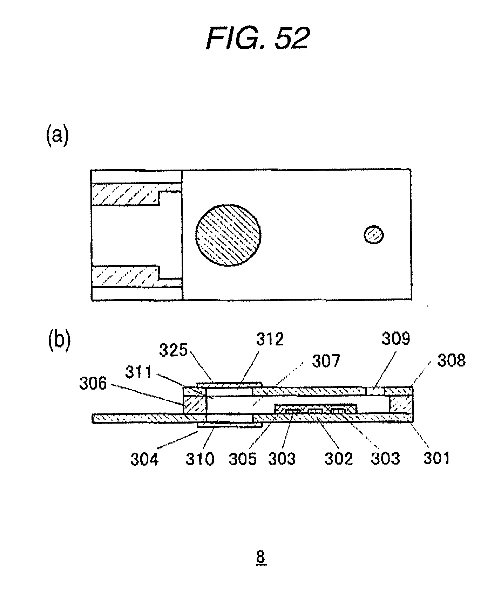

Fig. 52 shows a biosensor for laser perforating according to an eighteenth embodiment of the invention. The biosensor of this embodiment is a biosensor in which an opening of a sample supply passage is provided on the side of a film. Fig. 52(a) is a plan view, and Fig. 52(b) is a sectional view when the sensor of Fig. 52(a) is cut at a central portion in a longitudinal direction. In Fig. 52, there is a feature that the specimen reagent supply passage 307 is not connected with the out side of the biosensor, but connected with the spacer opening 311. Further, since the air hole 309 is configured so as to face the specimen reagent supply passage 307 across the reagent layer 305, the position thereof is changed.

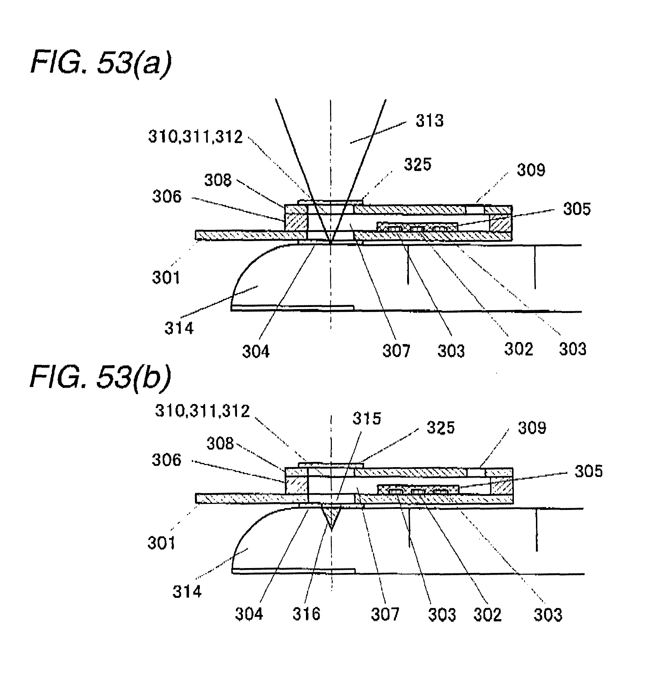

Next, the operation will be described using Fig. 53. Fig. 53(a) is a view showing the relationship between the sensor, a finger, and a laser beam during use. The laser beam 13 is partially absorbed by the film 325, but a through hole is not formed in the film, while the laser beam is focused at the film 304 to heat and evaporate the film, thereby forming a through hole. Subsequently, the laser beam is absorbed by the skin of the user's finger 314 to heat and evaporate the skin, thereby eventually damaging capillary vessels of a dermis.

Fig. 53(b) is a view shown a situation after the laser perforating in detail. In Fig. 53(b), a through hole 315 is formed in the film 304 with a laser beam. Similarly, a laser-perforated hole 316 is formed in the skin of the user's finger 314.

The blood that has oozed out from the wounded capillary vessels ooze out to the outside of the finger 314 through the laser-perforated hole 316. The blood that has oozed out is supplied to the reagent layer 305 to the reagent layer 305 with the air hole 309 through the specimen reagent supply passage 307 by the capillary phenomenon, so that the component concentration in the blood can be measured.

According to this configuration, a user can supply the blood that is an object to be measured to the biosensor as its is even if he/she does not bring his/her finger to the opening of the specimen reagent supply passage after the laser perforating.

### (Nineteenth Embodiment)

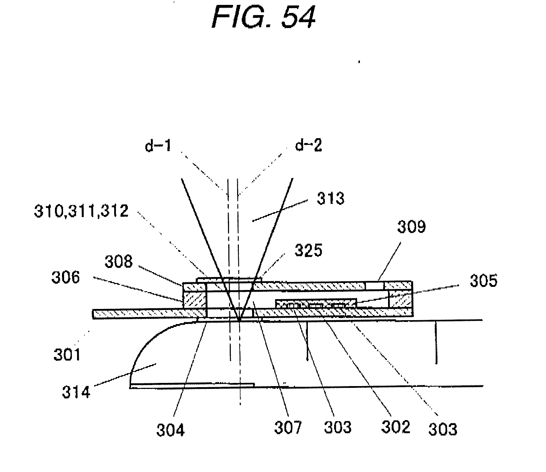

Fig. 54 shows an enlarged view of a laser perforating spot during laser perforating in a nineteenth embodiment of the invention. In this embodiment, laser perforating is performed with the laser optical axis being offset toward a sample supply passage from the center of a film opening on the side of a human body. In this drawing, d-1 is a centerline representing a substantial center of the openings 310, 311, and 312 of the sensor, and d-2 is a laser optical axis representing the center of the laser beam 313.

In this embodiment, the central axis d-1 of the opening of the sensor, and the laser optical axis d-2 are in different positions although they are parallel. Specifically, the laser optical axis d-2 is configured so as to come to a position offset toward the specimen reagent supply passage 307. Laser perforating is performed in a spot where the laser optical axis d-2 intersects a person's finger 314, and the blood that has oozed out from capillary vessels that are wounded in the dermis of a skin by the laser perforating oozes out to the outside of the finger 314. Since the blood that has oozed out is closer to the specimen reagent supply passage 307 than the sensor opening center d-1, it is possible to more efficiently reach the reagent layer 305 by the capillary phenomenon.

### (Twentieth Embodiment)

In a laser perforating device of this embodiment, the laser oscillator 620 is the same as that of Fig. 33 of the thirteenth embodiment. The laser oscillator 620 is composed of a laser rod 625 at ends of which mirror films 624 and 625 for a laser beam resonator are formed, a flash lamp 621 for exciting a laser beam, and a lamp house 622 for efficiently guiding the light of the flash lamp 621 to the laser rod 625. In addition, there is a power source (not shown) for operating the flash lamp.

The white light emitted from the flash lamp 621 is reflected directly or inside the lamp house 622, and a wavelength with absorption of the laser rod 625 is absorbed by the laser rod 625. Then, an active medium within the laser rod 625 is excited, and inverted distribution is formed. Spontaneous emission light propagates inside the laser rod 625, and is reflected by the mirror films 624 and 625 at both end faces of the laser rod 625, reciprocates in a mode that is an eigensolution within a light resonator, and is amplified by induced emission, and is extracted as a laser beam from one mirror film where reflectance is slightly lowered.

The flash lamp 621 emits white light as Xe gas inside of a glass tube discharges electricity by high-voltage charges charged in a capacitor by preliminary ionization by a trigger electrode. As such, in a case where a vacuum tube is provided and there is discharge by high-voltage charges, sputtered particles of an electrode drift in an Xe gas by a sputtering phenomenon of the electrode by discharge, and the emission intensity of the white light falls due to the optical absorption of the particles.

Further, since the shape of the electrode changes by sputtering, a spatial electron supply state particularly from a negative (-) electrode changes, the spatial homogeneity of discharge falls, and similarly, the emission intensity of the white light falls. Furthermore, leak is not avoided as long as a vacuum tube is provided, and the emission intensity of the white light falls even when the concentration of the Xe-gas changes. In this way, a change in the emission intensity of the flash lamp 621 of an excitation source reduces the output of the laser oscillator 620, and even in the same setting condition, laser perforating becomes impossible.

In the laser perforating device of this embodiment, a sensor that measures the white light that is emission of the flash lamp 621 is built. By monitoring the peak output of the sensor, it is compared with a peak output to be obtained in a setting condition. If the difference falls beyond a constant value (for example, 20%), a message that notify a user of a state and makes the user perform maintenance is issued. More preferably, it is also possible to perform the above notification by monitoring a laser pulse beam that is not emission but direct output of the flash lamp 621 that is input.

Further, the frequency of light emission is monitored using the above sensor. In a case where a value more than a certain setting trigger level is input to the sensor, this is counted by a memory. The count can display summing-up and the number of counts within a fixed period (after reset is made once) on the display of the laser perforating device.

Accordingly, if the count is close to a fixed number of times of summing-up, a user is notified of a urging of maintenance, and if the count becomes more than a fixed number of times of summing-up, it is possible to forbid use, and demand maintenance of a user.

Further, for example, in a case where a diabetic patient uses this apparatus as a blood sampling apparatus for measuring his/her own blood glucose level at home, and for controlling his/her blood glucose level, such as insulin administration and oral medicine administration, since a needle-type perforating device that is the existing perforating device is simple and few in its component parts, it is very inexpensive. Since this laser perforating device has overwhelmingly many component parts as compared with the needle-type perforating device, it is difficult to provide it at the same price as the needle-type perforating device.

However, in a case where a needle-type perforating device is used, a new needle is purchased and replaced at every use. Therefore, if the specific charging according to the times of use is allowed even in this laser perforating device, it is possible to make an initial price inexpensive.

As for the specific charging, a patient who receives treatment using an insurance system carries this laser perforating device, for example, when he/she goes to hospital regularly one time per month, and a healthcare professional can confirm the number of use, and add and charge a medical cost. Further, in a case where the perforating device is used in a medical institution, it is possible for a manufacturer or seller to go round periodically, and to confirm the number of use, and charge the cost. Moreover, it is also possible to give a wired or wireless communication function with the outside to this laser perforating device, to confirm the number of use from the outside by means of this function, and to charge the cost to a user.

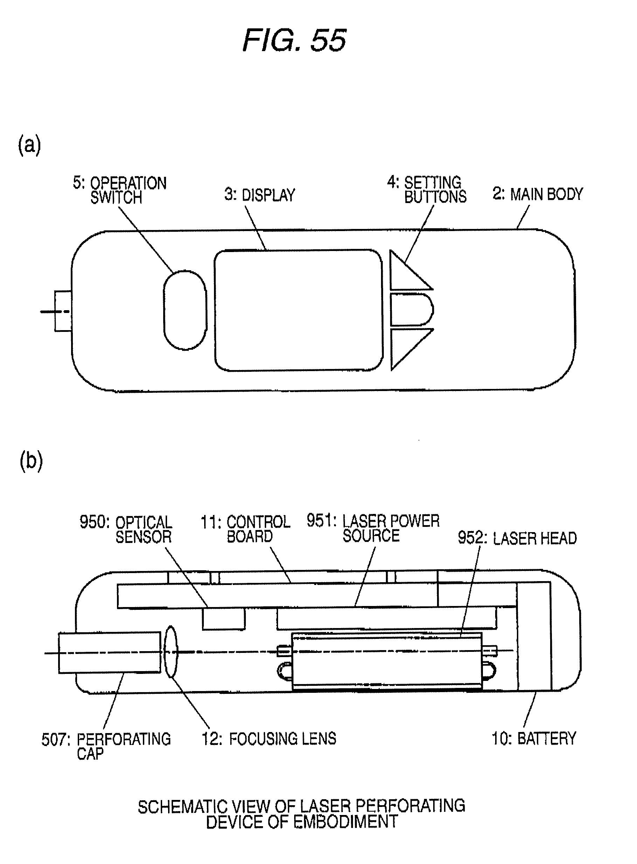

Fig. 55 is a schematic view of the laser perforatingperforating device according to this embodiment. The laser perforating device is composed of a main body 2, a laser head 952 and a laser power source 951 that constitutes a laser oscillator, a focusing lens 12, a perforating cap 507, a control board 11, a battery 10, an operation switch 5, a setting button 4, a display 3, and a photosensor 950. The battery 10 and the control board 11, the laser power source 951 and the photosensor 950, the control board 11, the laser power source 951, the setting button 4, the display 3, the operation switch 5, and the photosensor 950 are connected electrically and in signal.

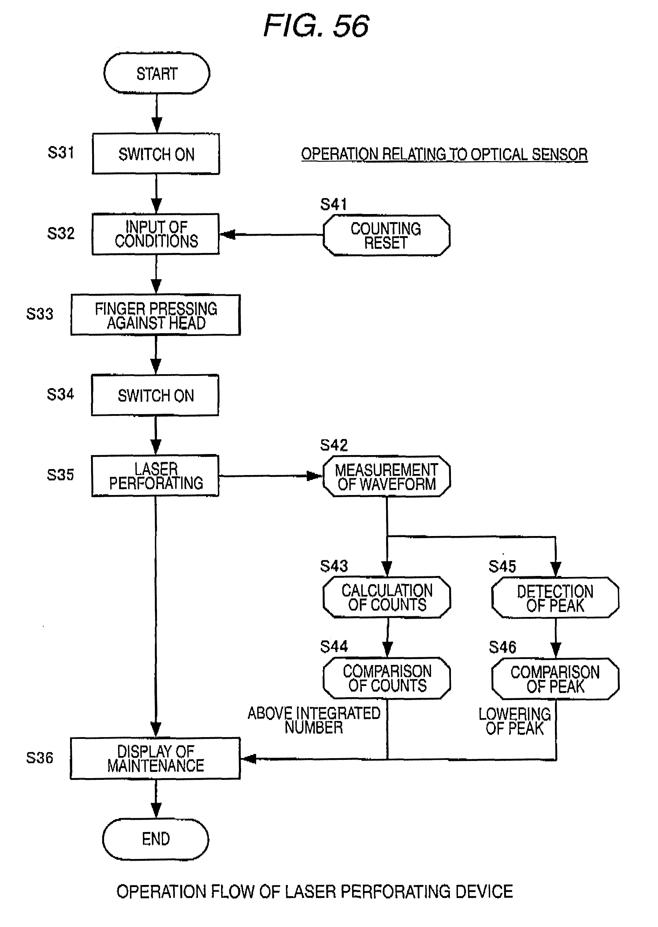

Fig. 56 shows the flow of use flow of the laser perforating device of this embodiment. Next, the operation will be described. First, when a user pushes the operation switch 4, the laser perforating device begins to start (Step S31). Next, the user inputs conditions, such as laser perforating conditions, using the setting button 4 (Step S32). At this time, the counting of a use number within a fixed period can be reset (Step S41). Here, when the progress into a fixed period in the previous flow is not made, the laser perforating device will automatically be in an end state.

After the input, when a user presses his/her finger that is a perforating spot against the perforating cap 507 (Step S33), "standby" is displayed on the display 3, and when the user pushes the operation switch 5 once again (Step S34), the laser oscillator 34 oscillates a laser pulse beam, the oscillated laser beam is focused by the focusing lens 12, and is focused on a skin of a user's finger while its beam diameter is made small within the hollow perforating cap 507. Then, the laser beam is absorbed by the skin, and the skin is heated and evaporated, and thereby perforated (Step S35). By the perforating, the epidermis of the skin and the uppermost surface of the dermis thereof evaporate, capillary vessels within, for example, a dermal papilla of the dermis is wounded, and blood oozes out. Then, the blood oozes out to the surface of the skin through the perforated hole.

At this time, the photosensor 950 within the laser perforating device measures the light emission waveform of the flash lamp 621 that emits white light that operates a laser oscillator (Step S42). Otherwise, the transmission waveform of a laser pulse beam reflected by the focusing lens 12 is observed. As the photosensor 950, a sensor that has sensitivity from the visible light of Si or InGaAs to the near-infrared light that does not include a laser pulse beam is available for white light.

Further, since the principal component of a skin that is an object is water, the wavelength of a laser pulse beam having a high absorption coefficient of water is selected. For example, the wavelength of 2.94 µm using Er:YAG solid-state laser media is most efficiently absorbed into water, and perforating is allowed with lowest laser pulse beam energy. For this reason, as the sensor of a laser pulse beam, a sensor that has sensitivity to infrared light, such as HgCdTe (MCT), and operates preferably at room temperature is selected.

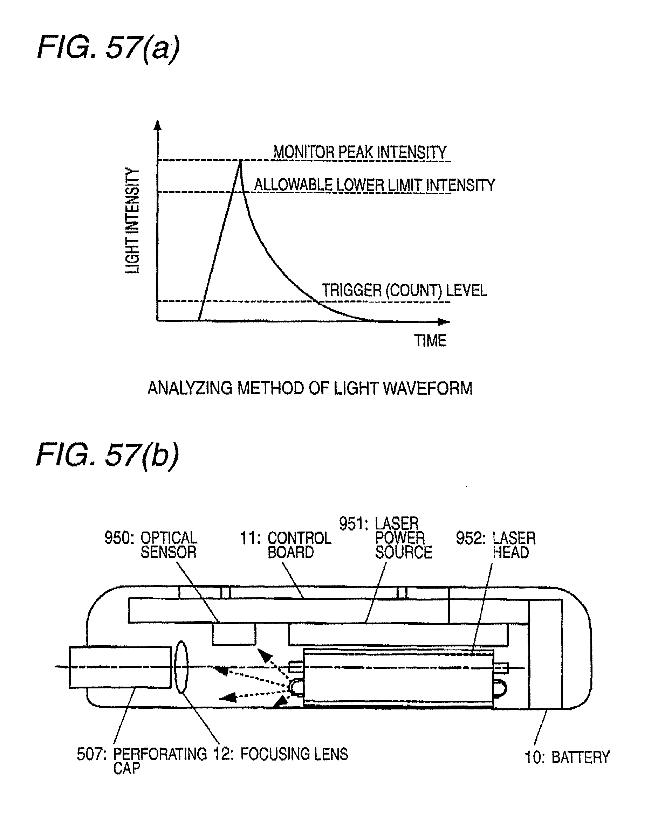

The light waveform measured by the photosensor 950 is analyzed as follows. Fig. 57(a) is a view for explaining the method of analyzing a light waveform. In this drawing, the axis of abscissa represents time, and the axis of ordinate represents detected light intensity. Further, Fig. 57(b) shows an aspect in which the photosensor 950 detects the light emitted from the laser head 952.

First, recording of a waveform is performed in a case where there is an input more than a preset trigger level. Under the ordinary circumstances, in the photosensor 950 set in the main body, the trigger lever is set to such a level that trigger is not effected with external white light or infrared light. In a case where there is an input more than this trigger, the number of counts of a memory in the control board 11 is increased by one (Step S43).

Next, similarly, a wave-like peak value is recorded on the memory in the control board 11 (Step S45). Then, comparison with an estimated peak value stored in advance in the memory on the basis of laser perforating conditions set by condition setting is performed. Then, a measured peak value is compared with the estimated peak value (Step S46). Then, in a case where the measured peak value to the estimated peak value is below an allowable lower limit level, the display that requests maintenance of the display 3 is performed.

Further, in a case where the number of times of integration from the start of use approaches the number of times of upper limit integration similarly stored in advance in the memory within the control board 11, a message that maintenance is near is displayed on the display 3, and in a case where the number of times of upper limit integration is reached, the display that requests the maintenance of the display 3 is performed. In a case where the display that request maintenance, it is also possible to perform the display that forbids the next use and to lock the apparatus.

In order to display summing-up and the number of times of integration within a fixed period, it is possible to operate the setting button 4 in a condition input screen, thereby making them displayed. Further, it is possible to set a password, thereby making only a charging party reset the number of times of integration within a fixed period. Moreover, reading of the number of times of integration or resetting of the number of times of integration within a fixed period can be operated from the outside by giving a function of communication with the outside, such as wired communication (telephone line or LAN circuit) or wireless communication (mobile communication, infrared communication, or wireless LAN), although not shown.

Although the invention has been described in detail with reference to the specific embodiments, it is clear to those skilled in the art that various alternations and modifications can be made without departing from the spirit and scope of the invention. This application claims benefit of Japanese patent application No. 2006-078430 filed on March 22, 2006, 2006-082303 filed on March 24, 2006, 2006-110673 filed on April 13, 2006, and 2006-111805 filed on April 14, 2006, which is hereby incorporated by reference.

### <Industrial Applicability>

As described above, the component concentration measuring apparatus according to the invention has the effect of preventing a plume containing an odor generated during perforating by a laser beam from leaking to the outside, and treating the plume as abnormal odor components, and is useful as a biosensor and a component concentration measuring apparatus that can perforate the skin of a human body with a laser beam, and rapidly and easily quantify a slight amount of various specific components in a sampled biological sample.

## Claims

1. A component concentration measuring apparatus comprising:
a main body having at least a laser device that emits a laser beam, a focusing means of the laser beam, an analyzing device of components of a humour, and a display means that displays a result calculated by the analyzing device;
a sheet;
an insertion body having an opening that does not shield the laser beam; and
a test paper,
wherein the main body provides an opening in the direction of an optical axis of the laser beam,
wherein the opening is mounted with the insertion body, and one end of the insertion body is mounted with the test paper, and
wherein the sheet provides a film that is located between the focusing means and the insertion body, and that is not perforated with the laser beam.

2. The component concentration measuring apparatus according to Claim 1, wherein the test paper is provided with an electrode for component measurement, the insertion body is provided with an electrode, and the electrode for component measurement of the test paper and the electrode of the insertion body are electrically connected by fitting with the test paper.

3. The component concentration measuring apparatus according to Claim 1, wherein the analyzing device analyzes the components of the body fluid by an enzyme reaction of a specimen reagent.

4. The component concentration measuring apparatus according to Claim 3, wherein the analyzing device is an optical device that analyzes the components of the body fluid by a color reaction of the specimen reagent.

5. The component concentration measuring apparatus according to Claim 1, wherein the test paper provides a film that is perforated with the laser beam on the optical axis of the laser beam.

6. The component concentration measuring apparatus according to Claim 5, wherein the test paper further provides a film that is not perforated with the laser beam on the optical axis of the laser beam.

7. The component concentration measuring apparatus according to Claim 1, wherein the test paper has also a function as the sheet.

8. The component concentration measuring apparatus according to Claim 7, wherein the test paper provides a film to be used as the sheet that is not perforated with the laser beam, on the optical axis of the laser beam when used as the sheet.

9. The component concentration measuring apparatus according to Claim 1, wherein an asymmetrical structure is provided on the side of the fitting between a main body of the insertion body, and an opening.

10. The component concentration measuring apparatus according to Claim 1, wherein the fitting between the test paper and the insertion body is performed by an asymmetrical structure.

11. The component concentration measuring apparatus according to Claim 10, wherein the fitting between the test paper and the insertion body is performed by the fitting between the opening of the test paper, and a protrusion of the insertion body.

12. The component concentration measuring apparatus according to Claim 1, wherein the film of the sheet, the insertion body, and at least a portion or all of the film of the test paper are provided with a deodorizing function.

13. The component concentration measuring apparatus according to Claim 1, wherein the test paper, the insertion body, and at least a portion or all of the sheet are provided with an antibacterial function.

14. A biosensor for analyzing components in a specimen sample using perforating by a laser beam for collection of the specimen sample,
wherein the sensor forms a sample supply passage through which the specimen sample supplied is sucked to between first and second substrates, by pasting between the first and second substrates, a filter, and a reagent that reacts with the components in the specimen sample are arranged within the sample supply passage, and an air hole that leads to the outside from the sample supply passage is provided in the second substrate, and
wherein the reagent has an enzyme and a chromogen that reacts specifically with the components, openings that share a portion or all are provided outside the sample supply passage in the first and second substrates, and at least any one of the openings of the first and second substrates is provided with a film.

15. The biosensor according to Claim 14, wherein the film has a deodorizing function.

16. The biosensor according to Claim 14 or 15, wherein the first and second substrates are further provided with openings that share a portion or all other than the openings.

17. A biosensor for laser perforating for analyzing components in a specimen sample using perforating by a laser beam for collection of the specimen sample,
wherein the sensor forms a sample supply passage through which the specimen sample supplied is sucked to between first and second substrates, at least by pasting between the first and second substrates, a reagent that reacts with the components in the specimen sample is provided within the sample supply passage, and an air hole that leads to the outside from the sample supply passage is provided in the second substrate,
wherein the sensor has an electrode system, the electrode system includes at least a measuring electrode and an electrode couple, and the reaction is detected by the electrode system, and
wherein openings that share a portion or all are provided outside the sample supply passage in the first and second substrates, and at least any one of the openings of the first and second substrates is provided with a film.

18. The biosensor for laser perforating according to Claim 17, wherein the film has a deodorizing function.

19. The biosensor for laser perforating according to Claim 17 or 18, wherein the first and second substrates are further provided with openings that share a portion or all other than the openings.

20. The biosensor for laser perforating according to Claim 17 or 18, wherein the first and second substrates are further provided with at least two openings that share a portion or all other than the openings so that the electrode system may be exposed.

21. A component concentration measuring apparatus comprising:
a main body having at least a laser device that emits a laser beam, a focusing means of the laser beam, an analyzing device of components of a humour, and a display means that displays a result calculated by the analyzing device;
a sheet;
an insertion body having an opening through which the laser beam passes; and
a test paper,
wherein the main body provides an opening in the direction of an optical axis of the laser beam,
wherein the opening is mounted with the insertion body, and one end of the insertion body is mounted with the test paper, and
wherein the sheet is located between the focusing means and the insertion body, and the sheet has a function of the test paper.

22. The component concentration measuring apparatus according to Claim 21, wherein the sheet provides a film that is not perforated with the laser beam when used as the sheet and that is perforated with the laser beam when used as the test paper, on the optical axis of the laser beam.

23. The component concentration measuring apparatus according to Claim 21, wherein the sheet provides a film that is perforated with the laser beam on the optical axis of the laser beam when used as the test paper, and provides a film that is not perforated with the laser beam on the optical axis of the laser beam when used as the sheet.

24. The component concentration measuring apparatus according to Claim 21, wherein a function to detect insertion of the sheet into the main body is further provided.

25. The component concentration measuring apparatus according to Claim 24, wherein the function to detect the insertion of the sheet into the main body is performed by mechanical contact operation by the insertion of the sheet.

26. The component concentration measuring apparatus according to Claim 24, wherein the function to detect the insertion of the sheet into the main body is performed by electrical connection with the electrode provided in the sheet,

27. The component concentration measuring apparatus according to any one of Claims 21 to 26, wherein at least one or both of the film of the sheet and the insertion body is provided with a deodorizing function.

28. The component concentration measuring apparatus according to any one of Claims 21 to 27, wherein at least one or both of the insertion body and the sheet is provided with an antibacterial function,

29. A perforating adapter to be mounted on a laser perforating device that irradiates a skin with a laser beam, thereby performing perforating, the adapter comprising:
a hollow body having openings at both ends thereof; and
first and second films provided so as to close the openings of both the ends,
wherein the first film absorbs the laser beam, and is perforated, and the second film transmits the laser beam without absorbing the laser beam.

30. The perforating adapter according to Claim 29, wherein the hollow body has a protrusion formed around the first film.

31. The perforating adapter according to Claim 30 or 31, wherein at least one of the first film, the hollow body, and the second film is provided with a deodorizing function.

32. The perforating adapter according to Claim 30 or 31, wherein the first film is provided with an antibacterial function.

33. A laser perforating device comprising the perforating adapter according to any one of Claims 29 to 32 so as to be detachable with a central axis of the hollow body and a laser optical axis being made to coincide with each other.

34. A laser irradiation method that irradiate a skin including epidermis and dermis with a laser pulse beam, thereby performing laser perforating, the method comprising the steps of:
radiating a laser pulse beam for perforating the epidermis; and
radiating a laser pulse beam for perforating the dermis in a perforating spot of the epidermis.

35. The laser irradiation method according to Claim 34,
wherein the laser pulse beam for perforating the epidermis is the same or longer in time width and is larger in energy than the laser pulse beam for perforating the dermis.

36. The laser irradiation method according to Claim 34,
wherein the time width of the laser pulse beam for perforating the epidermis is 100 to 400 µs, and the time width of the laser pulse beam for perforating the dermis is 50 to 300 µs.

37. The laser irradiation method according to Claim 34, wherein the difference between the time width of the laser pulse beam for perforating the epidermis and the time width of the laser pulse beam for perforating the dermis is 50 to 200 µs.

38. The laser irradiation method according to Claim 34, wherein the irradiation interval between the laser pulse beam for perforating the epidermis and the laser pulse beam for perforating the dermis is 500 ms or less.

39. The laser irradiation method according to Claim 34, wherein the total of the energy when the laser pulse beam for perforating the epidermis and the laser pulse beam for perforating the dermis irradiate is 100 to 300 J per one square centimeters.

40. The laser irradiation method according to Claim 34, wherein the energy of the laser pulse beam for perforating the dermis is 10 to 40% of the total of the energy of the laser pulse beam for perforating the epidermis and the laser pulse beam for perforating the dermis.

41. The laser irradiation method according to Claim 34, wherein the focusing diameter of the laser pulse beam is 0.15 mm or less.

42. The laser irradiation method according to Claim 34, wherein the laser pulse beam for perforating the epidermis includes a plurality of laser pulse beams.

43. The laser irradiation method according to Claim 42, wherein the time width of each of the plurality of laser pulse beams for perforating the epidermis is 100 to 400 µs.

44. The laser irradiation method according to Claim 42, wherein the irradiation interval of the plurality of laser pulse beams for perforating the epidermis is 500 ms or less.

45. The laser irradiation method according to Claim 42, wherein the total of the energy when the plurality of laser pulse beams for perforating the epidermis and the laser pulse beam for perforating the dermis irradiate is 5 to 100 J per one square centimeters.

46. A laser perforating device that irradiates a skin with a laser beam, thereby performing perforating, comprising:
a periodic sound generating means that generates a periodic sound,
wherein perforating is performed during generation of the periodic sound.

47. The laser perforating device according to Claim 46, wherein the periodic sound is a sound whose center frequency is repeated in monotone of 20 to 100 Hz.

48. The laser perforating device according to Claim 47, wherein the sound that is repeated in monotone includes an operation sound of a pump, and an engine sound.

49. The laser perforating device according to Claim 46, wherein the periodic sound is a sound that has a repeated tempo of 60 to 208 beats per 1 minute.

50. The laser perforating device according to Claim 49, wherein the sound having the repeated tempo includes a metronome sound, an ON/OFF sound of a mechanical switch, and a sound that strikes a keyboard.

51. The laser perforating device according to Claim 46, wherein the periodic sound generating means generates a plurality of kinds of periodic sounds that are different in sound quality or period, and randomly changes the kind of a periodic sound to be generated at every perforating.

52. The laser perforating device according to Claim 46, wherein the time from the generation of the periodic sound to perforating is changed randomly at every perforating.

53. A laser perforating method that irradiates a skin with a laser beam, thereby performing perforating, the method comprising steps of:
randomly selecting and generating a predetermined periodic sound from a plurality of kinds of periodic sounds that are different in sound quality or period; and
performing perforating after lapse of the random time from the generation of the predetermined periodic sound.

54. A laser perforating device that irradiates a skin with a laser beam, thereby performing perforating, the device comprising:
an adjusting means that adjusts the energy of the laser pulse beam according to the water amount of the skin measured by a water-amount measuring sensor that measures the water amount of the skin.

55. The laser perforating device according to Claim 54, comprising the water-amount measuring sensor.

56. The laser perforating device according to Claim 55, wherein the water-amount measuring sensor includes a sensor electrode that is set on the surface of a main body of the laser perforating device to measure the water amount of the skin.

57. The perforating device according to Claim 54, comprising a holding means that detachably holds a perforating cap having the water-amount measuring sensor,
wherein the perforating cap includes a hollow body having openings at both ends thereof, and first and second films provided so as to close the openings of both the ends,
wherein the first film transmits the laser beam and absorbs the laser beam, and is perforated, and the second film transmits the laser beam without absorbing the laser beam, and
wherein the water-amount measuring sensor includes a sensor electrode that is set on the same plane of the first film to measure the water amount of the skin.

58. The laser perforating device according to Claim 54, comprising a setting button that inputs user's attributes, wherein the adjusting means adjusts the energy of the laser pulse beam according to an attribute input from the setting button.

59. The laser perforating device according to Claim 54, comprising a humidity sensor that measures the humidity of the ambient air,
wherein the adjusting means adjusts the energy of the laser pulse beam according to the humidity of the ambient air measured by the humidity sensor.

60. A laser perforating method that irradiates a skin with a laser beam, thereby performing perforating, the method comprising the steps of:
measuring the water amount of the skin; and
adjusting the energy of the laser pulse beam according to the measured water amount of the skin,

61. An insertion holder to be mounted on a laser perforating device that irradiates a skin with a laser beam, thereby performing perforating, the insertion holder comprising:
a hollow body having openings at both ends thereof;
a film that is provided so as to close one end of the openings, and transmits the laser beam without absorbing the laser beam;
a biosensor provided so as to close the other end of the openings; and
an electrode that is provided at an outer or inner peripheral face of the hollow body to electrically connect the laser perforating device and the biosensor.

62. The insertion holder according to Claim 67, wherein a fingerrest film is formed in a portion of the surface of the biosensor, and a specimen reagent supply passage opening is formed in a side face of the biosensor.

63. The insertion holder according to Claim 61, wherein the biosensor has a measuring electrode and an electrode couple that are formed in a reagent layer.

64. An insertion holder to be mounted on a laser perforating device that irradiates a skin with a laser beam, thereby performing perforating, the insertion holder comprising:
a hollow body formed by bending a biosensor, and joining ends of the biosensor;
a film that is provided so as to close one end of the hollow body, and transmits the laser beam without absorbing the laser beam;
a film that is provided so as to close the other end of the hollow body, and absorbs the laser beam and is perforated; and
an electrode that is provided at an outer or inner peripheral face of the hollow body to electrically connect the laser perforating device and the biosensor.

65. A laser irradiation method of irradiating a skin via a film with a laser pulse beam, thereby performing laser perforating, the method comprising the steps of:
radiating a first laser pulse beam for perforating the film; and
irradiating a second laser pulse beam for perforating the skin, in a perforating spot of the film.

66. The laser irradiation method according to Claim 65, wherein the intensity of the first laser pulse beam is weaker than that of the second laser pulse beam.

67. A biosensor to be mounted on a laser perforating device that irradiates a skin with a laser beam, thereby performing perforating, the biosensor comprising:
a first substrate that has a reagent layer patched thereon, and has a first opening through which the laser beam passes;
a second substrate that is arranged so as to face the first substrate at a predetermined spacing therefrom, has a second opening corresponding to the first opening, and has an air hole in a position more distant from the second opening than the reagent layer; and
a specimen reagent supply passage formed from the first opening to the reagent layer between the first substrate and the second substrate.

68. The biosensor according to Claim 67, wherein the optical axis of the laser beam is set parallel to a centerline of the first opening, and in a position closer to the specimen reagent supply passage than the centerline of the first opening.

69. A laser perforating device that irradiate a skin with a laser beam excited by a flash lamp, thereby performing perforating, comprising an optical sensor that detects light emission of the flash lamp to monitor the degree of deterioration of the flash lamp.

70. The laser perforating device according to Claim 69, comprising a notifying means that notifies a user of a message that requests the maintenance of the device on the basis of the number of times of use of the device detected by the photosensor.

## Amended claims

### Amended claims under Art. 19.1 PCT

1. A component concentration measuring apparatus comprising:
a main body having at least a laser device that emits a laser beam, a focusing means of the laser beam, an analyzing device of components of a humour, and a display means that displays a result calculated by the analyzing device;
a sheet;
an insertion body having an opening that does not shield the laser beam; and
a test paper,
wherein the main body provides an opening in the direction of an optical axis of the laser beam,
wherein the opening is mounted with the insertion body, and one end of the insertion body is mounted with the test paper, and
wherein the sheet provides a film that is located between the focusing means and the insertion body, and that is not perforated with the laser beam.

2. The component concentration measuring apparatus according to Claim 1, wherein the test paper is provided with an electrode for component measurement, the insertion body is provided with an electrode, and the electrode for component measurement of the test paper and the electrode of the insertion body are electrically connected by fitting with the test paper.

3. The component concentration measuring apparatus according to Claim 1, wherein the analyzing device analyzes the components of the body fluid by an enzyme reaction of a specimen reagent.

4. The component concentration measuring apparatus according to Claim 3, wherein the analyzing device is an optical device that analyzes the components of the body fluid by a color reaction of the specimen reagent.

5. The component concentration measuring apparatus according to Claim 1, wherein the test paper provides a film that is perforated with the laser beam on the optical axis of the laser beam.

6. The component concentration measuring apparatus according to Claim 5, wherein the test paper further provides a film that is not perforated with the laser beam on the optical axis of the laser beam.

7. The component concentration measuring apparatus according to Claim 1, wherein the test paper has also a function as the sheet.

8. The component concentration measuring apparatus according to Claim 7, wherein the test paper provides a film to be used as the sheet that is not perforated with the laser beam, on the optical axis of the laser beam when used as the sheet.

9. The component concentration measuring apparatus according to Claim 1, wherein an asymmetrical structure is provided on the side of the fitting between a main body of the insertion body, and an opening.

10. The component concentration measuring apparatus according to Claim 1, wherein the fitting between the test paper and the insertion body is performed by an asymmetrical structure.

11. The component concentration measuring apparatus according to Claim 10. wherein the fitting between the test paper and the insertion body is performed by the fitting between the opening of the test paper, and a protrusion of the insertion body.

12. The component concentration measuring apparatus according to Claim 1, wherein the film of the sheet, the insertion body, and at least a portion or all of the film of the test paper are provided with a deodorizing function.

13. The component concentration measuring apparatus according to Claim 1, wherein the test paper, the insertion body, and at least a portion or all of the sheet are provided with an antibacterial function.

14. A biosensor for analyzing components in a specimen sample using perforating by a laser beam for collection of the specimen sample,
wherein the sensor forms a sample supply passage through which the specimen sample supplied is sucked to between first and second substrates, by pasting between the first and second substrates, a filter, and a reagent that reacts with the components in the specimen sample are arranged within the sample supply passage, and an air hole that leads to the outside from the sample supply passage is provided in the second substrate, and
wherein the reagent has an enzyme and a chromogen that reacts specifically with the components, openings that share a portion or all are provided outside the sample supply passage in the first and second substrates, and at least any one of the openings of the first and second substrates is provided with a film,

15. The biosensor according to Claim 14, wherein the film has a deodorizing function.

16. The biosensor according to Claim 14 or 15, wherein the first and second substrates are further provided with openings that share a portion or all other than the openings.

17. A biosensor for laser perforating for analyzing components in a specimen sample using perforating by a laser beam for collection of the specimen sample,
wherein the sensor forms a sample supply passage through which the specimen sample supplied is sucked to between first and second substrates, at least by pasting between the first and second substrates, a reagent that reacts with the components in the specimen sample is provided within the sample supply passage, and an air hole that leads to the outside from the sample supply passage is provided in the second substrate,
wherein the sensor has an electrode system, the electrode system includes at least a measuring electrode and an electrode couple, and the reaction is detected by the electrode system, and
wherein openings that share a portion or all are provided outside the sample supply passage in the first and second substrates, and at least any one of the openings of the first and second substrates is provided with a film.

18. The biosensor for laser perforating according to Claim 17, wherein the film has a deodorizing function.

19. The biosensor for laser perforating according to Claim 17 or 18, wherein the first and second substrates are further provided with openings that share a portion or all other than the openings.

20. The biosensor for laser perforating according to Claim 17 or 18, wherein the first and second substrates are further provided with at least two openings that share a portion or all other than the openings so that the electrode system may be exposed.

21. A component concentration measuring apparatus comprising:
a main body having at least a laser device that emits a laser beam, a focusing means of the laser beam, an analyzing device of components of a humour, and a display means that displays a result calculated by the analyzing device;
a sheet;
an insertion body having an opening through which the laser beam passes; and
a test paper,
wherein the main body provides an opening in the direction of an optical axis of the laser beam,
wherein the opening is mounted with the insertion body, and one end of the insertion body is mounted with the test paper, and
wherein the sheet is located between the focusing means and the insertion body, and the sheet has a function of the test paper.

22. The component concentration measuring apparatus according to Claim 21, wherein the sheet provides a film that is not perforated with the laser beam when used as the sheet and that is perforated with the laser beam when used as the test paper, on the optical axis of the laser beam.

23. The component concentration measuring apparatus according to Claim 21, wherein the sheet provides a film that is perforated with the laser beam on the optical axis of the laser beam when used as the test paper, and provides a film that is not perforated with the laser beam on the optical axis of the laser beam when used as the sheet.

24. The component concentration measuring apparatus according to Claim 21, wherein a function to detect insertion of the sheet into the main body is further provided.

25. The component concentration measuring apparatus according to Claim 24, wherein the function to detect the insertion of the sheet into the main body is performed by mechanical contact operation by the insertion of the sheet.

26. The component concentration measuring apparatus according to Claim 24, wherein the function to detect the insertion of the sheet into the main body is performed by electrical connection with the electrode provided in the sheet.

27. The component concentration measuring apparatus according to any one of Claims 21 to 26, wherein at least one or both of the film of the sheet and the insertion body is provided with a deodorizing function.

28. The component concentration measuring apparatus according to any one of Claims 21 to 27, wherein at least one or both of the insertion body and the sheet is provided with an antibacterial function.

29. A perforating adapter to be mounted on a laser perforating device that irradiates a skin with a laser beam, thereby performing perforating, the adapter comprising:
a hollow body having openings at both ends thereof; and
first and second films provided so as to close the openings of both the ends,
wherein the first film absorbs the laser beam, and is perforated, and the second film transmits the laser beam without absorbing the laser beam.

30. The perforating adapter according to Claim 29, wherein the hollow body has a protrusion formed around the first film.

31. (Amended)
The perforating adapter according to Claim 29 or 30 wherein at least one of the first film, the hollow body, and the second film is provided with a deodorizing function.

32. The perforating adapter according to Claim 30 or 31, wherein the first film is provided with an antibacterial function.

33. A laser perforating device comprising the perforating adapter according to any one of Claims 29 to 32 so as to be detachable with a central axis of the hollow body and a laser optical axis being made to coincide with each other.

34. A laser irradiation method that irradiate a skin including epidermis and dermis with a laser purse beam, thereby performing laser perforating, the method comprising the steps of:
radiating a laser pulse beam for perforating the epidermis; and
radiating a laser pulse beam for perforating the dermis in a perforating spot of the epidermis.

35. The laser irradiation method according to Claim 34,
wherein the laser pulse beam for perforating the epidermis is the same or longer in time width and is larger in energy than the laser pulse beam for perforating the dermis.

36. The laser irradiation method according to Claim 34,
wherein the time width of the laser pulse beam for perforating the epidermis is 100 to 400 µs, and the time width of the laser pulse beam for perforating the dermis is 50 to 300 µs.

37. The laser irradiation method according to Claim 34, wherein the difference between the time width of the laser pulse beam for perforating the epidermis and the time width of the laser pulse beam for perforating the dermis is 50 to 200 µs.

38. The laser irradiation method according to Claim 34, wherein the irradiation interval between the laser pulse beam for perforating the epidermis and the laser pulse beam for perforating the dermis is 500 ms or less.

39. The laser irradiation method according to Claim 34, wherein the total of the energy when the laser pulse beam for perforating the epidermis and the laser pulse beam for perforating the dermis irradiate is 100 to 300 J per one square centimeters.

40. The laser irradiation method according to Claim 34, wherein the energy of the laser pulse beam for perforating the dermis is 10 to 40% of the total of the energy of the laser pulse beam for perforating the epidermis and the laser pulse beam for perforating the dermis.

41. The laser irradiation method according to Claim 34, wherein the focusing diameter of the laser pulse beam is 0.15 mm or less.

42. The laser irradiation method according to Claim 34, wherein the laser pulse beam for perforating the epidermis includes a plurality of laser pulse beams.

43. The laser irradiation method according to Claim 42, wherein the time width of each of the plurality of laser pulse beams for perforating the epidermis is 100 to 400 µs.

44. The laser irradiation method according to Claim 42, wherein the irradiation interval of the plurality of laser pulse beams for perforating the epidermis is 500 ms or less.

45. The laser irradiation method according to Claim 42, wherein the total of the energy when the plurality of laser pulse beams for perforating the epidermis and the laser pulse beam for perforating the dermis irradiate is 5 to 100 J per one square centimeters.

46. (Deleted)

47. (Deleted)

48. (Deleted)

49. (Deleted)

50. (Deleted)

51. (Deleted)

52. (Deleted)

53. A laser perforating method that irradiates a skin with a laser beam, thereby performing perforating, the method comprising steps of:
randomly selecting and generating a predetermined periodic sound from a plurality of kinds of periodic sounds that are different in sound quality or period; and
performing perforating after lapse of the random time from the generation of the predetermined periodic found.

54. A laser perforating device that irradiates a skin with a laser beam, thereby performing perforating, the device comprising:
an adjusting means that adjusts the energy of the laser pulse beam according to the water amount of the skin measured by a water-amount measuring sensor that measures the water amount of the skin.

55. The laser perforating device according to Claim 54, comprising the water-amount measuring sensor.

56. The laser perforating device according to Claim 55, wherein the water-amount measuring sensor includes a sensor electrode that is set on the surface of a main body of the laser perforating device to measure the water amount of the skin.

57. The perforating device according to Claim 54, comprising a holding means that detachably holds a perforating cap having the water-amount measuring sensor,
wherein the perforating cap includes a hollow body having openings at both ends thereof, and first and second films provided so as to close the openings of both the ends,
wherein the first film transmits the laser beam and absorbs the laser beam, and is perforated, and the second film transmits the laser beam without absorbing the laser beam, and
wherein the water-amount measuring sensor includes a sensor electrode that is set on the same plane of the first film to measure the water amount of the skin.

58. The laser perforating device according to Claim 54, comprising a setting button that inputs user's attributes, wherein the adjusting means adjusts the energy of the laser pulse beam according to an attribute input from the setting button.

59. The laser perforating device according to Claim 54, comprising a humidity sensor that measures the humidity of the ambient air,
wherein the adjusting means adjusts the energy of the laser pulse beam according to the humidity of the ambient air measured by the humidity sensor.

60. A laser perforating method that irradiates a skin with a laser beam, thereby performing perforating, the method comprising the steps of:
measuring the water amount of the skin; and
adjusting the energy of the laser pulse beam according to the measured water amount of the skin.

61. An insertion holder to be mounted on a laser perforating device that irradiates a skin with a laser beam, thereby performing perforating, the insertion holder comprising:
a hollow body having openings at both ends thereof;
a film that is provided so as to close one end of the openings, and transmits the laser beam without absorbing the laser beam;
a biosensor provided so as to close the other end of the openings; and
an electrode that is provided at an outer or inner peripheral face of the hollow body to electrically connect the laser perforating device and the biosensor.

62. The insertion holder according to Claim 61 , wherein a fingerrest film is formed in a portion of the surface of the biosensor, and a specimen reagent supply passage opening is formed in a side face of the biosensor.

63. The insertion holder according to Claim 61, wherein the biosensor has a measuring electrode and an electrode couple that are formed in a reagent layer.

64. An insertion holder to be mounted on a laser perforating device that irradiates a skin with a laser beam, thereby performing perforating, the insertion holder comprising:
a hollow body formed by bending a biosensor, and joining ends of the biosensor;
a film that is provided so as to close one end of the hollow body, and transmits the laser beam without absorbing the laser beam;
a film that is provided so as to close the other end of the hollow body, and absorbs the laser beam and is perforated; and
an electrode that is provided at an outer or inner peripheral face of the hollow body to electrically connect the laser perforating device and the biosensor.

65. A laser irradiation method of irradiating a skin via a film with a laser pulse beam, thereby performing laser perforating, the method comprising the steps of:
radiating a first laser pulse beam for perforating the film; and
irradiating a second laser pulse beam for perforating the skin, in a perforating spot of the film.

66. The laser irradiation method according to Claim 65, wherein the intensity of the first laser pulse beam is weaker than that of the second laser pulse beam.

67. A biosensor to be mounted on a laser perforating device that irradiates a skin with a laser beam, thereby performing perforating, the biosensor comprising:
a first substrate that has a reagent layer patched thereon, and has a first opening through which the laser beam passes;
a second substrate that is arranged so as to face the first substrate at a predetermined spacing therefrom, has a second opening corresponding to the first opening, and has an air hole in a position more distant from the second opening than the reagent layer; and
a specimen reagent supply passage formed from the first opening to the reagent layer between the first substrate and the second substrate.

68. The biosensor according to Claim 67, wherein the optical axis of the laser beam is set parallel to a centerline of the first opening, and in a position closer to the specimen reagent supply passage than the centerline of the first opening,

69. A laser perforating device that irradiate a skin with a laser beam excited by a flash lamp, thereby performing perforating, comprising an optical sensor that detects light emission of the flash lamp to monitor the degree of deterioration of the flash lamp.

70. The laser perforating device according to Claim 69, comprising a notifying means that notifies a user of a message that requests the maintenance of the device on the basis of the number of times of use of the device detected by the photosensor.
